# EUROPEAN PATENT SPECIFICATION

(11) **EP 4 055 164 B1**
(45) Date of publication and mention of the grant of the patent: **07.10.2026**
(21) Application number: 20803809.1
(22) Date of filing: 06.11.2020
(51) Int. Cl.: C12N 15/113, A61K 31/712, A61K 31/343, A61P 35/00

(54) **TREATMENT OF ABERRANT FIBROBLAST PROLIFERATION**
BEHANDLUNG VON ANORMALER FIBROBLASTENPROLIFERATION
TRAITEMENT DE LA PROLIFÉRATION ABERRANTE DES FIBROBLASTES

(30) Priority: 08.11.2019 US 201916678398
(43) Date of publication of application: 14.09.2022
(73) Proprietor: Georg-August-Universitaet Goettingen Stiftung Oeffentlichen Rechts, Universitaetsmedizin, 37075 Goettingen (DE); Board of Regents, The University of Texas System, Austin, TX 78701 (US)
(72) Inventor: ZEISBERG, Michael, 37085 Goettingen (DE); ZEISBERG, Elisabeth, 37085 Goettingen (DE); TAMPE, Bjoern, 37077 Goettingen (DE); MUELLER, Gerhard Anton, 37077 Goettingen (DE); XINGBO, Xu, 37077 Goettingen (DE); TAMPE, Desiree, 37077 Goettingen (DE); KALLURI, Raghu, Houston, TX 77030-3907 (US)
(74) Representative: Grund, Martin
(86) International application number: PCT/EP2020/081363
(87) International publication number: WO 2021/089828

(56) References cited:
- WO-A2-2010/077693
- KR-A- 20190 047 489
- CASTIGLIONE ALESSANDRO ET AL: "EYA1-related disorders: Two clinical cases and a literature review", INTERNATIONAL JOURNAL OF PEDIATRIC OTORHINOLARYNGOLOGY, ELSEVIER, AMSTERDAM, NL, vol. 78, no. 8, 12 April 2014 (2014-04-12), pages 1201 - 1210, XP028879371, ISSN: 0165-5876, DOI: 10.1016/J.IJPORL.2014.03.032
- HAITAO GUAN ET AL: "MicroRNA-101 inhibits cell proliferation and induces apoptosis by targeting EYA1 in breast cancer", INTERNATIONAL JOURNAL OF MOLECULAR MEDICINE, vol. 37, no. 6, 11 April 2016 (2016-04-11), GR, pages 1643 - 1651, XP055765749, ISSN: 1107-3756, DOI: 10.3892/ijmm.2016.2557
- KONGMING WU ET AL: "EYA1 Phosphatase Function Is Essential to Drive Breast Cancer Cell Proliferation through Cyclin D1", CANCER RESEARCH, vol. 73, no. 14, 15 July 2013 (2013-07-15), US, pages 4488 - 4499, XP055769684, ISSN: 0008-5472, DOI: 10.1158/0008-5472.CAN-12-4078
- DEGUANG KONG ET AL: "EYA1 promotes cell migration and tumor metastasis in hepatocellular carcinoma", AM J TRANSL RES, vol. 11, 15 April 2019 (2019-04-15), pages 2328 - 2338, XP055766000
- SUPRIYA V. VARTAK ET AL: "Autocyclized and oxidized forms of SCR7 induce cancer cell death by inhibiting nonhomologous DNA end joining in a Ligase IV dependent manner", FEBS JOURNAL, vol. 285, no. 21, 8 October 2018 (2018-10-08), GB, pages 3959 - 3976, XP055765655, ISSN: 1742-464X, DOI: 10.1111/febs.14661
- WIBKE BECHTEL ET AL: "Methylation determines fibroblast activation and fibrogenesis in the kidney", NATURE MEDICINE, vol. 16, no. 5, 25 April 2010 (2010-04-25), New York, pages 544 - 550, XP055255256, ISSN: 1078-8956, DOI: 10.1038/nm.2135
- WIBKE BECHTEL ET AL: "SUPPLEMENTARY INFORMATION Methylation determines fibroblast activation and fibrogenesis in the kidney", NATURE MEDICINE, 25 April 2010 (2010-04-25), XP055769785, Retrieved from the Internet <URL:https://static-content.springer.com/esm/art%3A10.1038%2Fnm.2135/MediaObjects/41591_2010_BFnm2135_MOESM7_ESM.pdf> [retrieved on 20210128], DOI: 10.1038/nm.2135
- J ZHOU ET AL: "Eyes absent gene (EYA1) is a pathogenic driver and a therapeutic target for melanoma", ONCOTARGET, vol. 8, no. 62, 27 September 2017 (2017-09-27), pages 105081 - 105092, XP055765676, DOI: 10.1016/j.jid.2017.02.146
- JUN LI ET AL: "EYA1's Conformation Specificity in Dephosphorylating Phosphothreonine in Myc and Its Activity on Myc Stabilization in Breast Cancer", MOLECULAR AND CELLULAR BIOLOGY, vol. 37, no. 1, 17 October 2016 (2016-10-17), US, XP055765750, ISSN: 0270-7306, DOI: 10.1128/MCB.00499-16
- SUN Y ET AL: "The PI3K/Akt signal hyperactivates Eya1 via the SUMOylation pathway", ONCOGENE, NATURE PUBLISHING GROUP UK, LONDON, vol. 34, no. 19, 23 June 2014 (2014-06-23), pages 2527 - 2537, XP036972878, ISSN: 0950-9232, [retrieved on 20140623], DOI: 10.1038/ONC.2014.179
- CAI SHAOXIN ET AL: "EYA1 promotes tumor angiogenesis by activating the PI3K pathway in colorectal cancer", EXPERIMENTAL CELL RESEARCH, ELSEVIER, AMSTERDAM, NL, vol. 367, no. 1, 27 February 2018 (2018-02-27), pages 37 - 46, XP085377403, ISSN: 0014-4827, DOI: 10.1016/J.YEXCR.2018.02.028
- S. RICKARD ET AL: "Importance of clinical evaluation and molecular testing in the branchio-oto-renal (BOR) syndrome and overlapping phenotypes", JOURNAL OF MEDICAL GENETICS, vol. 37, no. 8, 1 August 2000 (2000-08-01), pages 623 - 627, XP055765760, DOI: 10.1136/jmg.37.8.623
- LI DUNHUI ET AL: "Precision Medicine through Antisense Oligonucleotide-Mediated Exon Skipping", TRENDS IN PHARMACOLOGICAL SCIENCES, ELSEVIER, HAYWARTH, GB, vol. 39, no. 11, 30 September 2018 (2018-09-30), pages 982 - 994, XP085508814, ISSN: 0165-6147, DOI: 10.1016/J.TIPS.2018.09.001

## Description

### FIELD OF THE DISCLOSURE

Provided are means for preventing, treating and/or delaying progression of aberrant fibroblast proliferation such as fibrosis and desmoplasia in a subject. Disclosed are in particular compounds that reduce the level of protein variant EYA1A in a parenchymal organ for use in preventing, treating or delaying progression of fibrosis and/or desmoplasia in said parenchymal organ, wherein the compound is an antisense oligonucleotide.

### BACKGROUND

The following discussion of the background is merely provided to aid the reader in understanding the invention and is not admitted to describe or constitute prior art to the present invention.

Fibroblasts are mesenchymal cells and the most common cell type of the connective tissues found throughout the body. They are the primary source of components of the extracellular matrix such as collagens, fibres, glycosaminoglycans and glycoproteins. Fibroblasts are also primarily responsible for remodeling in wound healing, and serve pivotal roles in inflammation, angiogenesis, cancer progression, and in physiological as well as pathological tissue fibrosis.

Excessive proliferation of fibroblasts is involved in numerous premalignant and malignant tumours, and cancer has been found to be associated with fibroblasts at all stages of disease progression, including metastasis. Fibroblasts are also regarded a considerable component of the general host response to tissue damage caused by cancer cells. Fibroblasts are furthermore involved in creating extracellular matrix structure and metabolic and immune reprogramming of the tumour microenvironment with an impact on adaptive resistance to chemotherapy.

40% of deaths worldwide are linked to fibrosis. Fibrosis typically follows inflammation or physical tissue injury, and is characterized by the formation of excessive fibrous connective tissue in an organ or tissue in a reparative or reactive process. In response to injury, for instance myocardial infarction, the process is called scarring. Fibrosis can impair tissue function and cause chronic diseases in a large variety of vital organs and tissues, including bone marrow. In skeletal muscle, fibrosis is typically associated with the muscular dystrophies. Fibrosis leads to destruction of organ architecture (Rockey, D.C., et al., N Engl J Med (2015) 372, 1138-1149; Tampe, D. & Zeisberg, M., Nat Rev Nephrol (2014) 10, 226-237).

Irreversible damage of parenchymal tissues going hand in hand with highly differentiated cells being replaced by scarring connective tissue leads to chronic failure of parenchymal organs such as the liver, kidney or the lungs. While a sufficient reserve of cells as well as mechanisms for repairing damaged areas are generally in place, in case of fibrosis tissue restoration is based on fibroblasts and functionally inappropriate connective tissue and thus loss of functional parenchyma. Fibroblasts are principal mediators of fibrosis. Fibrosis involves stimulated fibroblasts laying down connective tissue, and fibrogenesis is unequivocally associated with excessive fibroblast proliferation (Tampe & Zeisberg, 2014, supra; Bechtel, W., et al., Nat Med (2010) 16, 544-550; LeBleu, VS., et al., Nat Med (2013) 19, 1047-1053; Rodemann, H.P & Muller, GA., Proc Soc Exp Biol Med (1990) 195, 57-63).

The enhanced replicative stress and constant exposure to potentially genotoxic cellular metabolites can cause DNA damage, and DNA double-strand breaks (DSBs) are abundant within proliferating fibroblasts in chronic injury and fibrosis (Svegliati, S., et al., Sci Signal (2014) 7, ra84; Krizhanovsky, V, et al., Cell (2008) 134, 657-667; Zhou, W., et al., Nat Genet (2012) 44, 910-915). Abundant DNA damage, however, typically triggers cell cycle checkpoint activation, effectively pausing proliferation (Elledge, S.J., Science (1996) 274, 1664-1672). While parenchymal cells within chronically injured organs undergo permanent cell cycle arrest and senescence (Sturmlechner, I., et al., Nat Rev Nephrol (2017) 13, 77-89), it is not yet understood how fibroblasts can evade cell cycle arrest and sustain proliferative activity instead.

WO 2016/085981 suggests the use of isoprenoid compounds, in particular geranyl-geranylacetone, for inhibiting, reducing, or treating fibrosis. WO 2016/046130 suggests the use of a monoacylglycerol lipase (MGL) inhibitor. WO/2004/112565 teaches that the HNOEL-iso protein, which is localized in the ER, is a modulator in fibrosis. The document suggests the use of an inhibitor of the HNOEL-iso protein to reduce the proliferation of fibroblasts and to reduce or limit the formation of fibrotic regions in a tissue. Guan et al. describe the use of microRNA-101 or of siRNA targeting the EYA1 transcript to inhibit expression of EYA1 to treat breast cancer.

### SUMMARY

The invention relates to a compound that reduces the level of protein variant EYA1A in a parenchymal organ for use in a method of preventing, treating or delaying progression of fibrosis and/or desmoplasia in said parenchymal organ, wherein the compound is an antisense oligonucleotide against at least one of a portion of at least 11 consecutive bases within the sequence of SEQ ID NO: 34 and/or is a reverse complement of the portion within the sequence of SEQ ID NO: 34, an antisense oligonucleotide against at least one of a portion of at least 11 consecutive bases within the sequence of SEQ ID NO: 35 and/or is a reverse complement of the portion within the sequence of SEQ ID NO: 35, an antisense oligonucleotide against at least one of a portion of at least 11 consecutive bases within the sequence of SEQ ID NO: 36 and/or is a reverse complement of the portion of the sequence of SEQ ID NO: 36.

All disclosures relating to a method of treatment are to be considered as referring to the corresponding medical use instead.

The present disclosure provides methods that allow diagnosing and treating a disease involving aberrant fibroblast proliferation such as fibrosis or a tumour. These methods are based on the inventors' findings on the effect of the removal, vs. the lack thereof, of exon 10 of the gene encoding the protein EYA1. Provided are in particular means of controlling the formation of the EYA1 protein in such a way that it does not include exon 10. Provided are also screening methods for an agent suitable for treating a disease involving aberrant fibroblast proliferation. A respective screening method may be a method of screening for an agent suitable for controlling the formation of the EYA1 protein in such a way that it does not include exon 10.

The present disclosure provides methods that allow diagnosing and treating a disease involving aberrant fibroblast proliferation. These methods are based on the inventors' findings on the effect of the removal, vs. the lack thereof, of exon 10 of the gene encoding the protein EYA1. Provided are in particular means of controlling the formation of the EYA1 protein in such a way that it does not include exon 10. Provided are also screening methods for an agent suitable for treating a disease involving aberrant fibroblast proliferation, e.g. fibrosis. A respective screening method may be a method of screening for an agent suitable for controlling the formation of the EYA1 protein in such a way that it does not include exon 10.

Provided is a compound for use in a method of preventing, treating or delaying onset or progression of a disease that involves aberrant fibroblast proliferation in a parenchymal organ. The method may include administering a compound that reduces the level of tyrosine phosphatase activity effected by the protein EYA1A in the parenchymal organ. The method may also include administering a nucleic acid ligase IV inhibitor. The method may also include administering an antisense oligonucleotide against the p53-binding protein 1 (53BP1). The method may also include administering a compound that reduces the level of tyrosine phosphatase activity effected by the protein EYA1A in the parenchymal organ and a nucleic acid ligase IV inhibitor. The method may also include administering a compound that reduces the level of tyrosine phosphatase activity effected by the protein EYA1A in the parenchymal organ and an antisense oligonucleotide against the p53-binding protein 1 (53BP1). The method may also include administering (i) a compound that reduces the level of tyrosine phosphatase activity effected by the protein EYA1A in the parenchymal organ, (ii) a nucleic acid ligase IV inhibitor, and (iii) an antisense oligonucleotide against the p53-binding protein 1 (53BP1).

In some embodiments a method as disclosed herein includes administering a compound that reduces the level of the protein EYA1A. Put differently, compared to a state without the method being carried out, on the protein level less EYA1A is detectable. In some embodiments less EYA1A is detectable in a parenchymal organ of a subject. In some embodiments a method as disclosed herein includes administering a compound that effects the removal of exon 10 during splicing of a transcript of the Eya1 gene. Thereby the ratio between EYA1A and EYA1C is shifted toward EYA1C. In some embodiments the ratio between EYA1A and EYA1C is shifted toward EYA1C in a parenchymal organ of a subject. In some embodiments a method as disclosed herein includes administering an inhibitor of the phosphotyrosine phosphatase activity of EYA1A.

In some embodiments the aberrant fibroblast proliferation includes desmoplasia and/or fibrosis.

According to a first aspect, there is provided a compound that reduces the level of protein variant EYA1A in a parenchymal organ for use in a method of preventing, treating or delaying progression of fibrosis and/or desmoplasia in said parenchymal organ.

The method may involve additionally using one or more compounds of (i) a nucleic acid ligase IV inhibitor, (ii) an antisense oligonucleotide against the p53-binding protein 1, (iii) an inhibitor of the phosphotyrosine phosphatese activity of the protein EYA1A, or (iv) a nucleic acid molecule that includes an endonuclease 9 encoding sequence, a TET domain, and a sequence encoding an sgRNA specific for a portion of a CpG island of the gene encoding EYA1.

The antisense oligonucleotide against a splice junction in the gene encoding the protein EYA1 may be directed against a portion of at least 12 consecutive bases within the sequence of SEQ ID NO: 34 or a complement thereof. The antisense oligonucleotide may also be directed against a portion of at least 14 consecutive bases within the sequence of SEQ ID NO: 34 or a complement thereof. In some embodiments the antisense oligonucleotide against a splice junction in the gene encoding the protein EYA1 may be directed against at least 16 consecutive bases within the sequence of SEQ ID NO: 34.

The antisense oligonucleotide against a splice junction in the gene encoding the protein EYA1 may be directed against a portion of at least 12 consecutive bases within the sequence of SEQ ID NO: 35 or a complement thereof. The antisense oligonucleotide may also be directed against a portion of at least 14 consecutive bases within the sequence of SEQ ID NO: 35 or a complement thereof. In some embodiments the antisense oligonucleotide against a splice junction in the gene encoding the protein EYA1 may be directed against at least 16 consecutive bases within the sequence of SEQ ID NO: 35.

The nucleic acid ligase IV inhibitor may in some embodiments be SCR 5 or SCR 6. In some embodiments the nucleic acid ligase IV inhibitor may be SCR7. In some embodiments the nucleic acid ligase IV inhibitor may be SCR7 pyrazine or SCR 8. In some embodiments the nucleic acid ligase IV inhibitor may be SCR 9 or SCR 10. In some embodiments the nucleic acid ligase IV inhibitor may be SCR 11 or SCR 12. In some embodiments the nucleic acid ligase IV inhibitor may be SCR 13 or SCR14.

The antisense oligonucleotide against the p53-binding protein 1 may in some embodiments be directed against a portion of the sequence of SEQ ID NO: 31 or a complement thereof or against a portion of the sequence of SEQ ID NO: 32 or a complement thereof.

The inhibitor of the phosphotyrosine phosphatase activity of the protein EYA1A may in some embodiments be benzbromarone or a polyoxomolybdate phosphate complex, hexapotassium hexatriaconta-µ-oxooctadecaoxobis [µ9-[phosphato(3-)-κO:κO:κO:κO':κO':κO":κO":κO‴:κO‴]] octadeca-molybdate(6-). In some embodiments the inhibitor of the phosphotyrosine phosphatase activity of the protein EYA1A may be a salt having a cation of the formula

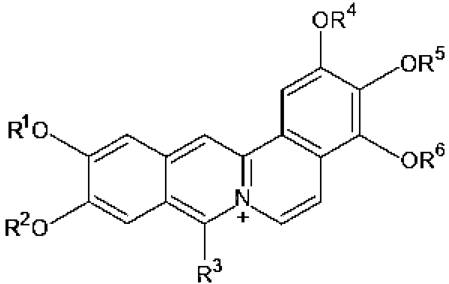

In this formula R¹ and R² are independently selected C₁-C₆ alkyl. R³ is methyl or H. R⁴, R⁵ and R⁶ are independently selected from H, methyl and ethyl.

In some embodiments the inhibitor of the phosphotyrosine phosphatase activity of the protein EYA1A may be a compound of the formula

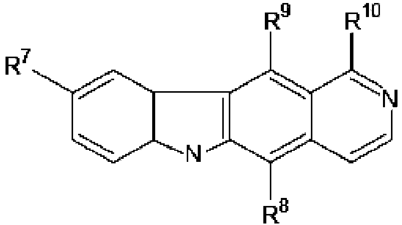

In this formula R⁷ is H, OH, C₁-C₆ alkoxy or phenoxy. R⁸ and R⁹ are independently selected from H and methyl. R¹⁰ is H, methyl, ethyl or (1-methyl-4-nitropyrazol-5-yl).

The sgRNA included in the nucleic acid molecule that includes an endonuclease 9 encoding sequence, a TET domain, and a sequence encoding an sgRNA specific for a portion of a CpG island of the gene encoding EYA1 may be specific for a sequence encoding an sgRNA specific for a portion of the sequence of one of SEQ ID NO: 6, 13, 21 or 33. Each of the endonuclease 9 encoding sequence and the TET domain included in the nucleic acid molecule are operably linked to a promoter. The promoters are independently selected from one another.

Aberrant fibroblast proliferation occurs in a parenchymal organ. The parenchymal organ is in some embodiments a kidney or a liver. In some embodiments the parenchymal organ is a heart or a lung. In some embodiments the parenchymal organ is a spleen or a pancreas.

In some embodiments aberrant fibroblast proliferation occurs in a desmoplasia, such as a cancer. The desmoplasia is in some embodiments a prostate cancer or a breast cancer. In some embodiments the desmoplasia is a desmoplastic malignant melanoma or a desmoplastic squamous cell carcinoma. In some embodiments the desmoplasia is a morpheaform basal cell carcinoma or a microcystic adnexal carcinoma. In some embodiments the desmoplasia is a cutaneous leiomyosarcoma or a cutaneous metastasis.

In some embodiments the compound used is capable of preventing or stopping impairment of organ function or histopathological patterns of chronified injury before, during, or after injury. Histopathological patterns of chronified injury include in some embodiments at least one of fibrosis and cirrhosis. In some embodiments, histopathological patterns of chronified injury include at least one of loss of functional parenchyma and atrophy.

In some embodiments the disease involving aberrant fibroblast proliferation is fibrosis. In some embodiments, the compound used is capable of at least one of preventing, treating or delaying progression to fibrosis. In some embodiments the fibrosis is cystic fibrosis or idiopathic pulmonary fibrosis. In some embodiments the fibrosis is liver cirrhosis or artrial fibrosis of the heart. In some embodiments the fibrosis is endomyocardial fibrosis or glial scar of the brain. In some embodiments the fibrosis is keloid of the skin or Crohn's disease.

The compound of the present disclosure may also be advantageously applied in the prevention, treatment, and/or in delaying progression of chronic cardiac injury. In some embodiments, the compound is for use in the treatment of prevention of diabetes mellitus, in particular diabetic end-organ damage, including diabetic nephropathy.

In some embodiments, the compound used is capable of preventing or treating chronic kidney disease. In some embodiments, the compound used is capable of preventing and treating chronic kidney disease. In some embodiments the compound used is capable of delaying onset of or delaying progression of chronic kidney disease. In some embodiments the compound used is capable of delaying onset of and of delaying progression of chronic kidney disease. In some embodiments the compound used is capable of preventing, treating, delaying onset and delaying progression of chronic kidney disease.

In some embodiments, the compound used is capable of preventing or treating chronic cardiac injury. In some embodiments, the compound used is capable of preventing and treating chronic cardiac injury. In some embodiments the compound used is capable of delaying onset of or delaying progression of chronic cardiac injury. In some embodiments the compound used is capable of delaying onset of and of delaying progression of chronic cardiac injury. In some embodiments the compound used is capable of preventing, treating, delaying onset and delaying progression of chronic cardiac injury.

In some embodiments, the compound used is capable of preventing or treating end-stage renal disease. In some embodiments, the compound used is capable of preventing and treating end-stage renal disease. In some embodiments, the compound used is capable of delaying progression to end-stage renal disease. In some embodiments the compound used is capable of preventing, treating and delaying progression to end-stage renal disease.

In some embodiments, the compound used is capable of preventing or treating pulmonary fibrosis. In some embodiments, the compound used is capable of preventing and treating pulmonary fibrosis. In some embodiments, the compound used is capable of delaying progression of pulmonary fibrosis. In some embodiments the compound used is capable of preventing, treating and delaying progression of pulmonary fibrosis.

In some embodiments, the compound used is capable of preventing or treating diabetes mellitus, including diabetic end-organ damage. In some embodiments, the compound used is capable of preventing and treating diabetes mellitus, including diabetic end-organ damage. In some embodiments the compound used is capable of delaying progression to or progression of diabetes mellitus, including diabetic end-organ damage. In some embodiments the compound used is capable of delaying progression to and progression of diabetes mellitus, including diabetic end-organ damage. In some embodiments the compound used is capable of preventing, treating, delaying progression to and progression of diabetes mellitus, including diabetic end-organ damage.

In some embodiments, the compound used is capable of preventing or treating diabetic nephropathy. In some embodiments, the compound used is capable of preventing and treating diabetic nephropathy. In some embodiments the compound used is capable of delaying progression to or progression of diabetic nephropathy. In some embodiments the compound used is capable of delaying progression to and progression of diabetic nephropathy. In some embodiments the compound used is capable of preventing, treating, delaying progression to and progression of diabetic nephropathy.

According to a second aspect, there is provided a compound that reduces the level of protein variant EYA1A in a parenchymal organ for use in a method of preventing, treating or delaying progression of fibrosis and/or desmoplasia in said parenchymal organ

The method may include administering to the subject an antisense oligonucleotide against a portion of at least 16 consecutive bases within the sequence of SEQ ID NO: 34, or a complement thereof, and/or an antisense oligonucleotide against a portion of at least 16 consecutive bases within the sequence of SEQ ID NO: 35, or a complement thereof. The antisense oligonucleotide against SEQ ID NO: 34 is directed against the splice junction of intron 9-10/exon 10 of the sequence encoding the protein EYA1. The antisense oligonucleotide against SEQ ID NO: 35 is directed against the splice junction of exon 10/intron 10-11 of the sequence encoding the protein EYA1. The sequence encoding the protein EYA1 can be found under GenBank accession number NG_011735.3. The exon termed exon 10 herein, is the twelfth exon listed in GenBank accession number NG_011735.3. The sequence of the reverse strand encoding the protein EYA1 can be found under Ensembl accession number ENSG00000104313 (Ensembl release 98 of September 2019), being the region 71,197,433-71,592,025 on human chromosome 8.

In some embodiments of the method according to the second aspect the antisense oligonucleotide against a portion of at least 16 consecutive bases within the sequence of SEQ ID NO: 34 is an antisense oligonucleotide against a sequence that includes the sequence of SEQ ID NO: 39 or a complement thereof. In some embodiments the antisense oligonucleotide against a portion of at least 16 consecutive bases within the sequence of SEQ ID NO: 34 is an antisense oligonucleotide against the sequence of SEQ ID NO: 39 or a complement thereof.

In some embodiments of the method according to the second aspect the antisense oligonucleotide against a portion of at least 16 consecutive bases within the sequence of SEQ ID NO: 34 is an antisense oligonucleotide against a sequence that includes the sequence of SEQ ID NO: 5 or a complement thereof. In some embodiments the antisense oligonucleotide against a portion of at least 16 consecutive bases within the sequence of SEQ ID NO: 34 is an antisense oligonucleotide against the sequence of SEQ ID NO: 5 or a complement thereof.

In some embodiments of the method according to the second aspect the antisense oligonucleotide against a portion of at least 16 consecutive bases within the sequence of SEQ ID NO: 35 is an antisense oligonucleotide against a sequence that includes one of the sequence of SEQ ID NO: 40, a complement thereof, a portion of the sequence SEQ ID NO: 40 or a complement of this portion. In some embodiments the antisense oligonucleotide against a portion of at least 16 consecutive bases within the sequence of SEQ ID NO: 35 is an antisense oligonucleotide against one of the sequence of SEQ ID NO: 40, a complement thereof, a portion of the sequence SEQ ID NO: 40 or a complement of this portion.

In some embodiments the antisense oligonucleotide against a portion against a sequence that includes one of the sequence of SEQ ID NO: 35, a complement thereof, a portion of the sequence SEQ ID NO: 35 or a complement of this portion is an antisense oligonucleotide against a sequence that includes the sequence of SEQ ID NO: 4, or a complement thereof. In some embodiments the antisense oligonucleotide against a portion against a sequence that includes one of the sequence of SEQ ID NO: 40, a complement thereof, a portion of the sequence SEQ ID NO: 40 or a complement of this portion is an antisense oligonucleotide against a sequence that includes the sequence of SEQ ID NO: 4, or a complement thereof.

In some embodiments of the method according to the second aspect the antisense oligonucleotide is a phosphorodiamidate morpholino oligonucleotide. In some embodiments the antisense oligonucleotide against at least a portion of SEQ ID NO: 39 and/or against at least a portion of SEQ ID NO: 35 furthermore includes a covalently linked octaguanidine dendrimer. In some embodiments the antisense oligonucleotide against SEQ ID NO: 5 and/or against SEQ ID NO: 4 furthermore includes a covalently linked octaguanidine dendrimer.

In some embodiments the subject expresses an EYA1 protein that includes the sequence of SEQ ID NO: 2. In some embodiments the subject expresses an EYA1 protein that includes the sequence of SEQ ID NO: 3. In some embodiments the subject expresses an EYA1 protein that includes the sequence of SEQ ID NO: 11. In some embodiments the subject expresses an EYA1 protein that includes the sequence of SEQ ID NO: 12. In some embodiments the subject expresses a naturally occurring EYA1 protein that includes the sequence of SEQ ID NO: 2. In some embodiments the subject expresses a naturally occurring EYA1 protein that includes the sequence of SEQ ID NO: 3. In some embodiments the subject expresses a naturally occurring EYA1 protein that includes the sequence of SEQ ID NO: 11. In some embodiments the subject expresses a naturally occurring EYA1 protein that includes the sequence of SEQ ID NO: 12. In some embodiments the subject expresses a naturally occurring EYA1 protein that includes the sequence of SEQ ID NO: 3. In some embodiments the subject expresses the human EYA1 protein. In some embodiments the subject is a human. In some embodiments the subject is a mouse or a rat. The mouse or the rat may express the human EYA1 protein that includes the sequence of SEQ ID NO: 2. The mouse or the rat may express the human EYA1 protein that includes the sequence of SEQ ID NO: 3. In some embodiments the mouse or the rat may express the human EYA1 protein that includes the sequence of SEQ ID NO: 11. The mouse or the rat may in some embodiments express the human EYA1 protein that includes the sequence of SEQ ID NO: 12.

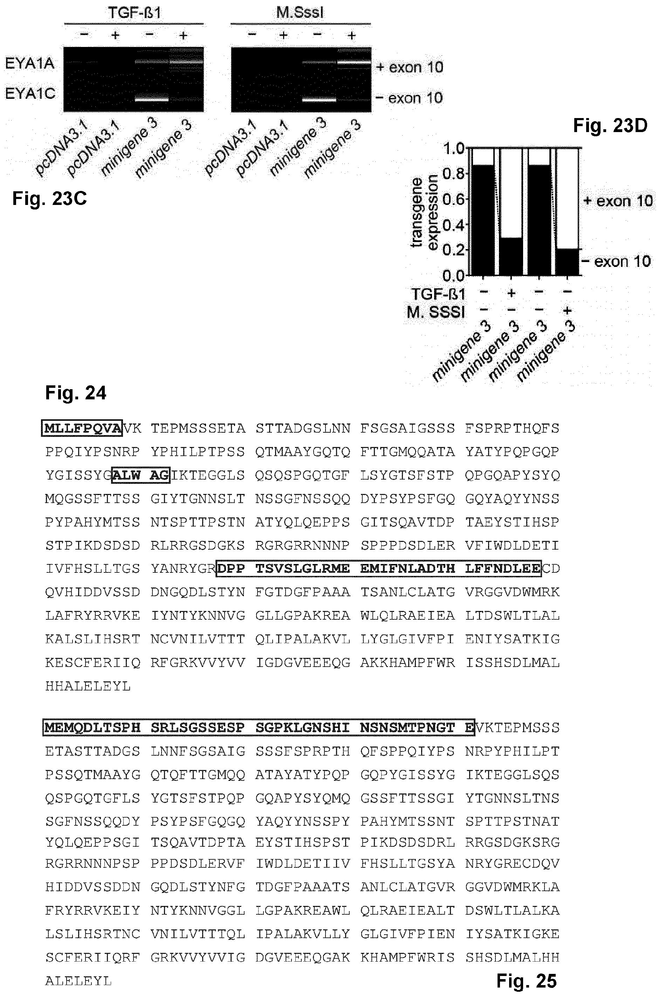

According to a third aspect, there is provided a a compound that reduces the level of protein variant EYA1A in a parenchymal organ for use in a method of preventing, treating or delaying progression of fibrosis and/or desmoplasia in said parenchymal organ in a subject. The method may include administering to the subject an antisense oligonucleotide against the sequence of SEQ ID NO: 36 or a complement thereof. The antisense oligonucleotide inhibits translation of isoform A of the protein EYA1. Isoform A of the protein EYA1 is defined by the amino acid sequence of SEQ ID NO: 1, depicted in Fig 24. In some embodiments the antisense oligonucleotide is an antisense oligonucleotide against SEQ ID NO: 9 or a complement thereof.

In some embodiments the method according to the third aspect includes administering to the subject an antisense oligonucleotide antisense oligonucleotide against a portion of at least 16 consecutive bases within the sequence of SEQ ID NO: 34, or a complement thereof, and/or an antisense oligonucleotide against a portion of at least 16 consecutive bases within the sequence of SEQ ID NO: 35, or a complement thereof. In some embodiments the method according to the third aspect includes administering to the subject an antisense oligonucleotide antisense oligonucleotide against a portion of at least 16 consecutive bases within the sequence of SEQ ID NO: 39, or a complement thereof, and/or an antisense oligonucleotide against a portion of at least 16 consecutive bases within the sequence of SEQ ID NO: 40, or a complement thereof. In some embodiments the method according to the third aspect includes administering to the subject an antisense oligonucleotide against SEQ ID NO: 5, or a complement thereof, and/or an antisense oligonucleotide against SEQ ID NO: 4, or a complement thereof.

In some embodiments of the method according to the third aspect the antisense oligonucleotide is a phosphorodiamidate morpholino oligonucleotide. In some embodiments the antisense oligonucleotide against SEQ ID NO: 9 furthermore includes a covalently linked octaguanidine dendrimer.

In some embodiments the subject expresses an EYA1 protein that includes the sequence of SEQ ID NO: 2. In some embodiments the subject expresses an EYA1 protein that includes the sequence of SEQ ID NO: 3. In some embodiments the subject expresses an EYA1 protein that includes the sequence of SEQ ID NO: 11. In some embodiments the subject expresses an EYA1 protein that includes the sequence of SEQ ID NO: 12. In some embodiments the subject expresses a naturally occurring EYA1 protein that includes the sequence of SEQ ID NO: 2. In some embodiments the subject expresses a naturally occurring EYA1 protein that includes the sequence of SEQ ID NO: 3. In some embodiments the subject expresses a naturally occurring EYA1 protein that includes the sequence of SEQ ID NO: 11. In some embodiments the subject expresses a naturally occurring EYA1 protein that includes the sequence of SEQ ID NO: 12. In some embodiments the subject expresses the human EYA1 protein. In some embodiments the subject is a human. In some embodiments the subject is a mouse or a rat. The mouse or the rat may express an EYA1 protein that includes the sequence of SEQ ID NO: 2. The mouse or the rat may express an EYA1 protein that includes the sequence of SEQ ID NO: 3. The mouse or the rat may express an EYA1 protein that includes the sequence of SEQ ID NO: 2. The mouse or the rat may express an EYA1 protein that includes the sequence of SEQ ID NO: 11. The mouse or the rat may express an EYA1 protein that includes the sequence of SEQ ID NO: 12. The EYA1 protein that includes the sequence of at least one of SEQ ID NO: 2, SEQ ID NO: 3, SEQ ID NO: 11 and SEQ ID NO: 12 may be a heterologous protein. The EYA1 protein that includes the sequence of at least one of SEQ ID NO: 2, SEQ ID NO: 3, SEQ ID NO: 11 and SEQ ID NO: 12 may be a recombinant protein.

The mouse or the rat may express a naturally occurring human EYA1 protein that includes the sequence of SEQ ID NO: 2. The mouse or the rat may express a naturally occurring human EYA1 protein that includes the sequence of SEQ ID NO: 3. The mouse or the rat may in some embodiments express the human EYA1 protein that includes the sequence of SEQ ID NO: 11. The mouse or the rat may in some embodiments express the human EYA1 protein that includes the sequence of SEQ ID NO: 12. The respective EYA1 protein may be a heterologous protein. The respective EYA1 protein may in some embodiments be a recombinant protein.

### BRIEF DESCRIPTION OF THE DRAWINGS

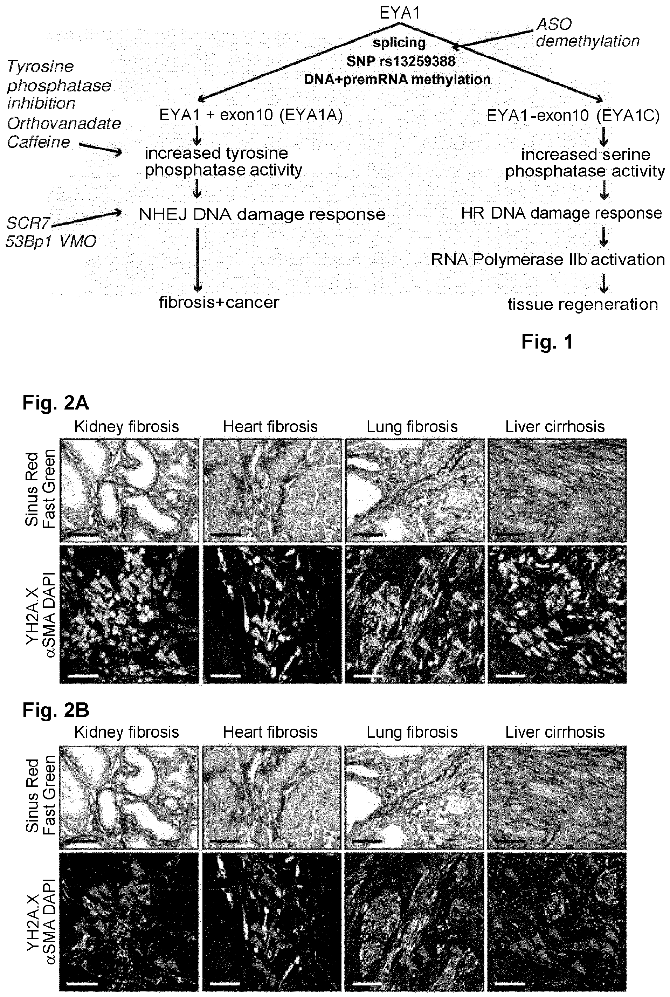

**Figure 1** is a schematic illustration on effects thought to be underlying a method disclosed herein. Alternative splicing of EYA1 leads to the formation of isoform EAY1A or EAY1C. The formation of isoform EAY1A is to be avoided, or the consequences thereof to be alleviated or reduced. Administering antisense-oligonucleotides (ASO) and/or demethylation of a site at intron 9-10 of an EAY1 precursor nucleic acid molecule can lead to the formation of isoform EAY1C instead of isoform EAY1A. Identifying the presence of SNP rs1325938 can be used for the prediction of the risk of occurrence of fibrosis and cancer. Inhibiting unfavourable tyrosine phosphatase activity of EYA1A, for instance by administering orthovanadate, reduces the occurrence of fibrosis. NHEJ, caused by EYA1A abundance, can be inhibited by using the ligase IV inhibitor SCR7 or by administering morpholinos targeting 53Bp1.

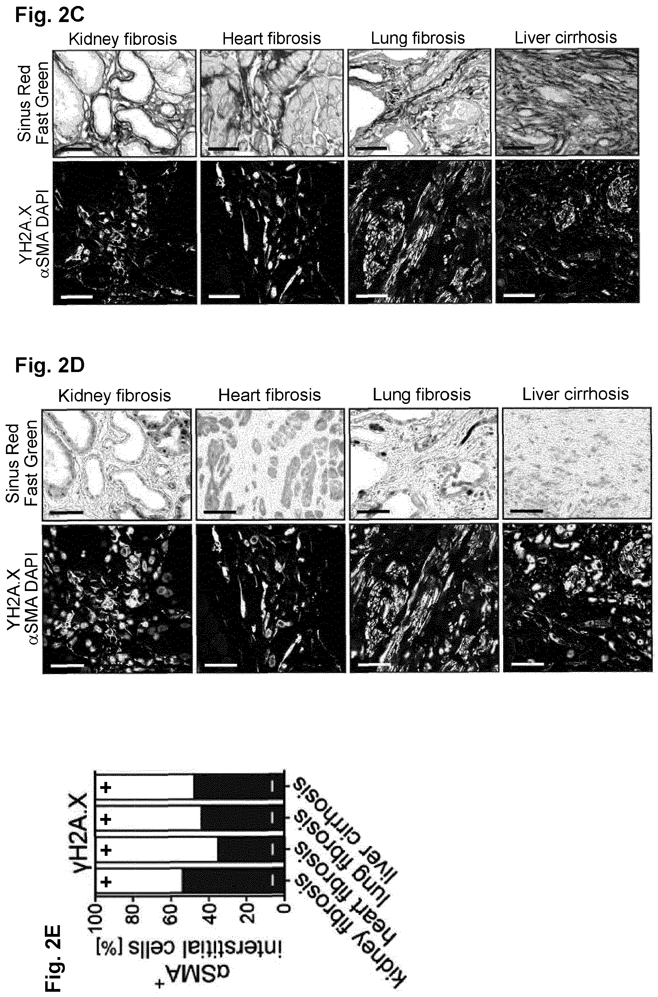

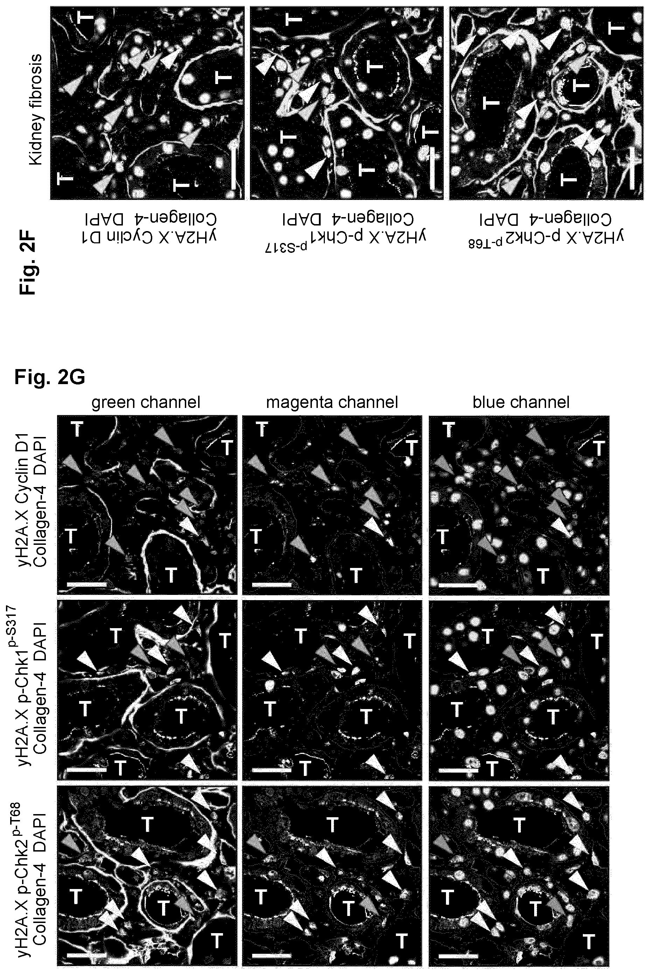

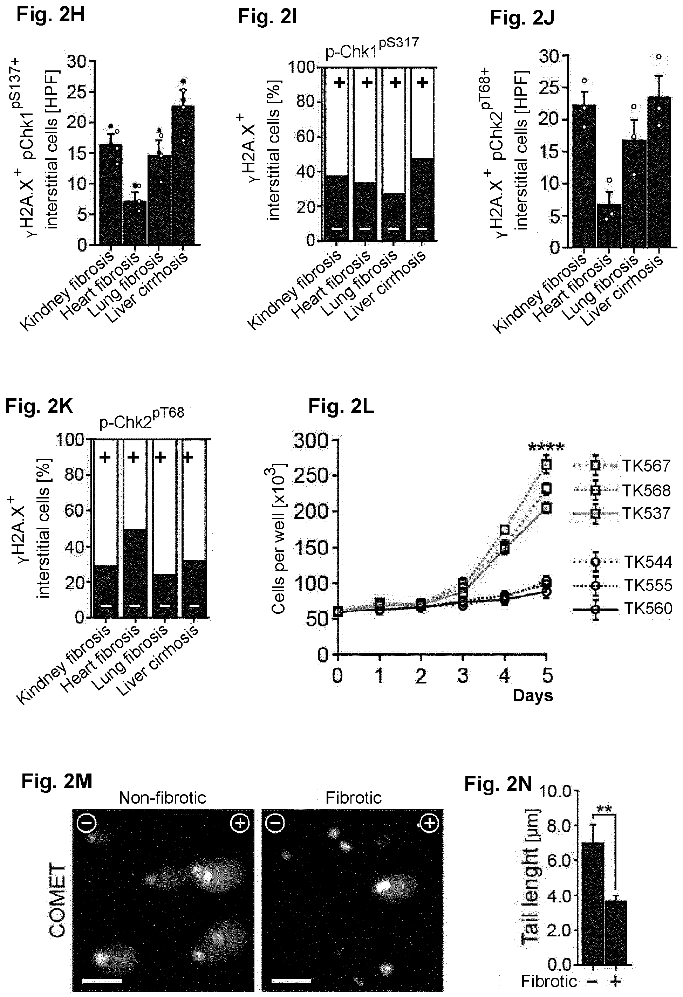

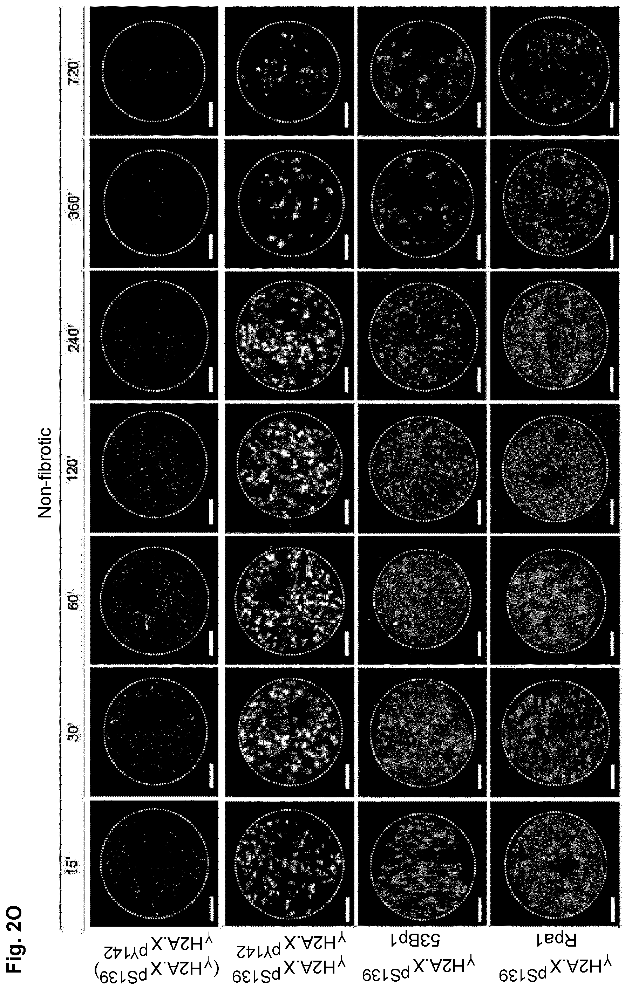

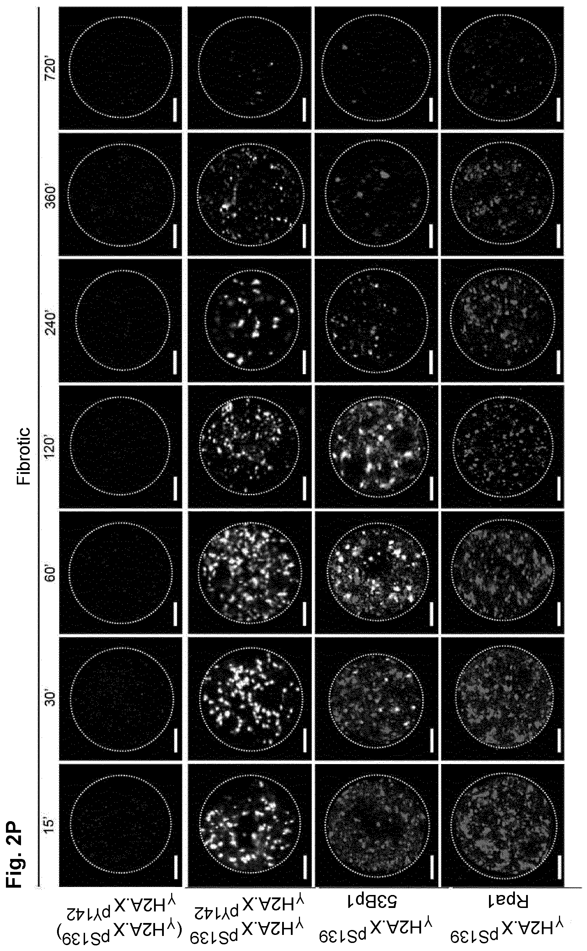

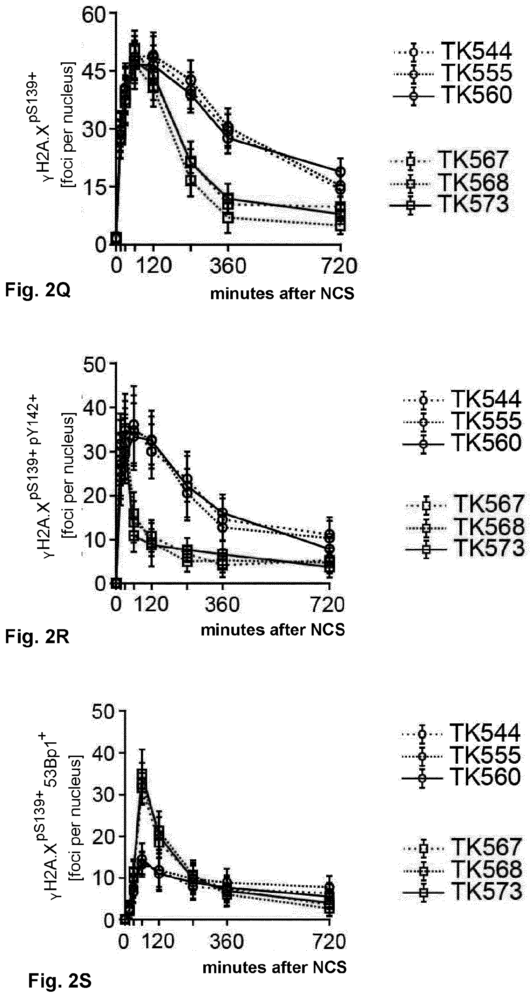

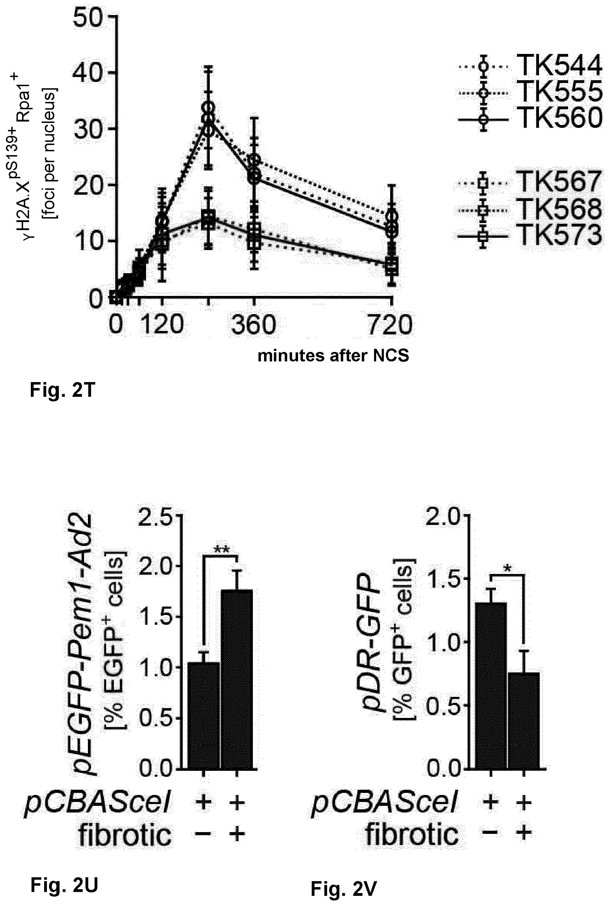

**Figure 2** illustrates that fibrotic fibroblasts resist genotoxic stress by accelerated recruitment of high-fidelity NHEJ DNA damage response. **Fig. 2A** shows representative photomicrographs of kidney, heart, lung and liver sections labelled for Sirius Red/Fast green (scale bars: 50 µm) and γH2A.X/alpha-smooth muscle actin (αSMA) (red/magenta, scale bars: 25 µm), as well as DAPI nucleic acid stain (blue). **Fig. 2B** shows the same photomicrographs as Fig. 2A with signals in blue colour and magenta colour reduced to 0, thereby effectively leaving signals not resulting from stain, green and red colour. **Fig. 2C** shows the same photomicrographs as Fig. 2A with signals in green colour and blue colour reduced to 0, thereby effectively leaving signals not resulting from stain, green and magenta colour. **Fig. 2D** shows the same photomicrographs as Fig. 2A with signals in blue colour and magenta colour reduced to 0, thereby effectively leaving signals not resulting from stain, green and red colour. The graph in **Fig. 2E** summarizes relative αSMA+ cells positive for γH2A.X (n=3 in each group). **Fig. 2F** shows representative photomicrographs of kidney sections labelled for γH2A.X/Cyclin D1/Collagen-4 (red/magenta, scale bars: 25 µm), γH2A.X/p-Chk1^{pS317}/Collagen-4 (red/magenta/green, scale bars: 25 µm) and γH2A.X/p-Chk2^{pT68}/ Collagen-4 (red/magenta/green, scale bars: 25 µm), as well as DAPI nucleic acid stain (blue). **Fig. 2G** shows the same photomicrographs as Fig. 2F with all colour signals other than green colour (left), magenta colour (center), and blue colour (right) reduced to 0. The graphs in **Figures 2H to 2K** summarize average and relative γH2A.X+ interstitial cells positive for p-Chk1^{pS317} or p-Chk2^{pT68} (T=tubules, n=3 in each group). **Fig. 2L** depicts the accumulation of human fibroblasts isolated from fibrotic kidney biopsies (TK567, TK568, and TK573) in comparison to primary fibroblasts from non-fibrotic kidneys (TK544, TK555, and TK560) in culture. Primary human kidney fibroblast cultures were seeded at a density of 60,000 per well and proliferative activity was determined at indicated time points (n=3 independent replicates, data are presented as means±s.d., **** p<0.0001, values of p were calculated using Student's t test comparing non-fibrotic and fibrotic fibroblast cultures). **Fig. 2M** shows fibroblasts isolated from non-fibrotic and fibrotic kidneys analyzed by neutral COMET assay (scale bars: 10 µm). **Fig. 2N** is a graph summarizing average tail lenghts of fibroblasts analyzed by neutral COMET assay (cf. Fig 2M, n=3 in each group, data are presented as means±s.d., ** p<0.01, values of p were calculated using Student's t test comparing indicated groups). **Figures 2O** and **2P** show representative photomicrographs of primary human fibroblast cultures isolated from non-fibrotic kidneys **(****Fig. 2O****)** and fibrotic kidneys **(****Fig. 2P****)** at indicated time points after NCS exposure. The respective upper two panels show photomicrographs of fibroblast cultures labelled for γH2A.X^{pS139}/γH2A.X^{pY142} (scale bars: 4 µm). The top panel shows the same photomicrographs as the panel below, with any other colour signals than green colour reduced to 0. The lower two panels show photomicrographs of fibroblast cultures labelled for γH2A.X^{pS139}/53Bp1 (penultimate panel, scale bars: 4 µm) and γH2A.X^{pS139}/Rpa1 (bottom panel, scale bars: 4 µm). **Figures 2Q** to **2T** are graphs summarizing average foci per nucleus of primary human fibroblast cultures, of which Figs. 2O and 2P show representative images (n=100 nuclei each, data are presented as means±s.d.). **Figures 2U** and **2V** depict an analysis of NHEJ and HR events upon DNA damage using pPEGFP-Pem1-Ad2 (NHEJ) and pDR-GFP (HR) reporter plasmids (n=3 independent replicates, data are presented as means±s.d., * p<0.05, ** p<0.01, values of p were calculated using Student's t test comparing indicated groups). Significantly increased NHEJ pEGFP-Pem1-Ad2 and reduced HR pDR-GFP reporter activities can be seen in fibrotic fibroblasts.

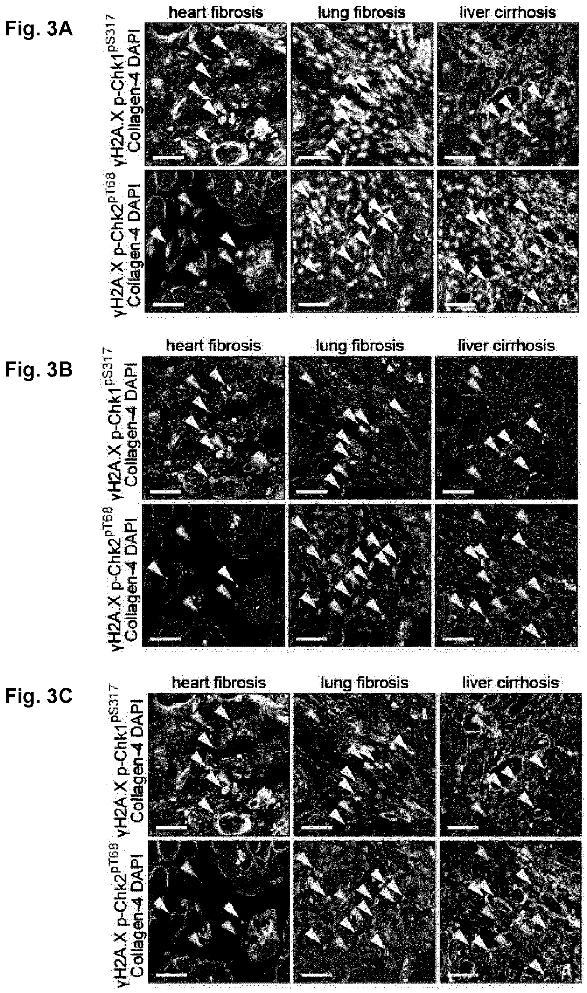

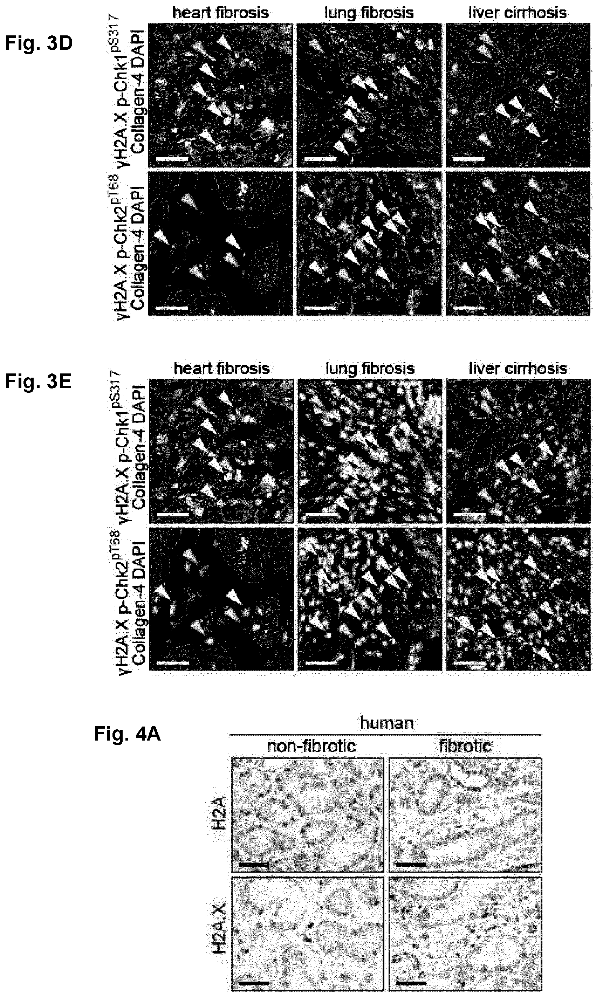

**Figure 3A** shows representative photomicrographs of heart and liver sections labelled for γH2A.X/p-Chk1^{pS317}/Collagen-4 (scale bars: 25 µm) and γH2A.X/p-Chk2^{pT68}/Collagen-4 (scale bars: 25 µm). **Fig. 3B** shows the same photomicrographs as Fig. 3A with all colour signals reduced to 0, thereby effectively leaving signals not resulting from stain. **Fig. 3C** shows the same photomicrographs as Fig. 3A with signals of all other colours than green reduced to 0. **Fig. 3D** shows the same photomicrographs as Fig. 3A with signals of all other colours than magenta reduced to 0. **Fig. 3E** shows the same photomicrographs as Fig. 3A with signals of all other colours than blue reduced to 0.

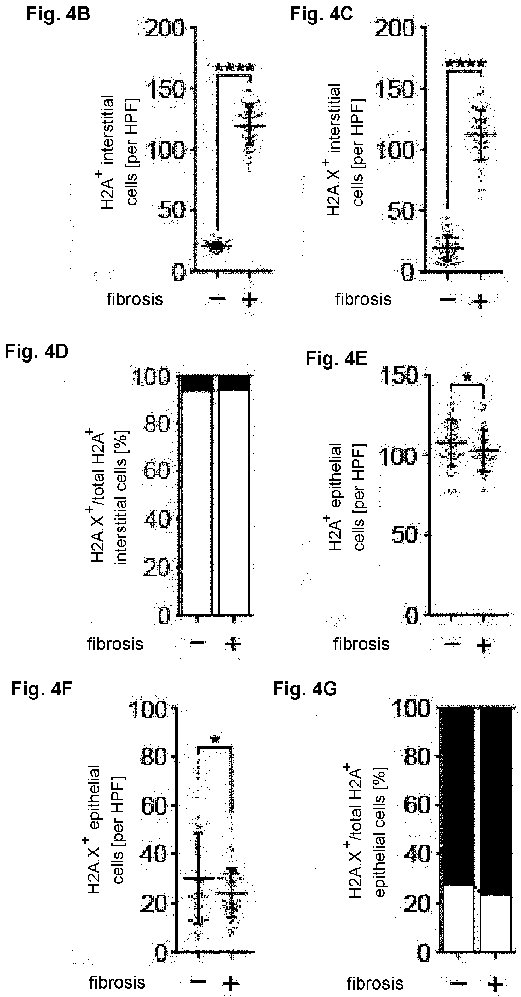

**Figure 4A** shows representative photomicrographs of human kidney sections that were labelled for H2A (scale bars: 50 µm) or H2A.X (scale bars: 50 µm). The graphs in **Figures 4B to 4G** summarize the respectively labelled cells (n=3, measurements were done in 10 visual fields, data are presented as means±s.d., * p<0.05, ** p<0.01, **** p<0.0001, values of p were calculated using Student's t test comparing indicated groups).

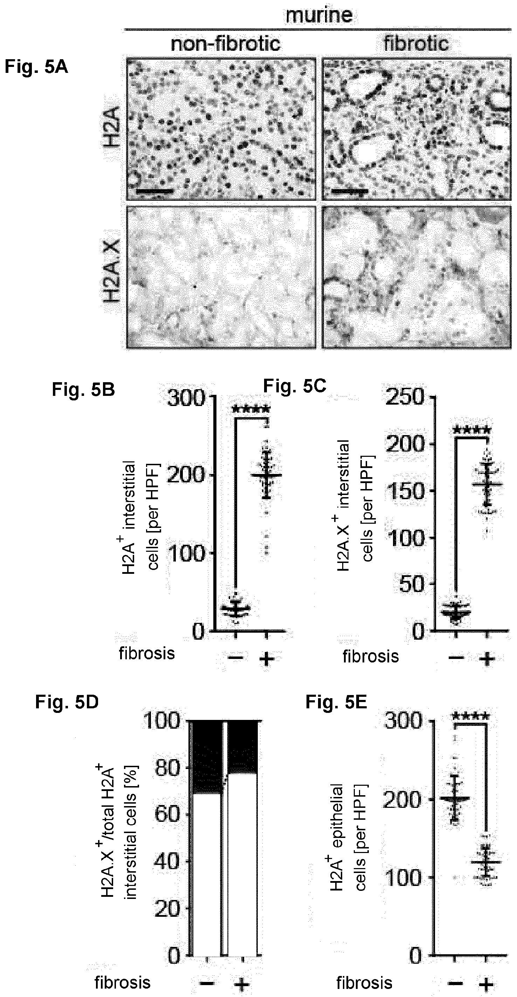

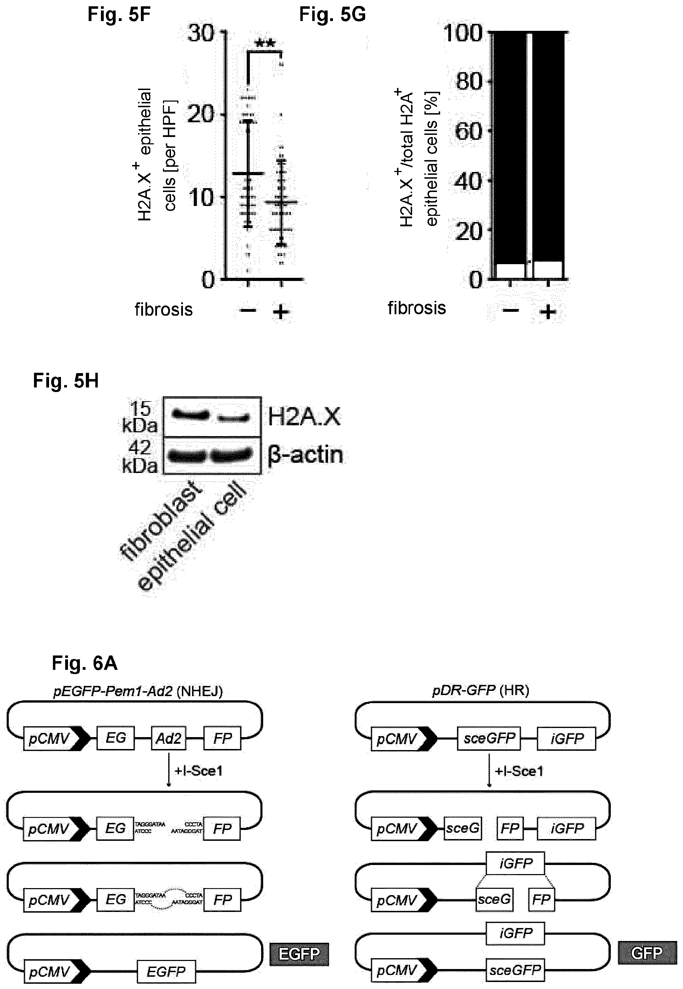

**Figure 5A** shows representative photomicrographs of murine kidney sections, labelled for H2A (scale bars: 50 µm) or H2A.X (scale bars: 50 µm). The graphs in **Figures 5B to 5G** summarize the respectively labelled cells (n=3, measurements were done in 10 visual fields, data are presented as means±s.d., * p<0.05, ** p<0.01, **** p<0.0001, values of p were calculated using Student's t test comparing indicated groups).
**Figure 5H** shows SDS-PAGE and subsequent immunoblotting of H2A.X levels in human fibroblast cultures (TK173) as compared to renal epithelial cells (HK2).
**Figure 6A** depicts schematic illustrations of reporter plasmids for NHEJ in which the *EGFP* gene is inactivated by the presence of the *Pem1 intron* and an *Ad2 exon* flanked by I-Sce1 recognition sites (*pEGFP-Pem1-Ad2*) or HR harbouring I-Sce1 recognition sites within the *GFP* gene and inactive *iGFP* matrices for HR (*pDR-GFP*).

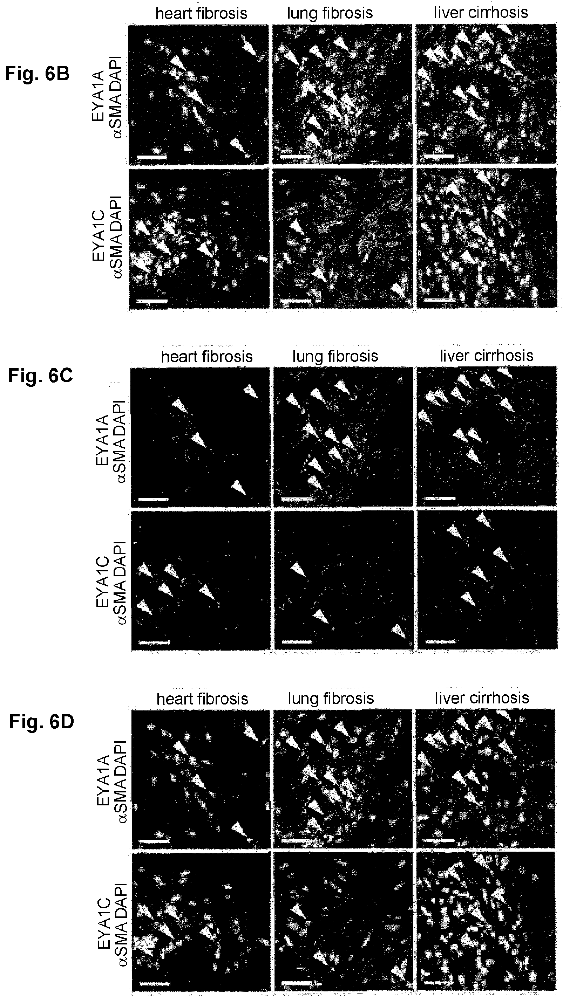

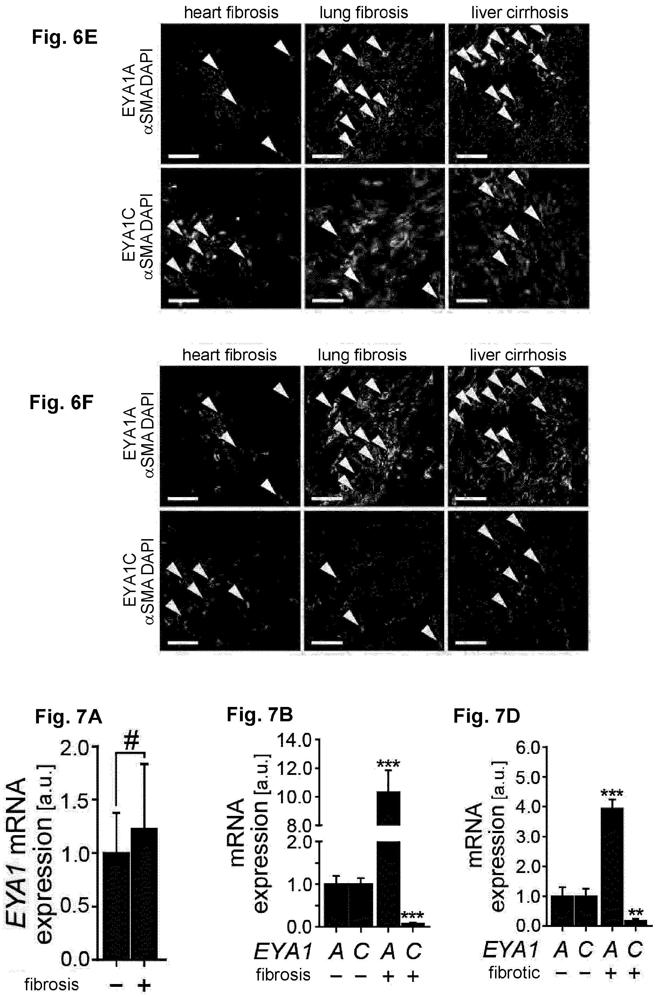

**Figure 6B** shows representative immunostainings of EYA1A/αSMA (red/green scale bars: 25 µm) and EYA1C/αSMA (green/red, scale bars: 25 µm), both including DAPI nucleic acid stain (blue), within fibrotic hearts, lungs and cirrhotic livers. **Fig. 6C** shows the same photomicrographs as Fig. 6B with all colour signals reduced to 0, thereby effectively leaving signals not resulting from stain. **Fig. 6D** shows the same photomicrographs as Fig. 6B with all other signals than signals in blue colour reduced to 0. **Fig. 6E** shows the same photomicrographs as Fig. 6B with all other signals than signals in red colour reduced to 0. **Fig. 6F** shows the same photomicrographs as Fig. 6B with all other signals than signals in green colour reduced to 0.

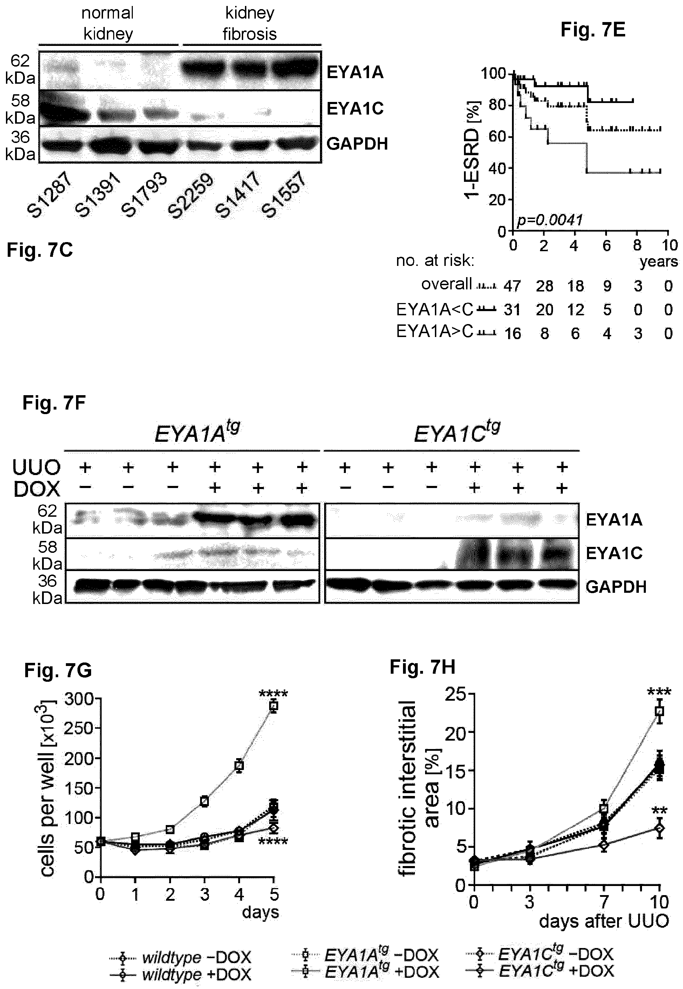

**Figure 7** illustrates that EYA1 isoforms determine DDR cell fate decisions and CKD progression. **Figures 7A and 7B** depict results of qRT-PCR analysis. The graphs reflect relative mRNA expression levels of total EYA1 and EYA1 isoforms EYA1A and EYA1C (n=3 in each group, data are presented as means±s.d., *** p<0.001, # no significance, values of p were calculated using Student's t test comparing fibrosis with no fibrosis). **Fig. 7C** depicts results of gelectrophoretic analysis, where (EYA1A and EYA1C were exposed to SDS-PAGE and subsequent immunoblotting in normal (S1287, S1391 and S1793) and fibrotic kidneys (S2259, S1417 and S1557). **Fig. 7D** shows an analysis by qRT-PCR of EYA1 isoform expression in primary fibroblast cultures isolated from non-fibrotic and fibrotic kidneys (n=3 in each group, data are presented as means±s.d., ** p<0.01, *** p<0.001, values of p were calculated using Student's t test comparing fibrotic with non-fibrotic fibroblasts). **Figure 7E** depicts kidney disease progression in patients with predominant EYA1A (EYA1A>EYA1C) or EYA1C (EYA1A<EYA1C) abundance within renal fibroblasts (n=47, values of p were calculated using log-rank/Mantel-Cox test comparing EYA1A>EYA1C and EYA1A<EYA1C). **Figure 7F** depicts a gelectrophoretic analysis of overexpression by SDS-PAGE and immunoblotting, after *wildtype* mice and mice harbouring DOX-inducible transgenes for EYA1A (*rtTAhCMV;hEYA1A-pTreTight,* referred as EYA1A^{tg}) or EYA1C (rtTAhCMV;hEYA1C-pTreTight, referred as EYA1C^{tg}) were challenged with unilateral ureteral obstruction (UUO), EYA1A and EYA1C. **Figure 7G** depicts the proliferative activity of primary murine fibroblasts from wildtype, EYA1A^{tg} or EYA1C^{tg} mice, seeded at a density of 60,000 per well, at indicated time points (n=3 independent replicates, data are presented as means±s.d., **** p<0.0001, values of p were calculated using Student's t test comparing DOX-treated EYA1Atg or EYA1C^{tg} cultures with corresponding uninduced fibroblasts). **Figure 7H** depicts the relative fibrotic interstitial area at indicated time points after ureteral obstruction of fibroblasts from wildtype, EYA1A^{tg} or EYA1C^{tg} mice (** p<0.01, *** p<0.001, values of p were calculated using Student's t test comparing DOX-treated wildtype, EYA1A^{tg} or EYA1C^{tg} mice with corresponding uninduced littermates).

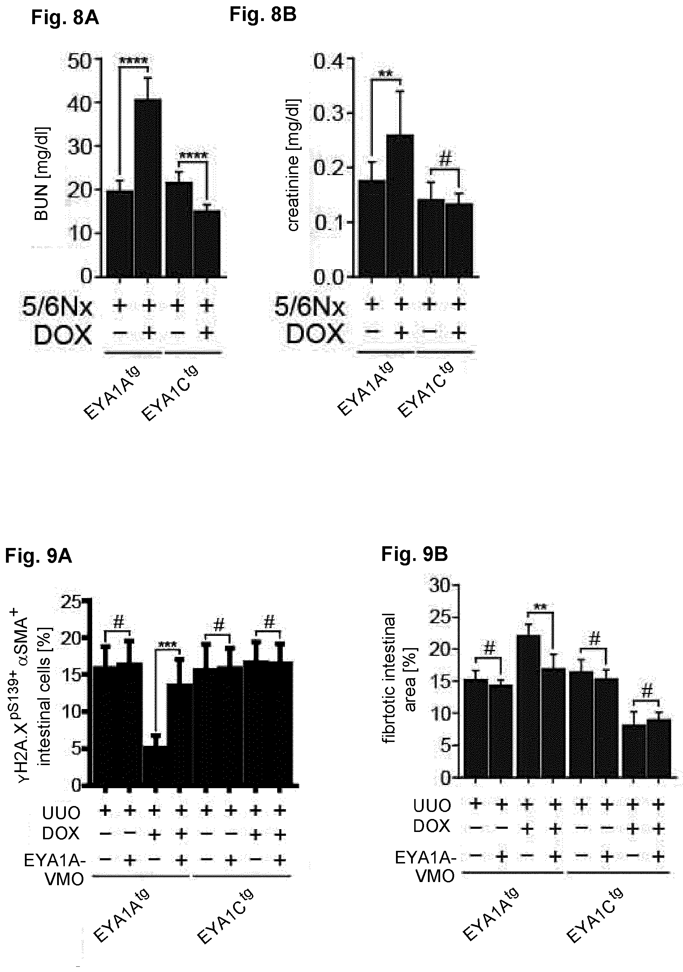

**Figures 8A and 8B** summarize average blood urea nitrogen (BUN) and plasma creatinine measurements in mice harbouring DOX-inducible transgenes for EYA1A (EYA1A^{tg}) or EYA1C (EYA1C^{tg}) 4 weeks after nephrectomy (n=10 in each group, data are presented as means±s.d., ** p<0.01, **** p<0.0001, # no significance, values of p were calculated using Student's t test comparing indicated groups).

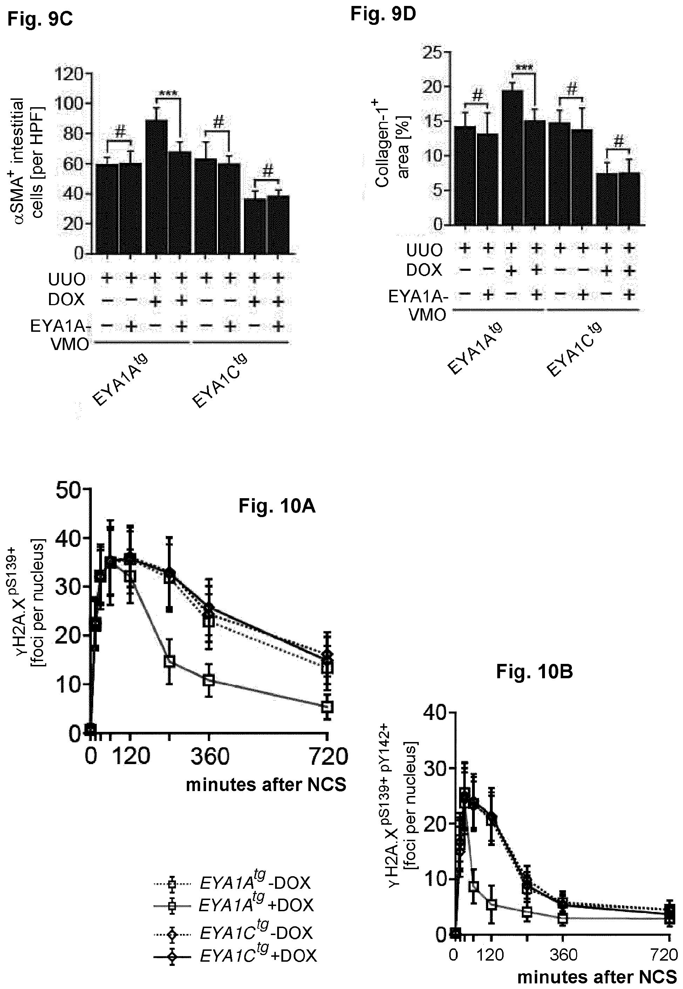

**Figure 9A** depicts relative αSMA⁺ cells positive for γH2A.X^{pY142} in kidney sections of wildtype mice and EYA1A^{tg} or EYA1C^{tg} mice 10 days after ureteral obstruction, treated with control or *in vivo* morpholinos specifically inhibiting EYA1A translation (EYA1A-VMO) (n=6 in each group, data are presented as means±s.d., ** p<0.01, *** p<0.001, # no significance, values of p were calculated using Student's t test comparing indicated groups). **Figure 9B** depicts the relative fibrotic interstitial area, **Figure 9C** **depicts** the average interstitial cells positive for αSMA, and **Figure 9D** the relative area positive for Collagen-1 of the same cells as in Fig. 9A. Data are obtained on the basis of labelling the kidney sections for EYA1A, EYA1C, γH2A.X^{pY142}, αSMA, MTS, αSMA or Collagen-1.

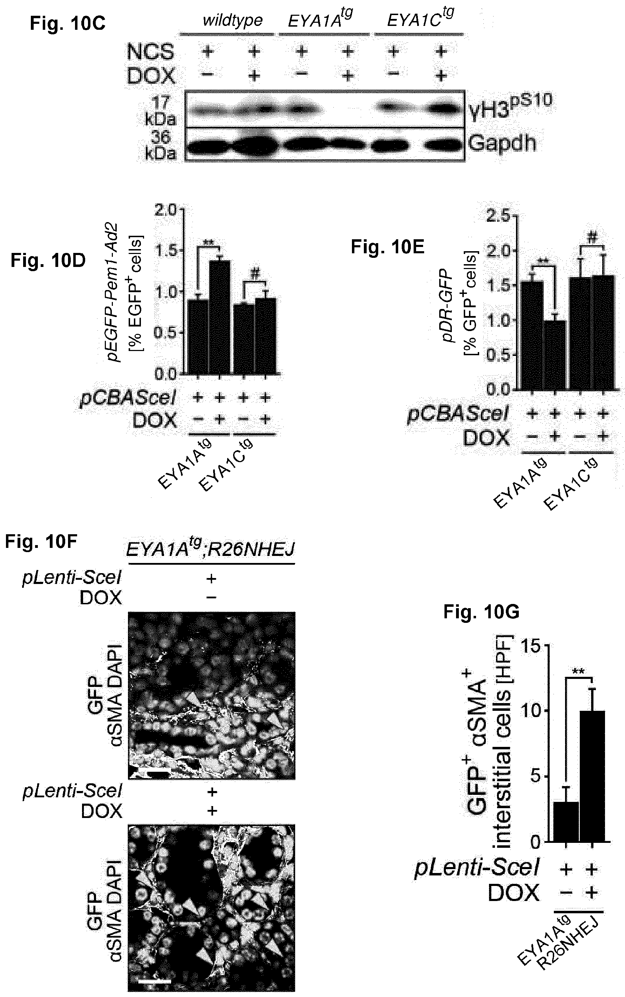

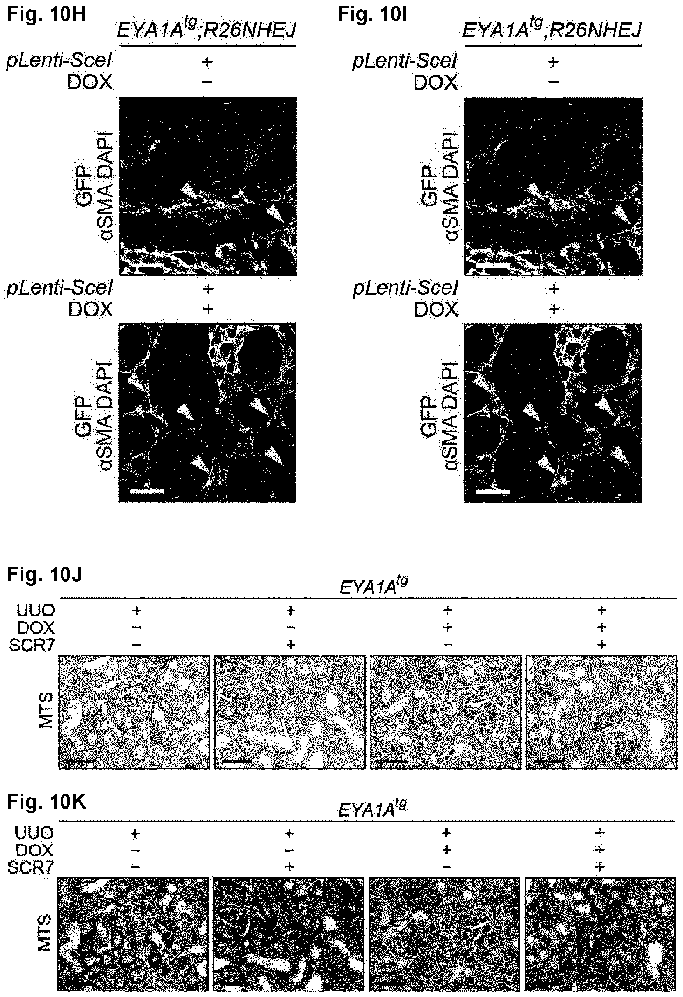

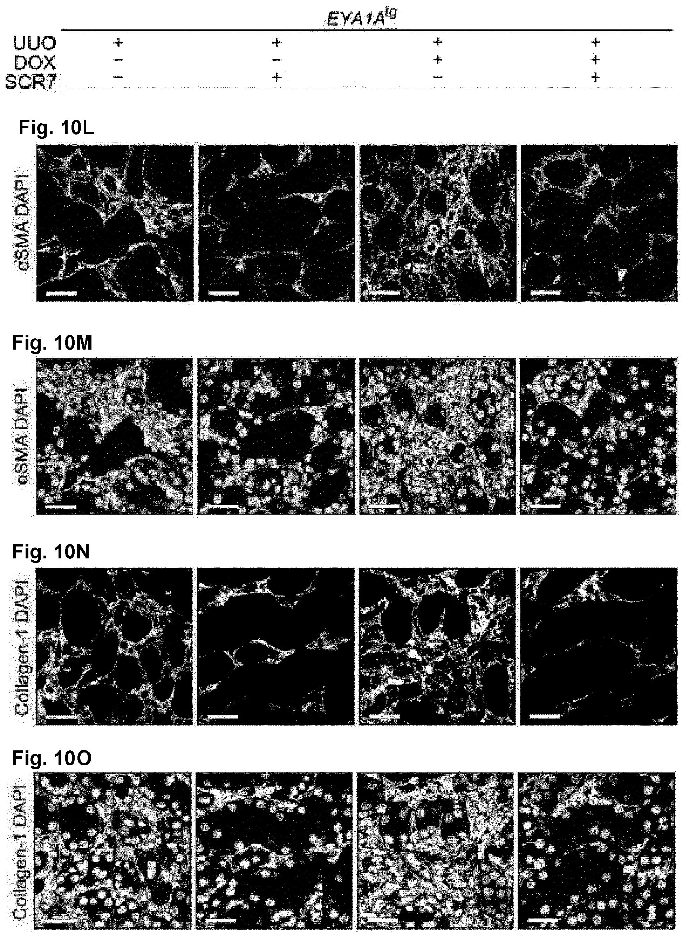

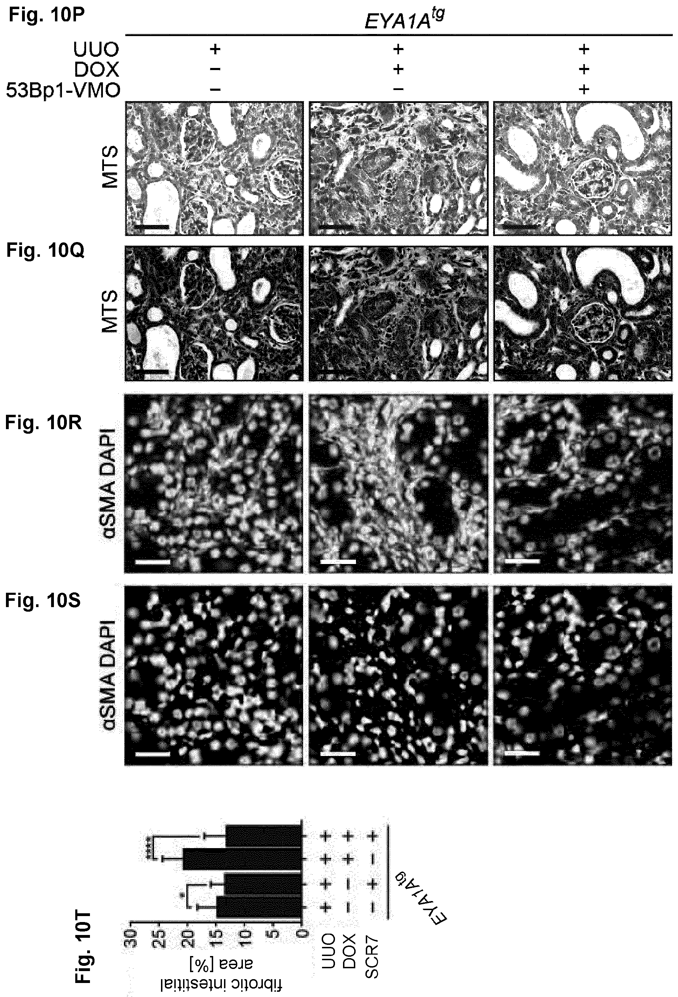

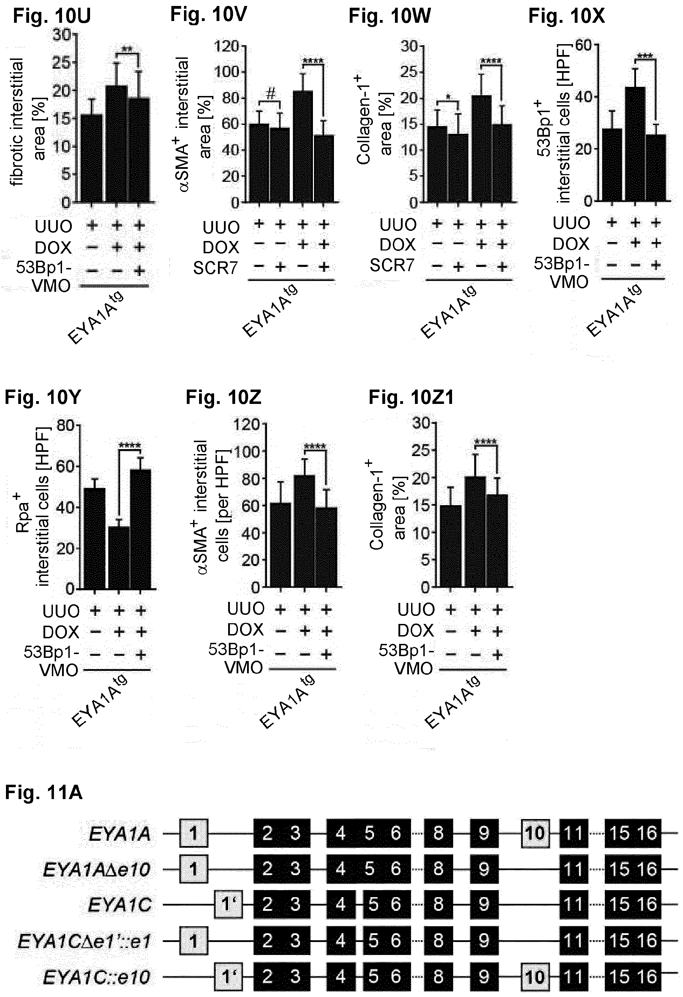

**Figure 10** illustrates that high-fidelity NHEJ causally contributes to fibrosis and fibroblast accumulation. **Figure 10A** depicts average foci per nucleus (n=100 nuclei, data are presented as means±s.d.) of primary murine EYA1A^{tg} or EYA1C^{tg} fibroblast cultures labelled for γH2A.X^{pS139} at indicated time points after NCS exposure. **Figure 10B** depicts average foci per nucleus (n=100 nuclei, data are presented as means±s.d.) of primary murine EYA1A^{tg} or EYA1C^{tg} fibroblast cultures labelled for γH2A.XpY¹⁴² at indicated time points after NCS exposure. **Figure 10C** depicts results of gelectrophoretic analysis, where γH3pS10 was analyzed by SDS-PAGE and subsequent immunobloting 24 hours after NCS exposure. **Figure 10D and 10E** show an analysis of NHEJ and HR events upon DNA damage using pEGFP-Pem1-Ad2 (NHEJ, Fig. 10D) and pDR-GFP (HR, Fig. 10E) reporter plasmids (n=3 independent replicates, data are presented as means±s.d., ** p<0.01, # no significance, values of p were calculated using Student's t test comparing indicated groups). **Figure 10F** shows representative photomicrographs of kidney sections labelled for GFP/αSMA (scale bars: 25 µm),as well as stained with DAPI, of transgenic *EYA1A^{tg}* mice harbouring NHEJ reporter transgenes (*EYA1A^{tg}*;*R26NHEJ*) were challenged with UUO and locally injected with intraparenchymal *pLenti-SceI.* **Figure 10G** summarizes average αSMA⁺ cells of the kidney sections described for Fig. 10F, positive for GFP, indicating NHEJ events (n=3 in each group, data are presented as means±s.d., ** p<0.01, values of p were calculated using Student's t test comparing indicated groups). **Figure 10H** shows the same photomicrographs as Fig. 10F with all colour signals reduced to 0, thereby effectively leaving signals not resulting from stain. **Figure 10I** shows the same photomicrographs as Fig. 10F with colour signals in blue colour reduced to 0, thus removing signals of DAPI stain. **Figure 10J** shows representative photomicrographs of kidney sections of transgenic EYA1A^{tg} mice challenged with UUO and treated with DNA ligase IV inhibitor SCR7 (20 mg/kg i.p. every alternate day), labelled for MTS (scale bars: 50 µm) 10 days after ureteral obstruction. **Figure 10K** shows the same photomicrographs as Fig. 10J with all colour signals reduced to 0, thereby effectively leaving signals not resulting from stain. **Figure 10L** shows representative photomicrographs of kidney sections of transgenic EYA1A^{tg} mice challenged with UUO and treated with DNA ligase IV inhibitor SCR7 (20 mg/kg i.p. every alternate day), labelled for αSMA (scale bars: 50 µm) 10 days after ureteral obstruction. **Figure 10M** shows the same photomicrographs as Fig. 10L with DAPI signals, which were removed in Fig 10L by reducing blue colour signals to 0. **Figure 10N** shows representative photomicrographs of kidney sections of transgenic EYA1A^{tg} mice challenged with UUO and treated with DNA ligase IV inhibitor SCR7 (20 mg/kg i.p. every alternate day), labelled for Collagen-1 (scale bars: 50 µm) 10 days after ureteral obstruction. **Figure 10O** shows the same photomicrographs as Fig. 10L with DAPI signals, which were removed in Fig 10L by reducing blue colour signals to 0. **Figure 10P** shows representative photomicrographs of kidney sections of transgenic EYA1A^{tg} mice challenged with UUO and treated with *in vivo* morpholinos blocking 53Bp1 translation (53Bp1-VMO), labelled for MTS (scale bars: 50 µm) 10 days after ureteral obstruction. **Figure 10Q** shows the same photomicrographs as Fig. 10L with all colour signals reduced to 0, thereby effectively leaving signals not resulting from stain. **Figure 10R** shows representative photomicrographs of kidney sections of transgenic EYA1A^{tg} mice challenged with UUO and treated with *in vivo* morpholinos blocking 53Bp1 translation (53Bp1-VMO), labelled for alpha-smooth muscle actin (αSMA) (scale bars: 50 µm) 10 days after ureteral obstruction, and stained with DAPI. **Figure 10S** shows the same photomicrographs as Fig. 10R with red colour signals reduced to 0, thereby effectively leaving signals not resulting from stain and DAPI stain. **Figure 10T** **and** **10U** summarize the relative fibrotic interstitial area of the kidney sections of which images are depicted in Figures 10L to 10O, and Fig. 10P to 10S, respectively (n=6 in each group, data are presented as means±s.d., * p<0.05, ** p<0.01, **** p<0.0001, values of p were calculated using Student's t test comparing indicated groups). **Figure 10V and 10W** show data of mice harbouring DOX-inducible transgenes for EYA1A (*EYA1A^{tg}*)*,* challenged with UUO and treated with DNA ligase IV inhibitor SCR7 (20 mg/kg i.p. every alternate day), representative photomicrographs of kidney sections labelled for alpha-smooth muscle actin (αSMA) (Fig. 10W, scale bars: 25 µm) or Collagen-1 (Fig. 10X, scale bars: 25 µm) 10 days after ureteral obstruction. The graphs summarize average interstitial cells positive for αSMA and relative means of area positive for Collagen-1 (n=6 in each group, data are presented as means±s.d., ** p*<*0.05,* **** *p*<*0.0001,* # *no significance,* values of *p* were calculated using Student's t test comparing indicated groups). Figures 10X, 10Y, 10Z and **10Z1** show data of kidney sections of transgenic EYA1A^{tg} mice challenged with UUO and treated with *in vivo* morpholinos blocking 53Bp1 translation (53Bp1-VMO), labelled for 53Bp1 (Fig. 10X), Rpal (Fig. 10Y), alpha-smooth muscle actin (αSMA, Fig. 10Z) and Collagen-1 (Fig. 10Z1) 10 days after ureteral obstruction. The figures summarize average interstitial cells of the kidney sections positive for the respective protein (n=6 in each group, data are presented as means±s.d., *** *p<0.001,* **** *p<0.0001,* values of *p* were calculated using Student's t test comparing indicated groups).

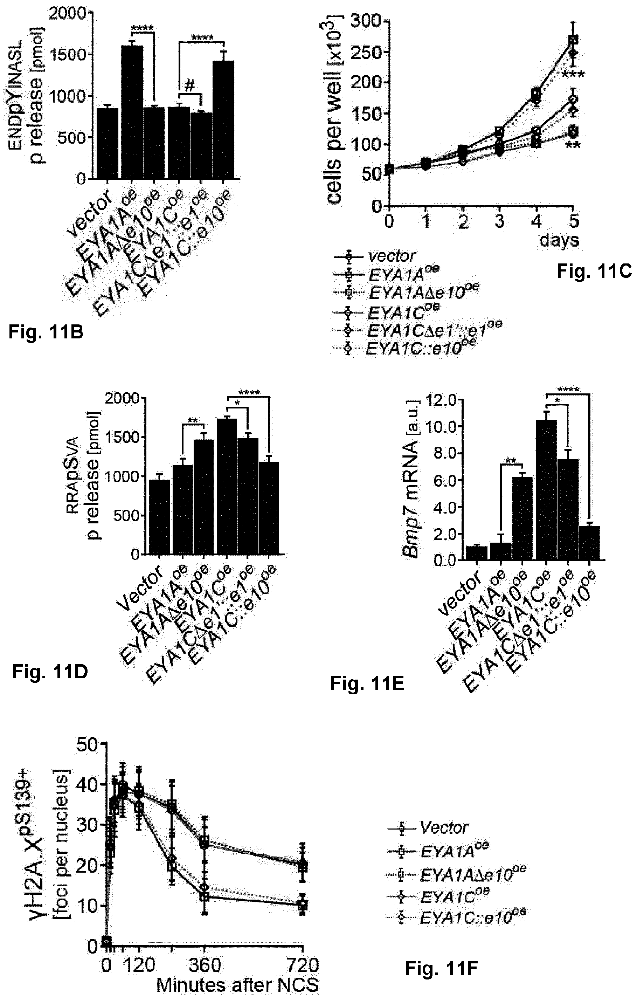

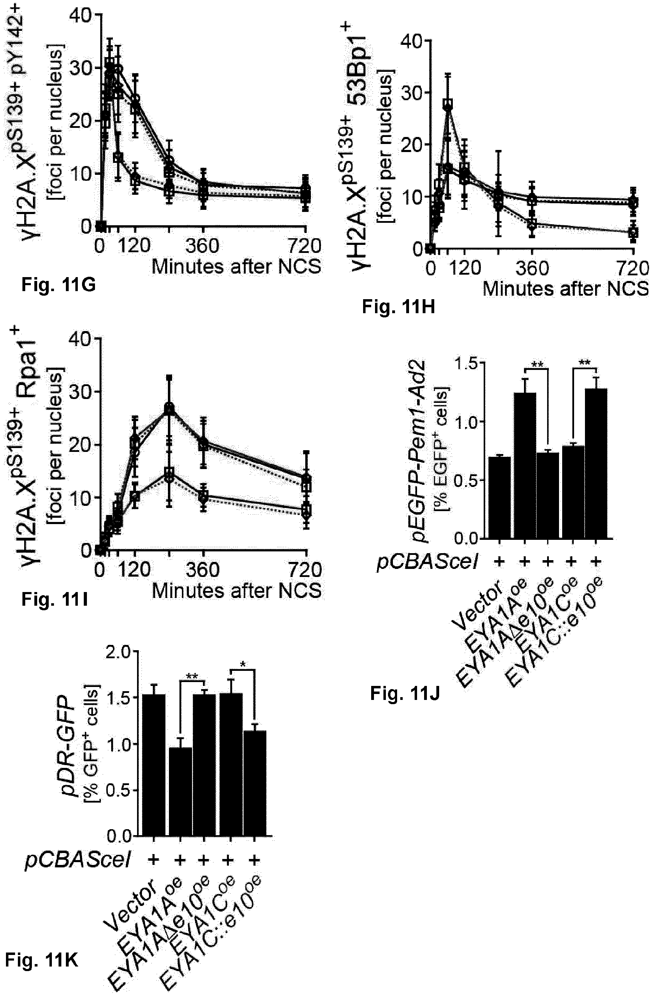

**Figure 11A** is a schematic illustration of EYA1A and EYA1C mutant constructs with EYA1A in which exon 10 has been removed (EYA1AΔe10), mutant EYA1C where exon 1' has been replaced with exon 1 (EYA1CΔ1'::e1) or EYA1C to which exon 10 has been added (EYA1C::e10). **Figure 11B** depicts data on the dephosphorylation activity using phosphotyrosine substrate ENDpYINASL (n=3 independent replicates, data are presented as means±s.d., **** p<0.0001, # no significance, values of p were calculated using one-way ANOVA with Bonferroni post-hoc analysis comparing indicated groups). **Figure 11C** depicts data on the effect on fibroblast proliferation activity. Primary murine fibroblasts transfected with indicated constructs were seeded at a density of 60,000 per well and proliferative activity was determined at indicated time points (n=3 independent replicates, data are presented as means±s.d., ** p<0.01, *** p<0.001, values of p were calculated using one-way ANOVA with Bonferroni post-hoc analysis comparing EYA1A and EYA1AΔe10 or EYA1C and EYA1C::e10). Noteworthy, data correlate with data shown in Fig. 11B. **Figure 11D** depicts data on the dephosphorylation activity using phosphoserine substrate RRApSVA (n=3 independent replicates, data are presented as means±s.d., * p<0.05, ** p<0.01, **** p<0.0001, values of p were calculated using one-way ANOVA with Bonferroni post-hoc analysis comparing indicated groups). **Figure 11E** depicts qRT-PCR for Bmp7 (n=3 independent replicates, data are presented as means±s.d., * p<0.05, ** p<0.01, **** p<0.0001, values of p were calculated using one-way ANOVA with Bonferroni post-hoc analysis comparing indicated groups). **Figures 11F to 11I** show results of an analysis of photomicrographs of primary murine fibroblast cultures transfected with control plasmids (vector) or plasmids overexpressing EYA1 mutant mice harbouring DOX-inducible transgenes. The legend depicted next to Fig. 11F applies to all figures of Figs 11F to 111. Cells were labelled for γH2A.X^{pS139} **(****Fig. 11F****),** γH2A.X^{pY142} **(****Fig. 11G****),** γH2A.X^{pS139}/53Bp1 **(****Fig. 11H****)** and γH2A.X^{pS139}/Rpa1 **(****Fig. 11I****)** at indicated time points after NCS exposure. Average foci per nucleus are shown (n=100 each, data are presented as means±s.d.). **Figures 11J and 11K** depict results of an analysis of NHEJ and HR events upon DNA damage using pEGFP-Pem1-Ad2 (NHEJ, Fig. 11J) and pDR-GFP (HR, Fig. 11K) reporter plasmids. Data are presented as means±s.d. (* p<0.05, ** p<0.01, # no significance, values of p were calculated using Student's t test comparing indicated groups).

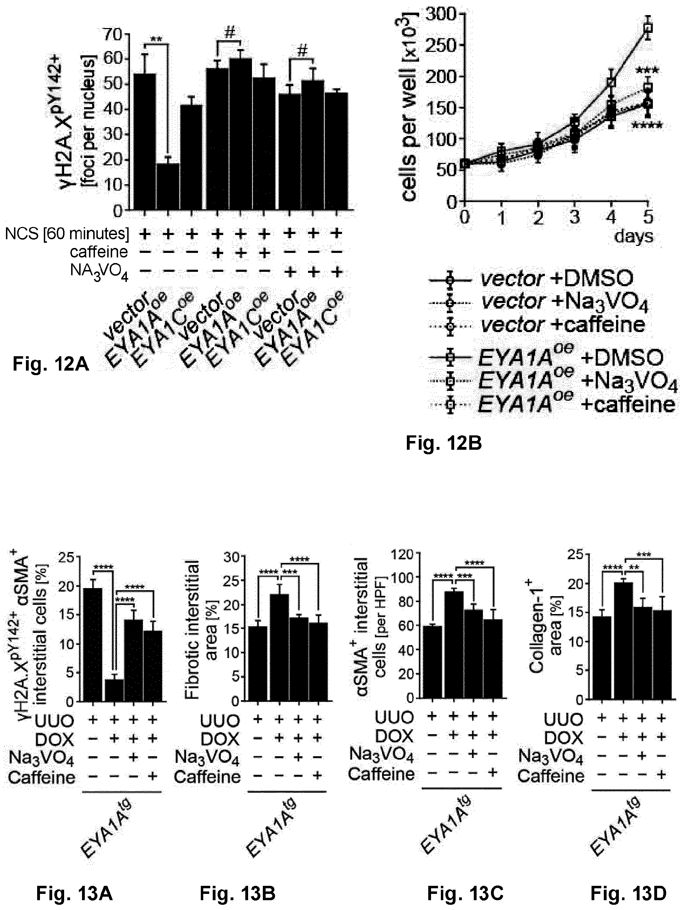

**Figure 12** shows the effect of the established EYA1 γH2A.X^{pY142} tyrosine phosphatase inhibitors Na₃VO₄ (blocking tyrosine phosphatase activity) and caffeine (blocking ATM-dependent recruitment of EYA1 towards γH2A.X) on γH2A.X^{pY142} dephosphorylation and fibroblast accumulation. **Figure 12A** depicts results of an analysis of photomicrographs of primary murine fibroblast cultures transfected with control plasmids (vector) and plasmids overexpressing EYA1A (EYA1A^{oe}) or EYA1C (EYA1C^{oe}). Cells were exposed to either Na₃VO₄ or caffeine and labelled for γH2A.X^{pY142} 60 minutes after NCS exposure (n=100 nuclei each, data are presented as means±s.d., ** p<0.01, # no significance, values of p were calculated using Student's t test comparing indicated groups). **Figure 12B** depicts results of an analysis of proliferative activity of primary murine fibroblasts transfected with indicated constructs, seeded at a density of 60,000 per well. Proliferative activity was determined at indicated time points after exposure to Na₃VO₄ or caffeine (n=3 independent replicates, data are presented as means±s.d., *** p<0.001, **** p<0.0001, values of p were calculated using one-way ANOVA with Bonferroni post-hoc analysis comparing control and Na₃VO₄⁻ or caffeine-treated cultures overexpressing EYA1A).

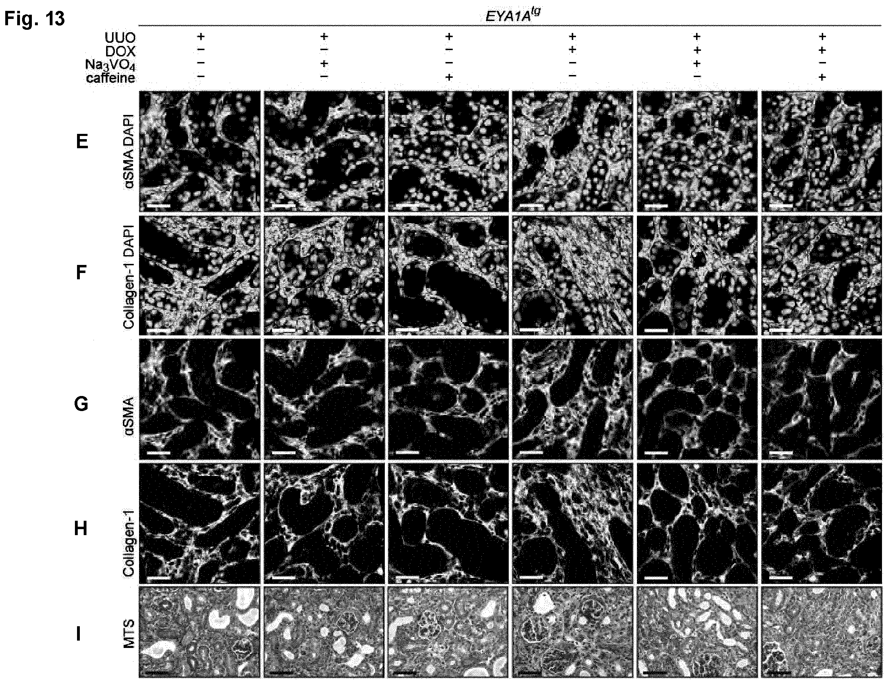

**Figure 13** illustrates a modulation of the impact of EYA1A overexpression on renal fibrogenesis using Na₃VO₄ and caffeine, which effectively blocked γH2A.X^{pY142} dephosphorylation. Transgenic EYA1A*^{tg}* mice were challenged with UUO and treated with either Na₃VO₄ (8 mg/kg i.p. once per day) or caffeine (12.5 mg/kg i.p. once per day). The graphs in **Figures 13A to 13D** summarize an analysis of photomicrographs of kidney sections labelled for γH2A.X^{pY142} and αSMA, αSMA, or collagen-1. **Fig. 13A** shows relative αSMA⁺ cells positive for γH2A.X^{pY142}, and **Fig. 13B** shows the fibrotic interstitial area 10 days after ureteral obstruction. **Fig. 13C** shows αSMA positive cells, and **Fig. 13D** collagen-1 positive cells (n=6 in each group, data are presented as means±s.d., **** p<0.0001, # no significance, values of p were calculated using one-way ANOVA with Bonferroni post-hoc analysis comparing indicated groups). **Fig. 13E** shows respective representative photomicrographs of kidney sections labelled for αSMA, as well as DAPI nucleic acid stain (scale bars: 25 µm). **Fig. 13F** shows representative photomicrographs of kidney sections labelled for collagen-1, as well as DAPI nucleic acid stain (scale bars: 25 µm). **Fig. 13G** shows the same photomicrographs as Fig. 13C with all colour signals other than red colour (αSMA) reduced to 0. **Fig. 13H** shows the same photomicrographs as Fig. 13D with all colour signals other than green colour (collagen-1) reduced to 0. **Fig. 13I** shows representative photomicrographs of kidney sections labelled for MTS (scale bars: 50 µm), also 10 days after ureteral obstruction.

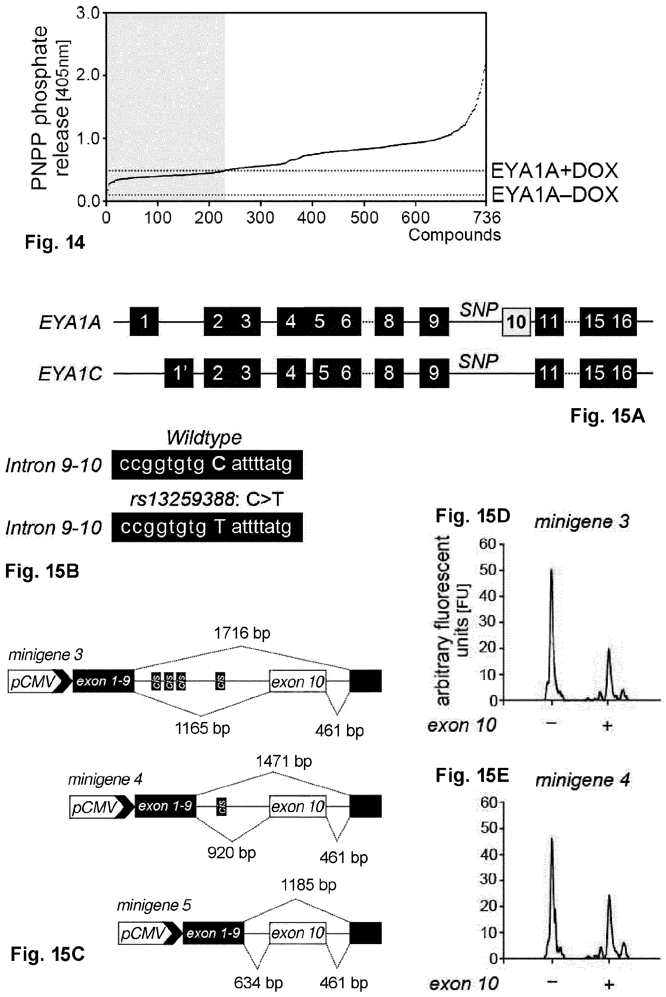

**Fig. 14** illustrates results of a screen for additional compounds for selective modulation of EYA1A phosphatase activity. The EYA1A phosphatase activity was estimated using p-nitrophenyl phosphate (pNPP) substrate. Briefly, phosphatase activity was determined in transgenic fibroblasts and pNP production was determined in comparison to control cultures. Among 736 compounds contained in the NCI mechanistic set (NCI Mechanistic Set II), 228 inhibitors of EYA1A phosphatase activity were identified.

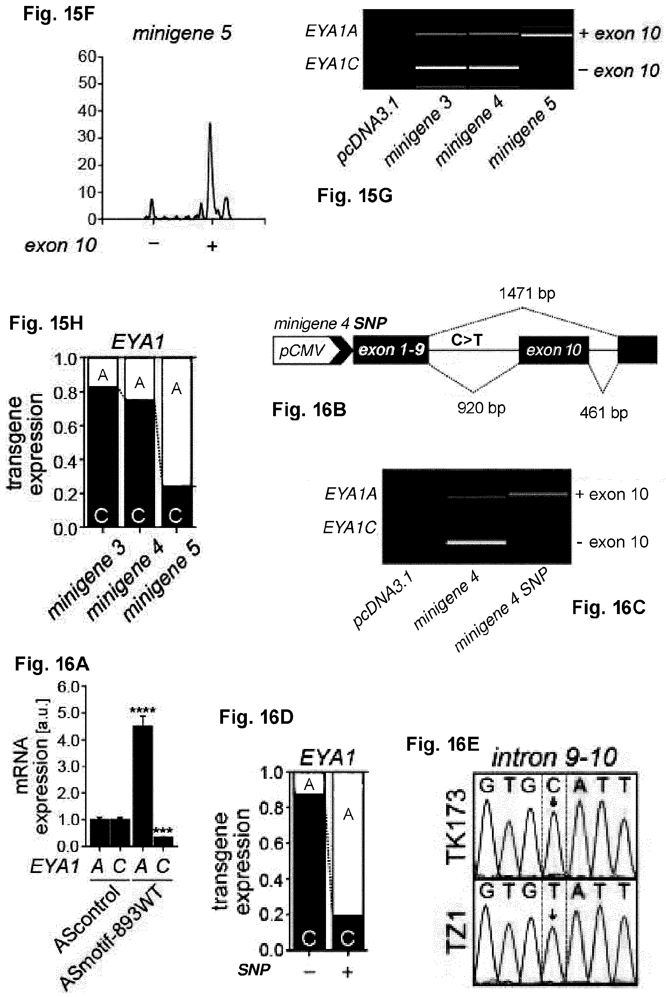

**Fig. 15A** illustrates different EYA1 splicing, differing in the presence or absence of exon 10. **Fig. 15B** illustrates that an intronic single nucleotide polymorphism (SNP rs13259388 C>T: ccggtgtgC/Tattttatg, the reverse being gtattttaT/Cgtgtggcc) is located -893 bp upstream exon 10 and disrupts an intronic splicing cis regulatory motif. The sequence of the SNP can be found at positions 307554 to 307538 of GenBank accession number NG_011735.3, version 3 as of 24 February 2018. The position of the T/C exchange can be found at position chr8:71245585 in the Ensembl database. **Fig. 15C** is a schematic illustration of minigene 3 harbouring *exons 1-11* and intronic fragments flanking *exon 10* containing cis regulatory elements (-1165 bp relative to *intron 9-10*/*exon 10 junction*), minigene 4 missing cis regulatory elements (lacking - 1165 bp to -920 bp) and minigene 5 (lacking -1165 bp to -634 bp). **Fig. 15D** depicts Bioanalyzer fluorescent units for an analysis of PCR products using minigene 3-specific oligonucleotides. **Fig. 15E** depicts Bioanalyzer fluorescent units for an analysis of PCR products using minigene 3-specific oligonucleotides. **Fig. 15F** depicts Bioanalyzer fluorescent units for an analysis of PCR products using minigene 3-specific oligonucleotides. **Fig. 15G** depicts a virtual gel image of PCR products using the minigene-specific oligonucleotides corresponding to Figs. 15D to 15F. **Fig. 15H** shows the transient expression of EYA1 isoforms using minigenes 3, 4 and 5.
**Fig. 16A** shows the analysis by qRT-PCR of the effect of administering morpholinos to block splicing cis regulatory motif (ASmotif-893WT) in TK173 fibroblast cultures. EYA1A mRNA expression was enhanced, while EYA1C mRNA expression was reduced (n=3 independent replicates, data are presented as means±s.d., *** p<0.001, **** p<0.0001, values of p were calculated using Student's t test comparing ASmotif-893WT with AScontrol cultures).

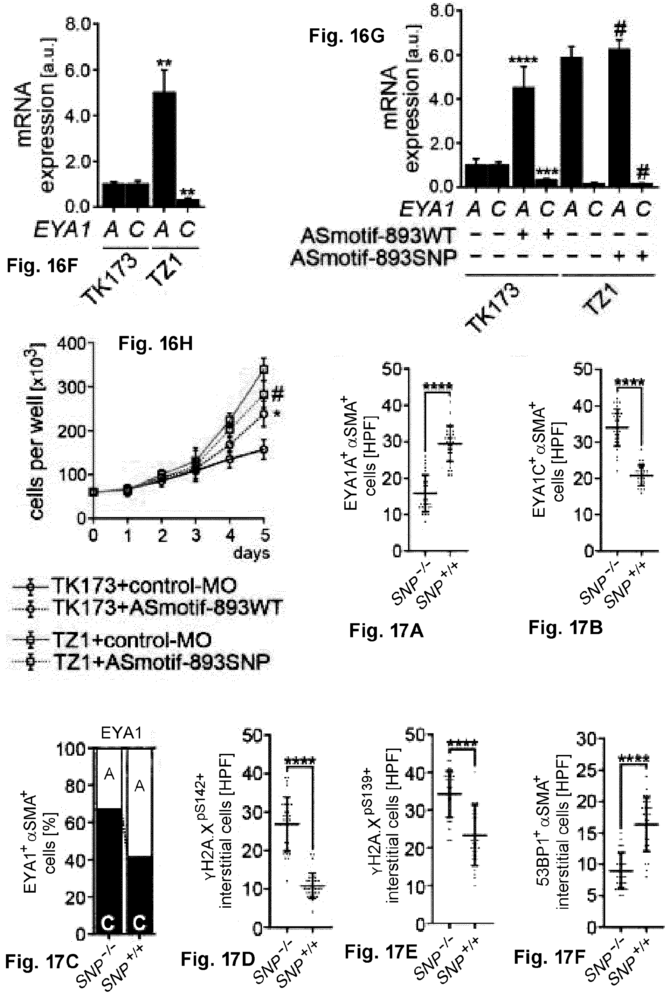

**Fig. 16B** is a schematic illustration of minigene 4 SNP, containing a C>T point mutation at -893 bp (mirroring SNP rs13259388). **Fig. 16C** depicts a virtual Bioanalyzer gel image of PCR products obtained from renal fibroblasts transfected with minigene 4 harbouring the wildtype "C" allele and minigene 4 SNP. **Fig. 16D** depicts the transient expression of EYA1 isoforms from renal fibroblasts transfected with minigene 4 and minigene 4 SNP, corresponding to Fig. 16C. Fibroblasts transfected with minigene 4 displayed exon 10 skipping, whereas exon 10 was retained when the mutated minigene 4 SNP containing the "T" allele was used. **Fig. 16E** depicts a sequencing chromatogram of fibroblast cultures established from patients homozygous for the wildtype "C" allele (TK173) or SNP rs13259388 "T" allele (TZ1). **Fig. 16F** depicts relative mRNA expression levels of isoforms EY1A and EYA1C, analyzed by qRT-PCR (n=3 independent replicates, data are presented as means±s.d., ** p<0.01, values of p were calculated using Student's t test comparing TZ1^{SNP+/+} with TK173^{SNP-/-} cultures). **Fig. 16G** shows a graph reflecting relative mRNA expression levels of isoforms EYA1A and EYA1C after administration of morpholinos to block the wildtype (ASmotif-893WT) or SNP rs13259388 (ASmotif-893SNP) motif, analyzed by qRT-PCR (n=3 independent replicates, data are presented as means±s.d., *** p<0.001, **** p<0.0001, # no significance, values of p were calculated using Student's t test comparing ASmotif-893WT/SNP with control cultures). **Fig. 16H** depicts the proliferative activity of human kidney fibroblasts seeded at a density of 60,000 per well, determined at indicated time points after ASmotif-893WT administration to TK173^{SNP-/-} and ASmotif-893SNP administration to TZ1^{SNP+/+} fibroblast cultures (n=3 independent replicates, data are presented as means±s.d., * p<0.01, # no significance, values of p were calculated using Student's t test comparing morpholino-treated TK173^{SNP-/-} or TZ1^{SNP+/+} with control cultures).

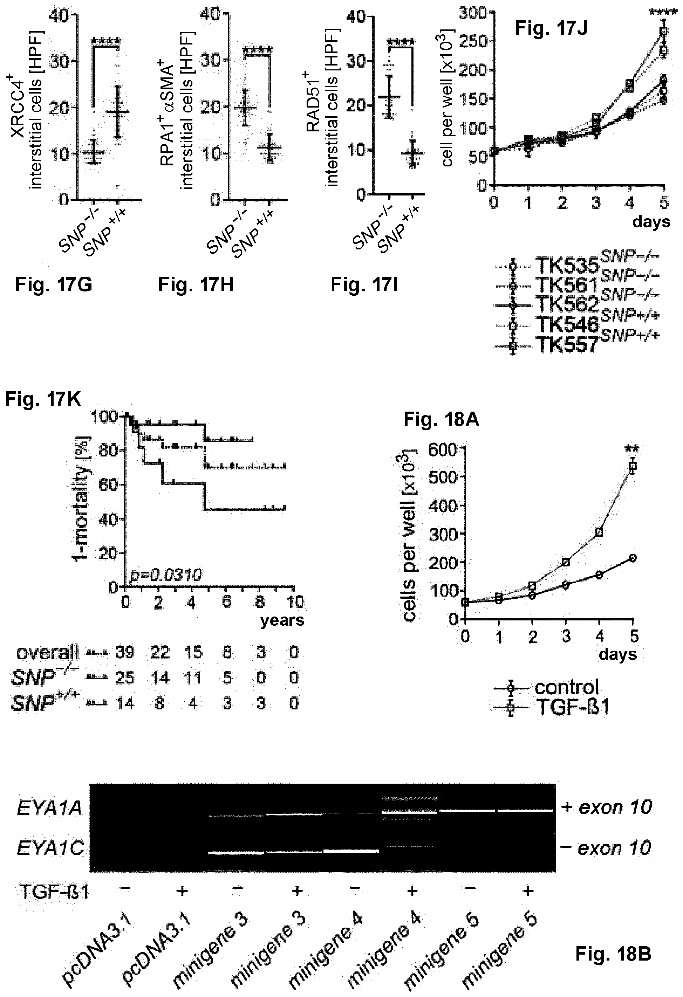

**Fig. 17** shows that intronic SNP *rs13259388* affects EYA1 *exon 10* splicing and disease progression in humans. **Figures 17A to 17I** depict the analysis of wildtype "C" allele (SNP^{-/-}) and homozygous SNP rs13259388 "T" allele (SNP^{+/+}) carriers kidney sections labelled for EYA1A/αSMA **(****Fig. 17A****),** EYA1C/αSMA **(****Fig. 17B****),** γH2A.X^{pY142} **(****Fig. 17D****),** γH2A.X^{pS139} **(****Fig. 17E****),** 53BP1/αSMA **(****Fig. 17F****),** XRCC4/αSMA **(****Fig. 17G****),** RPA1/αSMA **(****Fig. 17H****)** and RAD51 **(****Fig. 17I****)** (n=3 in each group, measurements were done in 10 visual fields, data are presented as means±s.d., **** p<0.0001, values of p were calculated using Student's t test comparing indicated groups). **Fig. 17C** graphically shows the results depicted in Figs. 17A and 17B with regard to the identified levels of EYA1 isoforms. **Fig. 17J** depicts a graph summarizing average proliferation of corresponding fibroblast cultures (n=3 independent replicates, data are presented as means±s.d., **** p<0.0001, values of p were calculated using Student's t test comparing SNP^{-/-} and SNP^{+/+} fibroblast cultures). **Fig. 17K** shows the kidney disease progression in negative patients (SNP^{-/-}) and homozygous carriers of SNP rs13259388 (SNP^{+/+}) towards ESRD (n=39, values of p were calculated using log-rank/Mantel-Cox test comparing SNP-/- and SNP+/+ carriers).

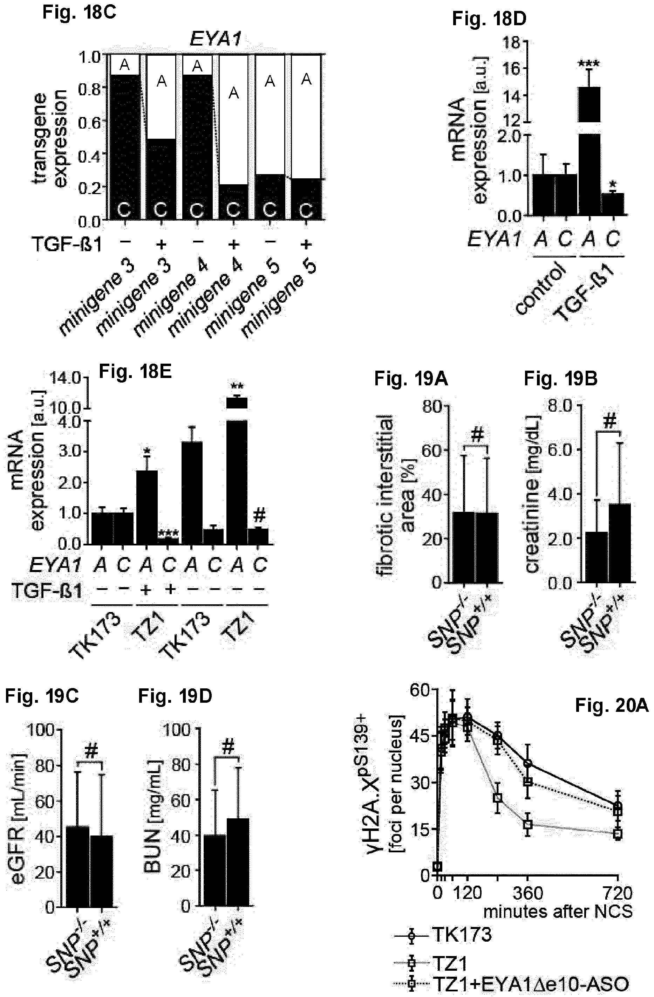

**Fig. 18A** depicts the effect of TGF-β1 exposure on the proliferative activity of human kidney fibroblasts (TK173), seeded at a density of 60,000 per well and determined at indicated time points (n=3 independent replicates, data are presented as means±s.d., ** p<0.01, values of p were calculated using Student's t test comparing control and TGF-β1-treated fibroblasts). **Figure 18B** depicts a virtual Bioanalyzer gel image of PCR products using minigene-specific oligonucleotides, and **Figure 18C** is a graphic representation, both reflecting relative mRNA levels of EYA1A and EYA1C in response to TGF-β1, analyzed by qRT-PCR (n=3 independent replicates, data are presented as means±s.d., * p<0.05, *** p<0.001, values of p were calculated using Student's t test comparing control and TGF-β1-treated fibroblasts). **Fig. 18D** depicts relative mRNA levels of EYA1A and EYA1C in response to TGF-β1, analyzed by qRT-PCR (n=3 independent replicates, data are presented as means±s.d., * p<0.05, *** p<0.001, values of p were calculated using Student's t test comparing control and TGF-β1-treated fibroblasts). **Fig. 18E** depicts relative mRNA expression levels of isoforms EY1A and EYA1C upon TGF-β1 exposure in TK173 and TZ1 fibroblast cultures, analyzed by qRT-PCR (n=3 independent replicates, data are presented as means±s.d., * p<0.05, ** p<0.01, *** p<0.001, # no significance, values of p were calculated using Student's t test comparing TZ1 with TK173 cultures).
**Figure 19** summarizes average means of fibrotic interstitial area **(****Fig. 19A****),** plasma creatinine **(****Fig. 19B****),** eGFR **(****Fig. 19C****)** and BUN **(****Fig. 19D****)** measurements comparing *wildtype* "C" allele (SNP^{-/-}) and homozygous SNP *rs13259388* "T" allele (SNP^{+/+}) carriers (n=39, data are presented as means±s.d., # no significance, values of p were calculated using Student's t test comparing indicated groups).

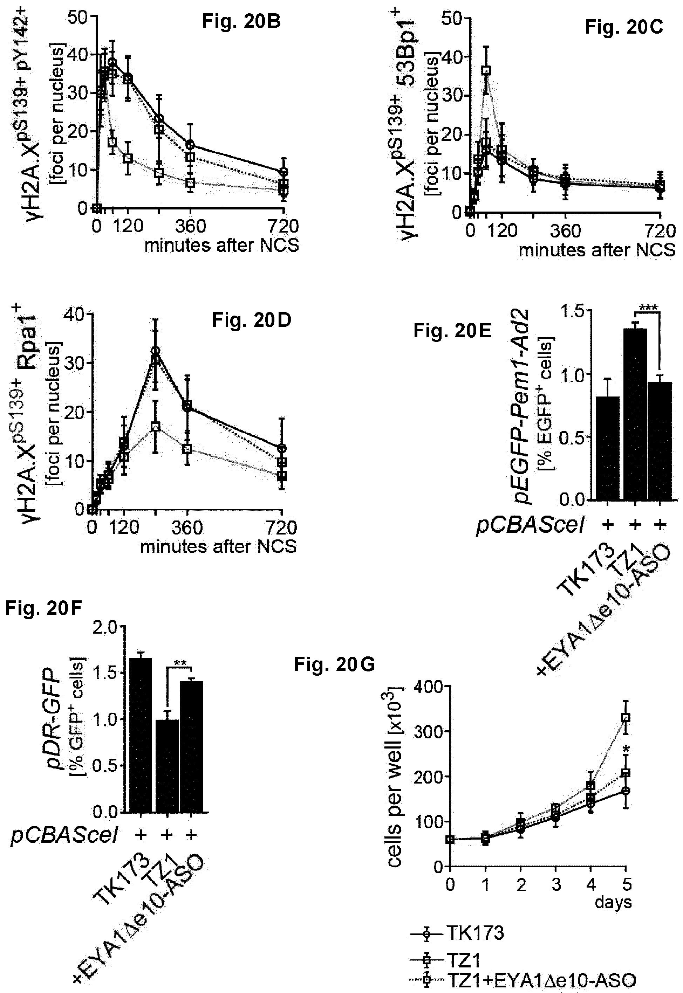

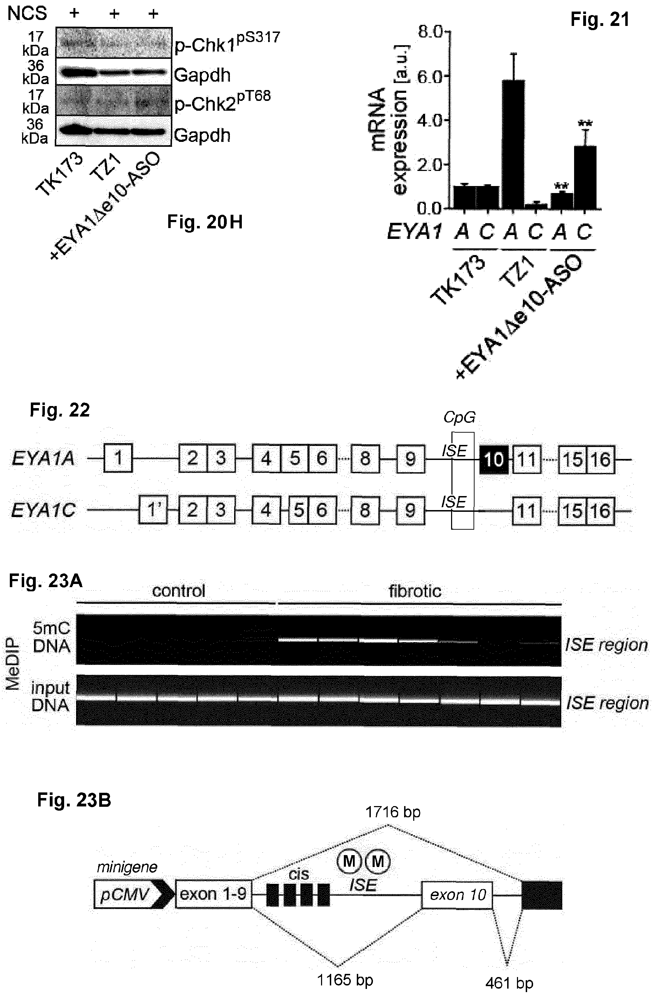

**Figures 20A to D** depict average foci per nucleus detected in human TK173 and TZ1 cultures transfected with EYA1Δe10-ASO and labelled for γH2A.X^{pS139} **(****Fig. 20A****),** γH2A.X^{pS139}/ γH2A.X^{pY142} **(****Fig. 20B****),** γH2A.X^{pS139}/53Bp1 **(****Fig. 20C****),** and γH2A.X^{pS139}/Rpa1 **(****Fig. 20D****)** at indicated time points after NCS exposure (n=100 nuclei each, data are presented as means±s.d.). **Fig. 20E** and **F** depict data on the analysis of NHEJ and HR events upon DNA damage using pEGFP-Pem1-Ad2 upon DNA damage using pEGFP-Pem1-Ad2 (NHEJ, **Fig. 20E****)** and pDR-GFP (HR, **Fig. 20F****)** reporter plasmids (n=3 independent replicates, data are presented as means±s.d., ** p<0.01, *** p<0.001, values of p were calculated using Student's t test comparing indicated groups). **Fig. 20G** depicts proliferative activity of human kidney fibroblasts at indicated time points after seeding at a density of 60,000 per well (n=3 independent replicates, data are presented as means ±s.d., and * p<0.05, values of p were calculated using Student's t test comparing EYA1Δe10-ASO-transfected TZ1 with untreated TZ1 cultures). **Fig. 20H** depicts an analysis of p-Chk1^{pS317} and p-Chk2^{pT68} by SDS-PAGE and subsequent immunoblotting 24 hours after NCS exposure.
**Fig. 21** depicts relative mRNA expression levels of isoforms *EYA1A* and *EYA1C* in human TK173, TZ1 and TZ1 after transfection of EYA1Δe10-ASO, analyzed by qRT-PCR (n=3 independent replicates, data are presented as means±s.d., ** p<0.01, values of p were calculated using Student's t test comparing EYA1Δe10-ASO-transfected TZ1 with untreated TZ1 cultures).
**Figure 22** schematically illustrates intronic EYA1 methylation upstream exon 10, where a CpG island is present, which affects EYA1 exon 10 splicing.
**Fig. 23A** depicts a virtual Bioanalyzer gel image of methylated DNA immunoprecipitation (MeDIP), showing that the CpG island is commonly methylated in fibrotic kidneys. Briefly, genomic DNA was extracted from kidney biopsies and methylated gDNA was immunoprecipitated by using 5'-methyl cytosine-specific antibodies. The PCR reaction was specific for the CpG island upstream EYA1 exon 10 and gel analysis is shown in comparison to total input DNA. **Fig. 23B** schematically illustrates the use of methylated minigene constructs to test whether methylation of the CpG island had an effect on exon 10 splicing. **Fig. 23C** depicts a virtual Bioanalyzer gel image of PCR products using minigene-specific oligonucleotides, and **Fig. 23D** is a graphic representation, both reflecting relative mRNA levels of EYA1A and EYA1C in response to TGF-β1 and in the absence and presence of CpG Methyltransferase (M. SssI). Methylation of the identified regions was associated with increased EYA1 exon 10 inclusion and generation of pro-fibrotic EYA1A.
**Figure 24** shows the sequence of EYA1 isoform A (SEQ ID NO: 1), as found in the SwissProt/Uniprot database under the accession number Q99502, version 2 of the sequence of 15 July 1998, where it has the identifier Q99502-2 (termed isoform B in SwissProt/Uniprot).
**Figure 25** shows the sequence of EYA1 isoform C, as found in the SwissProt/Uniprot database under the accession number Q99502, having the identifier Q99502-2 and termed isoform D in SwissProt/Uniprot.

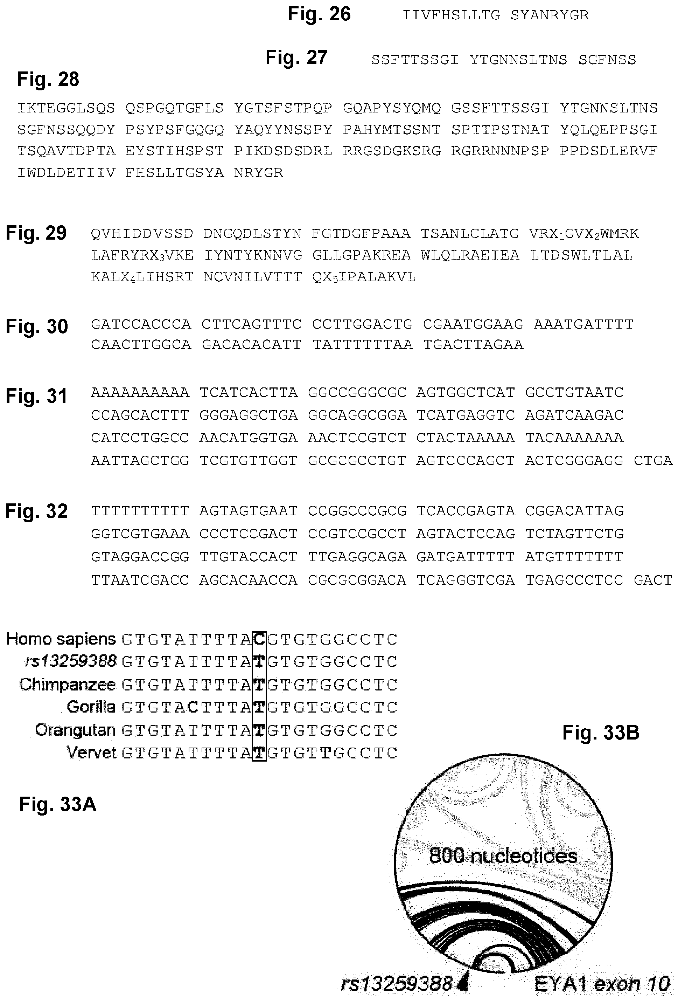

**Figure 26** depicts the sequence defined by SEQ ID NO: 2.
**Figure 27** depicts the sequence defined by SEQ ID NO: 3.
**Figure 28** depicts the sequence defined by SEQ ID NO: 11.
**Figure 29** depicts the sequence defined by SEQ ID NO: 12. In SEQ ID NO: 12, X₁ may be G or S or X₂ may be D or G, X₃ may be R or Q, X₄ may be S or P, and X₅ may be L or R.
**Figure 30** depicts the sequence defined by SEQ ID NO: 20, corresponding to exon 10.
**Figure 31** depicts the sequence defined by SEQ ID NO: 11.
**Figure 32** depicts the sequence defined by SEQ ID NO: 6.

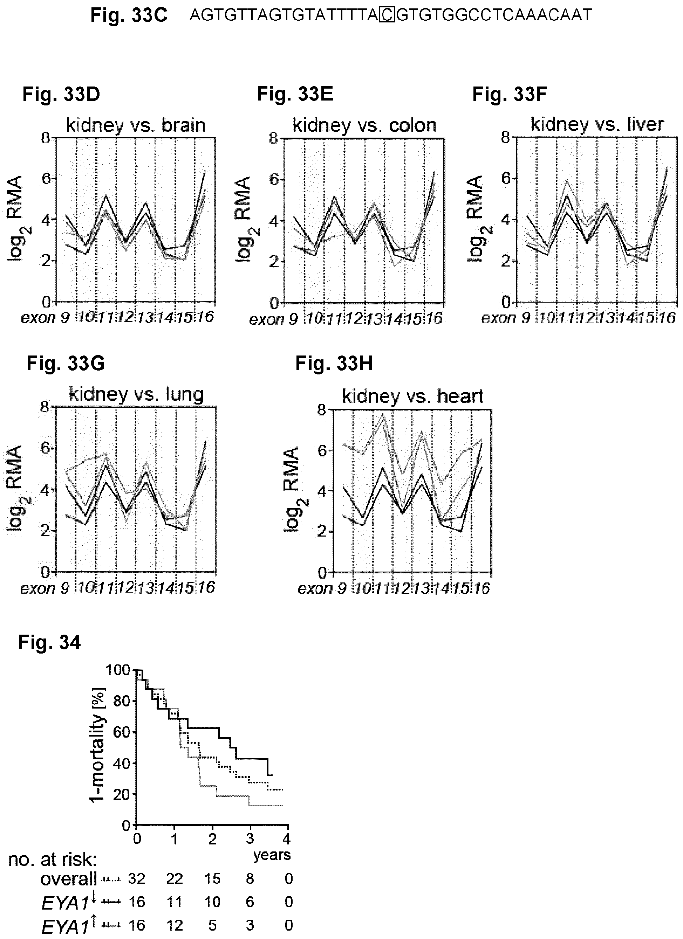

**Figure 33A** depicts results of a phylogenetic analysis. As can be taken from the figure, there is a conserved genomic region within *EYA1* corresponding to the identified intronic splicing *cis* regulatory motif among primate species including chimpanzee, gorilla, orangutan and vervet monkeys. **Figure 33B** depicts the results of an analysis of the intronic region upstream EYA1 *exon 10* with regard to predicted RNA bridge formation. **Figure 33C** illustrates the location of *rs13259388* in the surrounding human sequence. **Figures 33D to H** depict Log2 robust multi-array average (RMA) values of EYA1 exons 9-16 encoding the EYA1 domain, extracted from publicly available exon array datasets (GEO accession number GSE41409), where EYA1 exon 10 splicing is equally detectable in brain **(****Fig. 33D****),** colon **(****Fig. 33E****),** liver **(****Fig. 33F****),** lung **(****Fig. 33G****),** and heart **(****Fig. 33H****).**
**Figure 34** depicts data on the correlation between expression of EYA1 and death in patients suffering from pancreas carcinoma. The y axis depicts survivors (1-mortality). Data of 32 patients suffering from pancreas carcinoma at University hospital Goettingen were analysed. 16 patients had a low expression of EYA1 (black line), 16 patients had a high expression of EYA1 (grey line). Life expectancy compared to the entire group (dotted line) was significantly reduced in patients having a high expression of EYA1 (p=0.0033).

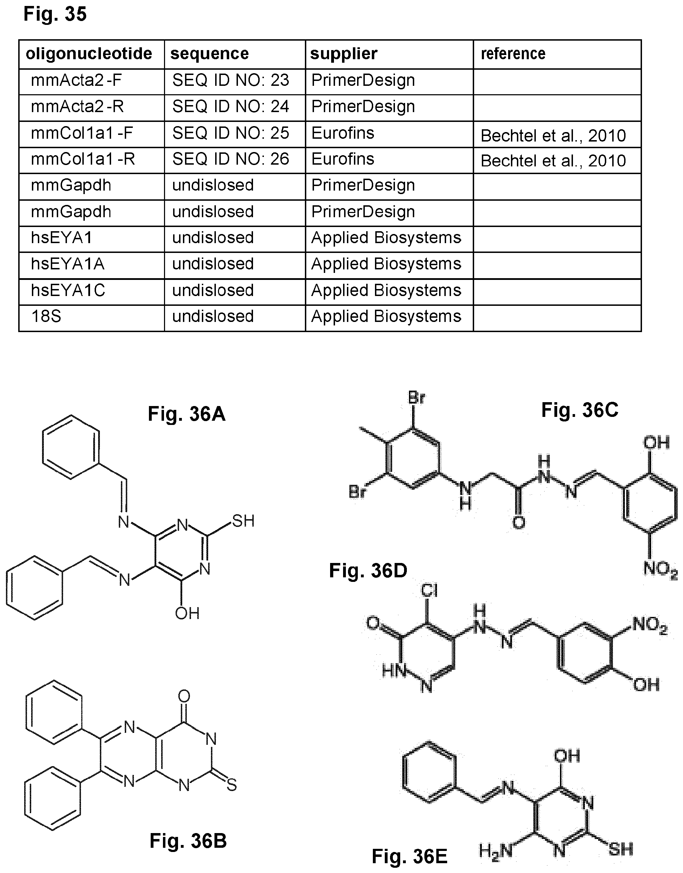

**Figure 35** depicts the source of oligonucleotides used in the Working Examples for qRT-PCR. "Undisclosed": these oligonucleotides are available from the indicated source (PrimerDesign, Chandler's Ford, UK; Applied Biosystems, part of Thermo Fisher Scientific, Dublin, Ireland), the supplier does, however, not disclose the corresponding sequences. SEQ ID NO: 23: 5'-CTCTTCCAG CCATCTTTCATTG-3'; SEQ ID NO: 24: 5'-GTTGTTAGCATAGAGATCCTTTCCT-3'; SEQ ID NO: 25: 5'-ATGGATTCCCGTTCGAGTA CG-3'; SEQ ID NO: 26: 5'-TCAGCTGGATAGCGACAT CG-3'.
**Figure 36** depicts examples of nucleic acid ligase IV inhibitors: SCR7 (Fig. 36A), SCR7 pyrazine (Fig. 36B), L67 (Fig. 36C), L82 (also called SCR5, Fig. 36D), and L189 (also called SCR6, Fig. 36C).

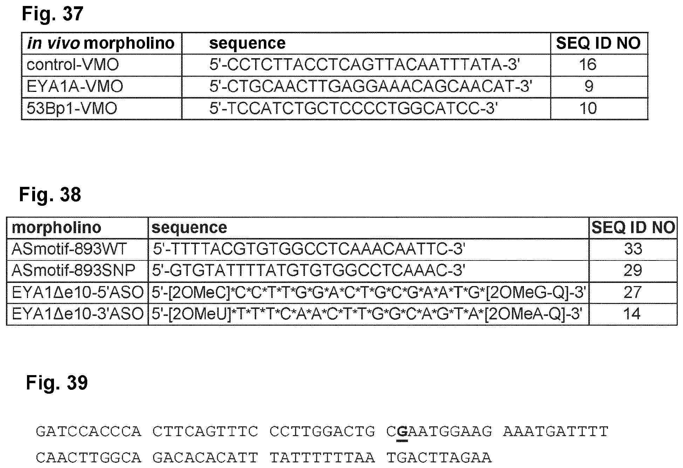

**Figure 37** depicts *in vivo* morpholino sequences used in the Working Examples. All three oligonucleotides were obtained from Gene Tools (Philomath, USA).
**Figure 38** depicts morpholino sequences used in the Working Examples. The oligonucleotides of SEQ ID NOs: 33 and 29 were obtained from Gene Tools (Philomath, USA).
**Figure 39** depicts the sequence of the exon of human Eya1 that is termed exon 10 herein. A variant exists where at the underlined position instead of G the sequence contains A. This variant falls within the term "exon 10" as used herein.

### DETAILED DESCRIPTION

The invention is defined by the appended claims.

### Definitions

Unless otherwise defined, all other scientific and technical terms used in the description, figures and claims have their ordinary meaning as commonly understood by one of ordinary skill in the art. Although similar or equivalent methods and materials to those described herein can be used in the practice or testing of the binding members, nucleic acids, vectors, host cells, compositions, methods and uses disclosed herein, suitable methods and materials are described below.

Unless otherwise stated, the following terms used in this document, including the description and claims, have the definitions given below.

The word "about" as used herein refers to a value being within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. The term "about" is also used to indicate that the amount or value in question may be the value designated or some other value that is approximately the same. The phrase is intended to convey that similar values promote equivalent results or effects as described herein. In this context "about" may refer to a range above and/or below of up to 10%. The word "about" refers in some embodiments to a range above and below a certain value that is up to 5%, such as up to up to 2%, up to 1%, or up to 0.5 % above or below that value. In one embodiment "about" refers to a range up to 0.1 % above and below a given value.

The term "administering", as used herein, refers to any mode of transferring, delivering, introducing, or transporting matter such as a compound, e.g. a pharmaceutical compound, or other agent such as an antigen, to a subject. Modes of administration include oral administration, topical contact, intravenous, intraperitoneal, intramuscular, intranasal, or subcutaneous administration (cf. below). Administration "in combination with" further matter such as one or more therapeutic agents includes simultaneous (concurrent) and consecutive administration in any order.

An "agent", as used herein, refers to any compound or combination of compounds. Typically, an agent is a single compound. A respective compound may be a low-molecular weight organic compound or a polymeric compound. An example of a polymeric compound is a protein such as EYA1 or a nucleic acid molecule such as a vector.

The term "antisense activity" means any detectable or measurable activity attributable to the hybridization of an antisense compound to a target nucleic acid molecule. In some embodiments, antisense activity is a decrease in the amount or expression of a target nucleic acid or protein encoded by such target nucleic acid.

In this regard, antisense technology is an effective means for modulating the expression of a specific gene product and is useful in a number of therapeutic, diagnostic, and research applications. The principle behind antisense technology is that an antisense compound, which hybridizes to a target nucleic acid, modulates gene expression activities such as transcription, splicing or translation through one of a number of antisense mechanisms. In one aspect, an antisense compound provided herein modulates splicing of a target gene. Such modulation includes promoting or inhibiting exon inclusion.

The terms "antisense oligonucleotide," "antisense oligomer," and "antisense compound" are used interchangeably and refer to a compound having a sequence of nucleotide bases and a subunit-to-subunit backbone that allow the antisense oligomer to hybridize to a target nucleic acid molecule through hydrogen bonding. An antisense compound is typically a single-stranded nucleic acid molecule of a length of 6 to 30, such as 15 to 30 nucleobases. An antisense compound may in some embodiments be an siRNA. In some embodiments, an antisense compound has a nucleobase sequence that, when written in the 5' to 3' direction, includes the reverse complement of the target segment of a target nucleic acid molecule to which it is targeted.

The term "detect" or "detecting", as well as the term "determine" or "determining" when used in the context of a signal such as a current, refers to any method that can be used to detect the flow of ions. Detection can be done both on a qualitative and a quantitative level. When used herein in combination with the words "level", "amount" or "value", the words "detect", "detecting", "determine" or "determining" are understood to generally refer to a quantitative rather than a qualitative level. Accordingly, a method as described herein may include a quantification of a current.

A "protein coding sequence" or a sequence that encodes a particular protein or polypeptide, is a nucleic acid sequence that gives rise to the formation of that particular protein or polypeptide. In the case of DNA it is transcribed into mRNA and is translated (in the case of mRNA) into a polypeptide *in vitro* or *in vivo* when placed under the control of a suitable regulatory sequence. The boundaries of the coding sequence are determined by a start codon at the 5' terminus (N-terminus) and a translation stop nonsense codon at the 3' terminus (C-terminus). A coding sequence may include cDNA from prokaryotic or eukaryotic mRNA, genomic DNA sequences from prokaryotic or eukaryotic DNA, or any synthetic nucleic acid. A transcription termination sequence will usually be located 3' to the coding sequence.

The term "essentially consists of" is understood to allow the presence of additional components in a sample or a composition that do not affect the properties of the sample or a composition. As an illustrative example, a pharmaceutical composition may include excipients if it essentially consists of an active ingredient.

The terms "expressing" and "expression" in reference to a polypeptide are intended to be understood in the ordinary meaning as used in the art. A polypeptide is expressed by a cell via transcription of a nucleic acid into mRNA, followed by translation into an initial polypeptide, which is folded and possibly further processed to a mature polypeptide. Hence, the statement that a cell or an organism is expressing such a polypeptide indicates that the polypeptide is found in or on the cell, and cells of the organism, respectively, and implies that the polypeptide has been synthesized by the expression machinery of the respective cell. EYA1 is usually located inside a cell, in the cytoplasm or in the nucleus.

With regard to the respective biological process itself, the terms "expression", "gene expression" or "expressing" refer to the entirety of regulatory pathways converting the information encoded in the nucleic acid sequence of a gene first into messenger RNA (mRNA) and then to a polypeptide. Accordingly, the expression of a gene includes its transcription into a primary hnRNA, the processing of this hnRNA into a mature RNA and the translation of the mRNA sequence into the corresponding amino acid sequence of the polypeptide. In this context, it is also noted that the term "gene product" refers not only to a polypeptide, including e.g. a mature polypeptide (including a splice variant thereof) encoded by that gene and a respective precursor protein where applicable, but also to the respective mRNA, which may be regarded as the "first gene product" during the course of gene expression.

By "fragment" in reference to a polypeptide such as receptor molecule is meant any amino acid sequence present in a corresponding polypeptide, as long as it is shorter than the full length sequence and as long as it is capable of performing the function of interest of the protein - in the case of EYA1 generally its tyrosinephosphate phosphatase activity, its transcriptional coactivator activity, and/or its serinephosphate and threoninephosphate phosphatase activity.

As used herein, a "heterologous" nucleic acid molecule or a "heterologous" nucleic acid sequence is a nucleic acid molecule and sequence, respectively, that does not occur naturally as part of the genome of the cell in which it is present, or a nucleic acid sequence which is found in a location or locations in the genome that differ from that in which it occurs in nature. Typically, a heterologous nucleic acid molecule and/or sequence carries or is a sequence that is not endogenous to the host cell and has been artificially introduced into the cell. The cell that expresses a heterologous nucleic acid sequence may contain DNA encoding the same or different expression products. A heterologous nucleic acid sequence need not be expressed and may be integrated into the host cell genome or maintained episomally.

As used herein, "hybridization" means the pairing of a complementary strand of an antisense compound to its target sequence. While not limited to a particular mechanism, the most common mechanism of pairing involves hydrogen bonding, which may be Watson-Crick, Hoogsteen or reversed Hoogsteen hydrogen bonding, between complementary nucleobases. For example, the natural base adenine is complementary to the natural nucleobases thymidine and uracil which pair through the formation of hydrogen bonds. The natural base guanine is complementary to the natural bases cytosine and 5-methyl cytosine. Hybridization can occur under varying circumstances.

An antisense compound as disclosed herein is specifically hybridizable to the target sequence indicated. A compound such as an antisense compound as disclosed herein is specifically hybridizable when there is a sufficient degree of complementarity to avoid non-specific binding of the antisense compound to non-target nucleic acid sequences under conditions in which specific binding is desired, i.e., under physiological conditions in the case of *in vivo* assays or therapeutic treatment, and under conditions in which assays are performed in the case of *in vitro* assays. In this regard, "stringent hybridization conditions" or "stringent conditions" refers to conditions under which an antisense compound will hybridize to its target sequence, but to a minimal number of other sequences. Stringent conditions are sequence-dependent and will be different in different circumstances, and "stringent conditions" under which antisense compounds hybridize to a target sequence are determined by the nature and composition of the antisense compounds and the assays in which they are being investigated.

"Identical" as used herein is the extent of sequence identity (homology), which may be determined using any computer program and associated parameters known in the art, such as BLAST 2.2.2 or FASTA version 3.0t78, for instance with the default parameters. Protein and/or nucleic acid sequence identities (homologies) may be evaluated using any of the variety of sequence comparison algorithms and programs known in the art. The term "essentially identical" or "substantially identical" refers to a sequence that has at least 85% sequence identity with the sequence referred to. For example, the sequence of a nucleic acid molecule is substantially identical to a given nucleic acid sequence if it has 90% or more sequence identity with the same. As a further example, the sequence of a peptide or protein is substantially identical to a given amino acid sequence if it has 90% or more sequence identity with the same. A sequence essentially identical or substantially identical with another sequence may also be a sequence that has at least 92% sequence identity or 94% sequence identity with the sequence referred to. In some embodiments a sequence essentially identical or substantially identical with another sequence is a sequence that has at least 95% sequence identity or 96% sequence identity with the sequence referred to. In some embodiments a sequence essentially identical or substantially identical with another sequence is a sequence that has 97% sequence identity or more, or 98% sequence identity or more with the sequence referred to. In some embodiments a sequence essentially identical or substantially identical with another sequence is a sequence that has 99% sequence identity or more, or 99.5% sequence identity or more with the sequence referred to.

The term "isolated" indicates that the cell or cells, or the peptide(s) or nucleic acid molecule(s) has/have been removed from its/their normal physiological environment, e.g. a natural source, or that a peptide or nucleic acid is synthesized. Use of the term "isolated" indicates that a naturally occurring sequence has been removed from its normal cellular (i.e., chromosomal) environment. Thus, the sequence may be in a cell-free solution or placed in a different cellular environment. An isolated cell or isolated cells may for instance be included in a different medium such as an aqueous solution than provided originally, or placed in a different physiological environment. Typically, isolated cells, peptides or nucleic acid molecule(s) constitute a higher fraction of the total cells, peptides or nucleic acid molecule(s) present in their environment, e.g. solution/suspension as applicable, than in the environment from which they were taken. An isolated polypeptide or nucleic acid molecule is an oligomer or a polymer of amino acids (2 or more amino acids) or nucleotides coupled to each other, including a polypeptide or nucleic acid molecule that is isolated from a natural source or that is synthesized. The term "isolated" does not imply that the sequence is the only amino acid chain or nucleotide chain present, but that it is essentially free, e.g. about 90 - 95% pure or more, of e.g. non-amino acid material and/or non-nucleic acid material, respectively, naturally associated with it.

As used herein, "modulation of splicing" refers to altering the processing of a pre-mRNA transcript such that the spliced mRNA molecule contains either a different combination of exons as a result of exon skipping or exon inclusion, a deletion in one or more exons, or additional sequence not normally found in the spliced mRNA (e.g., intron sequence). In the context of the present disclosure, modulation of splicing refers to altering splicing of EYA1 pre-mRNA to achieve exon skipping or exon inclusion. In one embodiment, exon skipping results in an EYA1 mRNA transcript lacking exon 10 and exon inclusion results in an EYA1 mRNA transcript containing exon 10. In one embodiment modulation of splicing includes promoting or effecting the removal of exon 10.

The terms "Morpholino oligomer", "Morpholino" and "phosphorodiamidate Morpholino oligomer", the latter abbreviated "PMO", are used interchangeably herein and refer to a single-stranded nucleic acid analog of 15 to 30 bases, typically about 25 bases. In a Morpholino oligomer the bases are covalently linked to a backbone of methylenemorpholine rings linked through phosphorus-containing groups, typically phosphorodiamidate groups. A monomeric unit of a Morpholino oligomer typically has the following structure:

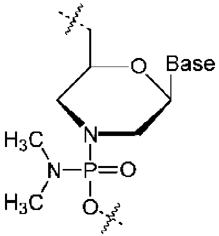

A "nucleobase" means a heterocyclic moiety capable of pairing with a base of another nucleic acid molecule. The term "contiguous nucleobases" refers to nucleobases immediately adjacent to each other.

The term "nucleic acid molecule" as used herein refers to any nucleic acid in any possible configuration, such as single stranded, double stranded or a combination thereof. Examples of nucleic acids include for instance DNA molecules, RNA molecules, analogues of the DNA or RNA generated using nucleotide analogues or using nucleic acid chemistry, locked nucleic acid molecules (LNA), protein nucleic acids molecules (PNA), alkylphosphonate and alkylphosphotriester nucleic acid molecules and tecto-RNA molecules (e.g. Liu, B., et al., J. Am. Chem. Soc. (2004) 126, 4076-4077). LNA has a modified RNA backbone with a methylene bridge between C4' and O2', providing the respective molecule with a higher duplex stability and nuclease resistance. Alkylphosphonate and alkylphosphotriester nucleic acid molecules can be viewed as a DNA or an RNA molecule, in which phosphate groups of the nucleic acid backbone are neutralized by exchanging the P-OH groups of the phosphate groups in the nucleic acid backbone to an alkyl and to an alkoxy group, respectively. DNA or RNA may be of genomic or synthetic origin and may be single or double stranded. Such nucleic acid can be e.g. mRNA, cRNA, synthetic RNA, genomic DNA, cDNA synthetic DNA, a copolymer of DNA and RNA, oligonucleotides, etc. A respective nucleic acid may furthermore contain non-natural nucleotide analogues and/or be linked to an affinity tag or a label.

Many nucleotide analogues are known and can be used for nucleic acids that are used in the methods described herein. A nucleotide analogue is a nucleotide containing a modification at for instance the base, sugar, or phosphate moieties. As an illustrative example, a substitution of 2'-OH residues of siRNA with 2'F, 2'O-Me or 2'H residues is known to improve the *in vivo* stability of the respective RNA. A variety of modifications at the sugar moiety are known in the art. The furanosyl sugar ring of a nucleoside can be modified in a number of ways including, but not limited to, addition of a substituent group, bridging of two non-geminal ring atoms to form a bicyclic nucleic acid (BNA) and substitution of an atom or group such as -S-, -N(R)- or -C(R)(R') for the ring oxygen at the 4'-position. Modified sugar moieties are well known and can be used to alter, typically increase, the affinity of a molecule such as an antisense compound for its target and/or increase nuclease resistance. Illustrative examples of modified sugars include but are not limited to a bicyclic modified sugar (BNA), including LNA and ENA (4'-(CH₂)₂-O-2' bridge); and a substituted sugar, especially a 2'-substituted sugar having a 2'-F, 2'-OCH 2 or a 2'-O(CH2)₂-OCH₃ substituent group. A sugar can also be replaced with a sugar mimetic group among others. Methods for the preparations of modified sugars are well known to those skilled in the art. Modifications at the base moiety may be a natural or a synthetic modification of A, C, G, and T/U, a different purine or pyrimidine base, such as uracil-5-yl, hypoxanthin-9-yl, and 2-aminoadenin-9-yl, as well as a non-purine or a non-pyrimidine nucleotide base. Other nucleotide analogues serve as universal bases. Examples of universal bases include 3-nitropyrrole and 5-nitroindole. Universal bases are able to form a base pair with any other base. Base modifications often can be combined with for example a sugar modification, such as for instance 2'-O-methoxyethyl, e.g. to achieve unique properties such as increased duplex stability.

As used in this document, the expression "pharmaceutically acceptable" refers to those active compounds, materials, compositions, carriers, and/or dosage forms which are, within the scope of sound medical judgment, suitable for use in contact with the tissues of human beings and animals without excessive toxicity, irritation, allergic response, or other problems or complications, commensurate with a reasonable benefit/risk ratio.

The terms "polypeptide" and "protein" are used interchangeably and refer to a polymer of amino acid residues and are not limited to a certain minimum length of the product. Where both terms are used concurrently, this twofold naming accounts for the use of both terms side by side in the art.

The term "predicting the risk" as used in the disclosure refers to assessing the probability that a subject will suffer from PAH in the future. As will be understood by those skilled in the art, such an assessment is usually not intended to be correct for 100% of the subjects to be investigated. The term, however, requires that a prediction can be made for a statistically significant portion of subjects in a proper and correct manner. Whether a portion is statistically significant can be determined by those skilled in the art using various well-known statistic evaluation tools, e.g., determination of confidence intervals, p-value determination, Student's t-test, and Mann-Whitney test. Suitable confidence intervals are generally at least 90%, at least 95%, at least 97%, at least 98%, or at least 99%. Suitable p-values are generally 0.1, 0.05, 0.01, 0.005, or 0.0001. In one embodiment of the disclosed methods, the probability envisaged by the present disclosure allows that the prediction of an increased, normal, or decreased risk will be correct for at least 60%, at least 70%, at least 80%, or at least 90% of the subjects of a given cohort or population. Predictions of risk in a disclosed method relates to predicting whether or not there is an increased risk for PAH compared to the average risk for developing PAH in a population of subjects rather than giving a precise probability for the risk.

In this regard the term "prognosis", commonplace and well-understood in medical and clinical practice, refers to a forecast, a prediction, an advance declaration, or foretelling of the probability of occurrence of a disease state or condition in a subject not (yet) having the respective disease state or condition. In the context of the present disclosure prognosis refers to the forecast or prediction of the probability as to whether a subject will or will not suffer from PAH.

The term "preventing" in the medical/physiological context, i.e. in the context of a physiological state, refers to decreasing the probability that an organism contracts or develops an abnormal condition.

The word "recombinant" is used in this document to describe a nucleic acid molecule that, by virtue of its origin, manipulation, or both is not associated with all or a portion of the nucleic acid molecule with which it is associated in nature. Generally, a recombinant nucleic acid molecule includes a sequence which does not naturally occur in the respective wildtype organism or cell. Typically a recombinant nucleic acid molecule is obtained by genetic engineering, usually constructed outside of a cell. Generally, a recombinant nucleic acid molecule is substantially identical and/or substantial complementary to at least a portion of the corresponding nucleic acid molecule occurring in nature. A recombinant nucleic acid molecule may be of any origin, such as genomic, cDNA, mammalian, bacterial, viral, semisynthetic or synthetic origin. The term "recombinant" as used with respect to a protein / polypeptide means a polypeptide produced by expression of a recombinant polynucleotide.

The term "reducing the risk", as used in this document, means to lower the likelihood or probability of a disease state or condition, e.g., fibrosis, from occurring in a subject, especially when the subject is predisposed to such or at risk of contracting a disease state or condition, e.g., fibrosis.

As used herein, a sgRNA (Single guide RNA) is a RNA molecule, typically a synthetic RNA molecule, that includes or is composed of a targeting sequence and scaffold sequence derived from bacterial crRNA and tracrRNA. An sgRNA may also include one or more aptameric accessory RNA domains. An sgRNA molecule encodes gene editing information in the form of complementary sequences. It contains one or more crRNAs and one tracrRNA. The sgRNA, once formed, is used to target an endonuclease 9, once formed, to a specific genomic locus in genome editing.

The term "subject" as used herein, also addressed as an individual, refers to an animal, generally a mammal, that expresses the protein EYA1. A subject may be a rodent or a livestock animal. A subject may also be a companion animal or a zoological animal. In some embodiments the subject may be a mammalian species such as a cattle or a goat. The subject may also be a sheep. In some embodiments the subject is a horse. The subject may also be a dog or a cat. In some embodiments the subject is a ferret or a chinchilla. In some embodiments the subject is a pig. The subject may also be a monkey or an ape. In some embodiments the subject may be a rabbit or a mouse. The subject may also be a rat or a Guinea pig. The subject may also be a hamster. In one embodiment the subject is a human.

The terms "treatment" and "treating" as used herein, refer to a prophylactic or preventative measure having a therapeutic effect and preventing, slowing down (lessen), or at least partially alleviating or abrogating an abnormal, including pathologic, condition in the organism of a subject. Those in need of treatment include those already with the disorder as well as those prone to having the disorder or those in whom the disorder is to be prevented (prophylaxis). Generally, a treatment reduces, stabilizes, or inhibits progression of a symptom that is associated with the presence and/or progression of a disease or pathological condition. The term "therapeutic effect" refers to the inhibition or activation of factors causing or contributing to the abnormal condition. A therapeutic effect relieves to some extent one or more of the symptoms of an abnormal condition or disease. The term "abnormal condition" refers to a function in the cells or tissues of an organism that deviates from their normal functions in that organism. An abnormal condition can inter alia relate to cell proliferation, cell differentiation, or cell survival.

The term "variant" as used herein can refer to a nucleotide sequence in which the sequence differs from the sequence most prevalent in a population, for example by one nucleotide, in the case of the point mutations described herein. For example, some variations or substitutions in the nucleotide sequence encoding a EYA1 protein can alter a codon so that a different amino acid is encoded resulting in a variant polypeptide. The term "variant" can also refer to a polypeptide in which the sequence differs from a given sequence as explained further below. A variant may for example be a polypeptide in which the sequence differs from the sequence most prevalent in a population. A polypeptide sequence can for instance differ at a position that does not change the amino acid sequence of the encoded polypeptide, i.e. a conserved change. Variant polypeptides can be encoded by a mutated EYA1 sequence.

The terms "comprising", "including," containing", "having" etc. shall be read expansively or open-ended and without limitation. Singular forms such as "a", "an" or "the" include plural references unless the context clearly indicates otherwise. Thus, for example, reference to a "vector" includes a single vector as well as a plurality of vectors, either the same - e.g. the same operon - or different. Likewise reference to "cell" includes a single cell as well as a plurality of cells. Unless otherwise indicated, the term "at least" preceding a series of elements is to be understood to refer to every element in the series. The terms "at least one" and "at least one of" include for example, one, two, three, four, or five or more elements. It is furthermore understood that slight variations above and below a stated range can be used to achieve substantially the same results as a value within the range. Also, unless indicated otherwise, the disclosure of ranges is intended as a continuous range including every value between the minimum and maximum values.

The scope and meaning of any use of a term will be apparent from the specific context in which the term is used. Certain further definitions for selected terms used throughout this document are given in the appropriate context of the detailed description, as applicable. Unless otherwise defined, all other scientific and technical terms used in the description, figures and claims have their ordinary meaning as commonly understood by one of ordinary skill in the art.

### Tissues concerned

Aberrant fibroblast proliferation can occur in many tissues. Progression of many cancer types has been found to involve fibroblast proliferation, and the presence of excessive fibroblasts has been suggested as a marker in diagnosis and therapy of premalignant and malignant tumours. Studies on targeting so called "carcinoma-associated fibroblasts" or "cancer-associated fibroblasts", a heterogeneous population of cells defining a major component of tumour stroma, releasing inter alia TGFβ, are ongoing. Collagen, synthesized by fibroblasts (supra), has been found to be involved in inter alia cancer cell behaviour as well as cancer initiation and progression via interactions with other extracellular matrix proteins (Xu et al., J. Transl. Med. (2019) 17, 309, doi: 10.1186/s12967-019-2058-1). An example of a disease associated with aberrant fibroblast proliferation is a melanoma. Two further examples of a disease associated with aberrant fibroblast proliferation are breast cancer and gastric cancer. Pancreatic adenocarcinoma, in particular pancreatic ductal adenocarcinoma, and ovarian cancer are two further examples of a disease associated with aberrant fibroblast proliferation. Aberrant fibroblast proliferation has also been found to occur in liver cirrhosis and hepatocellular carcinoma. Dermatofibroma, such as atrophic dermatofibroma, as well as a plexiform fibrohistiocytic tumour are further examples of a disease associated with aberrant fibroblast proliferation. As yet a further example, the activation and proliferation of fibroblasts that are resistant to apoptosis has been identified to be involved in remodelling of the myocardium that can progress to heart failure with preserved ejection fraction (Oatmen, KE, Nat Rev Cardiol. (2019), doi: 10.1038/s41569-019-0286-y).

Desmoplasia, a term typically only used to define desmoplastic small round cell tumours, is on a general basis the growth of fibrous and/or connective tissue or stroma. Fibroblasts are the major contributor to desmoplasia. Typically, the stromal reaction in a cancerous tumour is similar to the stromal reaction induced by injury or wound repair, namely increased ECM and growth factor production and secretion, which consequently cause growth of the tissue. In some embodiments, a disease associated with aberrant fibroblast proliferation can therefore be defined as involving desmoplasia.

EYA1 has been found to be overexpressed in various types of cancer, such as Wilms' tumours, breast cancer, gastric carcinoma, hepatocellular carcinoma and a subset of leukemia patients. The phosphatase activity of EYA1 has been found to enhance breast cancer cellular proliferation (Wu, K, et al., Cancer Res. (2013) 73(14), 4488-4499). In stages 1, 2, 3 and 4S of neuroblastoma, overexpression of EYA1 has been observed, and EYA1 has been suggested as a prognostic marker in neuroblastoma (Hansen, J.N., et al., J Cancer Res Ther (Manch) (2016) 4(2), 11-18.).

EYA1 has furthermore been found to contribute to the Epithelial-Mesenchymal Transition (EMT)-like phenotype change occurring in hepatocellular carcinoma (Kong, D., et al., Am. J. Transl. Res. (2019) 11(4), 2328-2338). The authors suggest EYA1 as a prognostic factor for hepatocellular carcinoma patients. Against this background, the methods developed by the present inventors are a promising approach in preventing, treating or delaying progression of a cancerous tumour.

As explained above, aberrant fibroblast proliferation furthermore occurs in fibrosis. Fibrosis can also occur in many tissues within the human or animal body, typically as a result of inflammation or damage. Two illustrative examples are the lungs and the liver. In the lungs, pulmonary fibrosis and radiation-induced lung injury following treatment for cancer can occur. Pulmonary fibrosis may be in the form of cystic fibrosis or idiopathic pulmonary fibrosis. In the liver cirrhosis can occur. Two further illustrative examples are the kidney and the heart. In the kidney fibrosis is a characteristic feature of all forms of chronic renal disease. In chronic kidney disease progressive decline of renal function and continuous accumulation of extracellular matrix occurs, which leads to a diffuse fibrosis in the form of non reversible scarring. The common process is interstitial fibrosis, the deposition of pathological fibrillar matrix in the potential space between tubules and peritubular capillaries. It is generally accepted that the underlying process is a wound-healing response to tissue injury that does not resolve. In the heart, in typical cases an excess deposition of extracellular matrix occurs in the cardiac muscle, a process called cardiac fibrosis. Cardiac fibroblasts are an essential cell type in the heart, responsible for the homeostasis of the extracellular matrix. Upon chronic insult, these cells transform to a myofibroblast phenotype and contribute to cardiac fibrosis. In some cases fibrosis in the heart occurs in the form of an abnormal thickening of the heart valves due to inappropriate proliferation of cardiac fibroblasts.

In embodiments where the disease is renal fibrosis, it is a non reversible connective tissue development leading inevitably to chronic progressive renal disease, the common final pathway in the majority of primary renal disease. Chronic kidney disease (CKD) may have various different initial causes such as diabetes mellitus, hypertension/vascular disease or direct injury, with a subsequent fibrotic response. CKD involves the retention and subsequent accumulation of solutes such as indoxyl sulfate and p-cresyl sulfate that would be cleared by a healthy kidney. Chronic kidney disease generally leads to a progressive decline in kidney function. The effect of the destruction of nephrons is compensated by hypertrophy and a supranormal glomerular filtration rate of remaining nephrons, while the scarring process already progresses. In addition, compensatory hyperfiltraion causes overwork injury in the remaining nephrons, causing further fibrosis. Accordingly, renal fibrosis not only occurs in the end stage of CKD with reduced glomerular filtration rate, but already at early stages with a glomerular filtration rate of 90 mL/min/1.73 m² or higher.

### EYA1

EYA1 (synonym EYA transcriptional coactivator and phosphatase 1) encodes for a member of the evolutionary conserved eyes absent family. The parent eyes absent gene was originally identified in eyeless drosophila mutants.

In humans, unlike in any other species, alternative splicing generates EYA1 transcript variants, raising the possibility that humans gained alternative splicing as a means to differentially regulate EYA1 substrate specificities, cf. also below. Alternative splicing of EYA1 has previously been associated with mutations leading to skipping of exon 8, involved in Branchio-Oto-Renal Syndrome, an inherited disease characterized by branchial closure defects leading to cysts or periauricular pits/tags, hearing loss, and renal abnormalities.

The human protein Eyes absent homolog 1, encoded by the gene EYA1, can be found in the SwissProt/Uniprot database under the entry Q99502, version 164 of 12 September 2018, version 2 of the sequence of 15 July 1998. The gene sequence, including the sequence encoded, can be found in the GenBank database under the accession number Y10260.1, version 1 of the sequence as of 8 February 1997, the version of the database entry is of 2 February 2011. The gene sequence on human on chromosome 8 encoding EYA1 that has been curated by NCBI as a reference standard, has GenBank accession number NG_011735.3, version 3 of the sequence dated 24 February 2018, database entry as of 3 June 2019. It is a linear DNA sequence of 357,698 bp, which includes a total 18 exons, which are consecutively numbered in the GenBank entry.

Both the SwissProt/Uniprot database database entry and the GenBank database list three isoforms formed by alternative splicing. The GenBank database names these proteins encoded by the transcript variants isoforms A, B or C, and D. The SwissProt/Uniprot database names these isoforms according to the transcripts isoform EYA1A (SwissProt/Uniprot identifier: Q99502-1), isoform EYA1B (SwissProt/Uniprot identifier: Q99502-2), and isoform EYA1D (SwissProt/Uniprot identifier: Q99502-3). The sequence of GenBank accession number Y10260.1 is termed the EYA1A gene, encoding a protein of 559 amino acids. This protein is termed isoform EYA1B in the SwissProt/Uniprot database.

A further sequence found in the GenBank database under the accession number AJ000097.1 is termed the mRNA for the EYA1B gene. The database entry is the second version (termed "version 1", following a first "sequence 0" of 5 December 1997) of the sequence as of 26 November 1998, the version of the database entry is of 1 February 2011. It encodes a protein of 592 amino acids and differs from variant A in that the first eight amino acids are replaced by a stretch of 41 amino acids. This protein is termed isoform EYA1A in the SwissProt/Uniprot database (SwissProt/Uniprot identifier: Q99502-1), and it has been chosen as the canonical sequence. A GenBank database entry having accession number NM_172058.4, version 4 of the sequence as of 7 May 2019, database entry of 8 August 2019, is termed transcript variant EYA1B. It differs from the sequence of GenBank accession number AJ000097.1 in the exchange of the first base for a sequence of 73 bases as well as a few base exchanges and an additional TTT triplet following base 2845 of the sequence of GenBank accession number AJ000097.1. A similar sequence can be found in the GenBank database as isoform C with accession number NM_000503.6. The database entry is the sixth version of the sequence as of 23 November 2018, the version of the database entry is of 8 August 2019.

The last isoform is found in the GenBank database as transcript D with accession number AF467247.1. The database entry is the first version of the sequence as of 2 February 2002, the version of the database entry is of 11 March 2010. This protein is also termed transcript EYA1D in the SwissProt/Uniprot database (SwissProt/Uniprot identifier: Q99502-3). Isoform EYA1D differs from isoform EYA1C - according to GenBank numbering - in that amino acids 140 to 144 and 351 to 380 of isoform EYA1C are missing. The isoform of transcript EYA1D has a length of 557 amino acids. The GenBank/GenPept accession number of the protein sequence, called EYA1D, is AAL73437, version 1 of 2 February 2002, database entry of 11 March 2010.

In the following the numbering of proteins according to GenBank is essentially followed. Accordingly, the isoform of GenBank accession number Y10260.1, which has SwissProt/Uniprot identifier Q99502-1 as EYA1A according to SwissProt/Uniprot numbering, is called EYA1A. The isoform of GenBank accession number AF467247.1, termed transcript D in GenBank, and having SwissProt/Uniprot identifier Q99502-3 as transcript EYA1D according to SwissProt/Uniprot numbering, is called protein EYA1C.

Eya family members are unique in possessing both phosphoserine/phosphothreonine and phosphotyrosine phophatase activities. Eya family members are primarily known as transcriptional activators through their serine phosphatase activity (Li, X, et al., Nature (2003) 426, 247-254). Three phosphotyrosine phosphatase substrates of EYA1 have been described, namely the atypical protein kinase C zeta, the estrogen receptor beta and the histone H2A variant H2A.X. However, only histone H2A variant H2A.X could be confirmed as a substrate of EYA1. H2A.X is involved in the repair versus apoptosis response to DNA damage, see e.g. Rebay, I, Mol. Cell Biol (2016) 36, 5, 668-677. The protein EYA1 localizes at sites of DNA damage at double-strand breaks. Studies in drosophila and mice failed to establish EYA1 as dynamic dual-function phosphatase though (Li et al., 2003, supra; Millhouse, S, Manley, JL, Mol Cell Biol (2005) 25, 533-544).

### EYA1 and DNA Damage Repair

Since a single unrepaired DNA double-strand break can lead to genome rearrangements and potentially to cell death, cells have evolved three repair mechanisms to face this threat and maintain genomic integrity: non-homologous end joining (NHEJ), microhomology-mediated end joining (MMEJ), and homologous recombination (HR).

Homologous recombination can be carried out where an identical or essentially identical sequence is present that can be used as a template. The enzymatic machinery involved is nearly identical to the machinery responsible for chromosomal crossover during meiosis. This pathway allows a damaged chromosome to be repaired using a sister chromatid or a homologous chromosome as a template. In NHEJ a particular DNA ligase directly joins the two ends. To guide accurate repair, NHEJ relies on short homologous sequences called microhomologies present on the DNA ends to be joined. If the respective single-stranded overhangs are compatible, repair is usually accurate. However, NHEJ can also introduce mutations and loss of damaged nucleotides at the break site can lead to deletions. MMEJ starts with short-range end resection by a nuclease on either side of a double-strand break to reveal microhomology regions. MMEJ is always accompanied by a deletion, so that MMEJ is a mutagenic pathway for DNA repair.

The global DNA damage response (DDR) system begins with activation of a protein kinase, the protein kinase ATM, which detects the damaged DNA and leads to chromatin relaxation at sites of double-strand breaks. Protein kinase ATM furthermore phosphorylates the chromatin-bound histone variant H2A.X at its serine 139. The phosphorylated H2A.X is γ-H2A.X4, which is required for subsequent interaction with downstream DNA repair proteins as well as chromatin modifying proteins. At the basal level, i.e. without DNA damage induction, H2A.X is furthermore phosphorylated at tyrosine 142 by a transcription factor (Xiao, A, et al., Nature (2009) 457, 57-62). When protein kinase ATM phosphorylates Ser139 of H2A.X upon DNA damage, the phosphotyrosine phosphatase activity of EYA1 removes this phosphate group at tyrosine 142. As a result, H2A.X interacts with the mediator protein MDC1, which promotes signal amplification. MDC1 interacts with γ-H2A.X via the phosphorylated S139 site and facilitates further recruitment of EYA to the DNA lesion.

The present inventors have found that the exclusion/inclusion of EYA1 exon 10 is a critical factor. When exon 10 is excised, then referred to as EYA1C, EYA1 acts predominantly as a serine phosphatase. This is functionally relevant, because EYA1 is shuttled to target genes by co-transcription factors of the Six/Dachs family, where it enhances transcription by dephosphorylation of the RNA polymerase IIb. Targeted genes include BMP7, and ultimately, EYA1C enhances kidney regeneration and is renoprotective.

In contrast, when exon 10 is included, then referred to as EYA1A, the protein acts predominantly as a tyrosine phosphatase on γ-H2A.X, removing tyrosine residue 142. The inventors furthermore found that, as consequence of EYA1A upregulation, accelerated dephosphorylation of tyrosine residue 142 of γ-H2A.X changes the response of fibroblasts to DNA damage from utilizining predominantly the homologous recombination (HR) rather than NHEJ. The consequence of this HR-to-NHEJ switch is that now fibroblasts can continue to proliferate under genotoxic stress, which is highly abundant within injured kidneys, ultimately contributing to progression of chronic kidney disease (cf. Figure 1).

The present inventors have identified ways of targeting the new pathway, as indicated in Fig. 1. They found that removal of the critical exon 10 can be therapeutically induced by administration of antisense compounds and also through demethylation of the site at intron 9-10. They identified SNP rs1325938 as having high predictive value as a biomarker for the progression of chronic kidney disease. They furthermore found that inhibition of the disadvantageous tyrosine phosphatase activity of EYA1A, for example through orthovanadate, attenuates fibrosis. They also found that inhibition of NHEJ, which results from EYA1A abundance, through the ligase IV inhibitor SCR7 or administration of *in vivo* morpholinos targeting 53Bp1, attenuates kidney fibrosis. Their data suggests that these findings are not limited to the kidney, but also apply to fibrosis in other organs and cancer.

An antisense compound according to the present disclosure may be directed against the generation of transcript variant EYA1A by splicing. More precisely, the antisense compound may be directed against one or both of the splice junctions flanking *exon 10,* thereby modulating exon 10 splicing.

An antisense compound according to the present disclosure may also be directed against the splice product termed EYA1A herein. More specifically, an antisense compound may serve in inhibiting translation of EYA1A. Thereby formation of EYA1A is reduced or prevented.

An antisense compound directed against the *intron 9-10*/*exon 10* and/or *exon 10*/*intron 10-11* splice junctions, is able to prevent the inclusion of exon 10 of the sequence that encodes the protein EYA1 into this protein, thereby preventing the formation of splice variant EYA1A. An antisense oligonucleotide directed against the *intron 9-10*/*exon 10* and *exon 10*/*intron 10-11* splice junctions is also abbreviated EYA1Δe10-ASO herein. An antisense oligonucleotide directed against the *intron 9-10*/*exon 10* splice junction is also abbreviated EYA1Δe10-5'ASO herein. An antisense oligonucleotide directed against the *exon 10*/*intron 10-11* splice junction is also abbreviated EYA1Δe10-5'ASO herein.

The exons of the human EYA1 encoding gene can be identified in the Ensembl entry (supra) of human EYA1. Exon 9 has been assigned exon ID ENSE00003547586. It is found at positions 322203 to 322286 of the sequence of EYA1 at positions 71,197,433-71,592,025 on human chromosome 8 identified in Ensembl by accession number ENSG00000104313. Exon 9 can furthermore be found at positions 283308 to 283391of the NCBI reference sequence NG_011735.3 (supra, version 3 as of 24 February 2018). Exon 10 has been assigned Ensembl exon ID ENSE00003642688, and is found at positions 347334 to 347423 of the sequence of EYA1 at positions 71,197,433-71,592,025 on human chromosome 8 identified in Ensembl by accession number ENSG00000104313 (Ensembl release 98 of September 2019. Exon 11 has been assigned Ensembl exon ID ENSE00003481860, and is found at positions 375003 to 375061 of the sequence of EYA1 identified in Ensembl by accession number ENSG00000104313. Exon 11 can furthermore be found at positions 336,108 to 336,166 of the NCBI reference sequence NG_011735.3.

An antisense compound directed against the *intron 9-10*/*exon 10* splice junction includes a sequence that is at least 90 % identical to the sequence of SEQ ID NO: 5, or a complement thereof. In some embodiments the antisense compound directed against the *intron 9-10*/*exon 10* splice junction includes a sequence that is at least 94 % identical to the sequence of SEQ ID NO: 5, or a complement thereof. In some embodiments the antisense compound directed against the *intron 9-10*/*exon 10* splice junction includes a sequence that is at least 96 % identical to the sequence of SEQ ID NO: 5, or a complement thereof. In some embodiments the antisense compound directed against the *intron 9-10*/*exon 10* splice junction includes a sequence that is at least 98 % identical to the sequence of SEQ ID NO: 5, or a complement thereof. In some embodiments the antisense compound directed against the *intron 9-10*/*exon 10* splice junction includes a sequence that is at least 99 % identical, or at least 99.5 % identical to the sequence of SEQ ID NO: 5, or a complement thereof. In some embodiments the antisense compound directed against the *intron 9-10*/*exon 10* splice junction includes a sequence that is identical to the sequence of SEQ ID NO: 5, or a complement thereof. In some embodiments the antisense compound directed against the *intron 9-10*/*exon 10* splice is the sequence of SEQ ID NO: 5, or a complement thereof.

In some embodiments the antisense compound directed against the *exon 10*/*intron 10-11* splice junction includes a sequence that is at least 90 % identical to the sequence of SEQ ID NO: 4, or a complement thereof. In some embodiments the antisense compound directed against the *exon 10*/*intron 10-11* splice junction includes a sequence that is at least 94 % identical to the sequence of SEQ ID NO: 4, or a complement thereof. In some embodiments the antisense compound directed against the *exon 10*/*intron 10-11* splice junction includes a sequence that is at least 96 % identical to the sequence of SEQ ID NO: 4, or a complement thereof. In some embodiments the antisense compound directed against the *exon 10*/*intron 10-11* splice junction includes a sequence that is at least 98 % identical to the sequence of SEQ ID NO: 4, or a complement thereof. In some embodiments the antisense compound directed against the *exon 10*/*intron 10-11* splice junction includes a sequence that is at least 99 % identical, or at least 99.5 % identical to the sequence of SEQ ID NO: 4, or a complement thereof. In some embodiments the antisense compound directed against the *exon 10*/*intron 10-11* splice junction includes a sequence that is identical to the sequence of SEQ ID NO: 4, or a complement thereof. In some embodiments the antisense compound directed against the *exon 10*/*intron 10-11* splice is the sequence of SEQ ID NO: 4, or a complement thereof.

As already noted above, an antisense compound as disclosed herein may contain one or more nucleosides having modified sugar moieties.

An antisense compound according to the present disclosure can be conveniently and routinely made through the well-known technique of solid phase synthesis. Equipment for such synthesis is commercially available through various sources. Any other means for such synthesis known in the art may additionally or alternatively be employed. It is well known to use similar techniques to prepare oligonucleotides such as a phosphorothioate and an alkylated derivative.

The protein EYA1 is in some embodiments a variant of a naturally occurring EYA1 protein. For example, it may be negligible to exchange certain amino acid residues that are nor critical for allowing the binding to other factors, the metal binding and/or the protein tyrosine phosphatase activity or the protein serine and protein threonine phosphatase activity. It may also be desired modify a EYA1 protein at one or more positions for detection purposes. A respective variant is a protein that contains an amino acid sequence that has at least about 98% sequence identity to a naturally existing EYA1 protein. In some embodiments a respective variant contains an amino acid sequence that has at least about 99% sequence identity to a naturally existing EYA1 protein. A variant of the EYA1 protein is typically functional in that it allows the above described catalytic activity, and/or RNA binding and/or physiological activity.

Typically, the difference from a naturally occurring EYA1 protein is a substitution. In some embodiments the difference from a naturally occurring EYA1 protein is a deletion. A variant of a naturally occurring EYA1 protein may be a gene obtained from the expression of a gene sequence altered by site specific mutagenesis.

Variants of a naturally occurring EYA1 protein may be prepared by protein and/or chemical engineering, introducing appropriate modifications into the nucleic acid sequence encoding the polypeptide, or by protein/peptide synthesis. A variant may be obtained by any combination(s) of one or more deletions, substitutions, additions and insertions to nucleic acid and/or the amino acid sequence, provided that the obtained polypeptide defines a functional EYA1 protein. In some embodiments a variant of a polypeptide provided herein differs from a particular sequence of a polypeptide provided herein by up to five substitutions. A substitution in an amino acid sequence of a polypeptide provided herein may be a conservative substitution. Examples of conservative substitutions include:
1. Substituting alanine (A) by valine (V);
2. Substituting arginine (R) by lysine (K);
3. Substituting asparagine (N) by glutamine (Q);
4. Substituting aspartic acid (D) by glutamic acid (E);
5. Substituting cysteine (C) by serine (S);
6. Substituting glutamic acid (E) by aspartic acid (D);
7. Substituting glycine (G) by alanine (A);
8. Substituting histidine (H) by arginine (R) or lysine (K);
9. Substituting isoleucine (I) by leucine (L);
10. Substituting methionine (M) by leucine (L);
11. Substituting phenylalanine (F) by tyrosine (Y);
12. Substituting proline (P) by alanine (A);
13. Substituting serine (S) by threonine (T);
14. Substituting tryptophan (W) by tyrosine (Y);
15. Substituting phenylalanine (F) by tryptophan (W);
   and/or
16. Substituting valine (V) by leucine (L)
   and *vice versa.*

A variant of a naturally occurring EYA1 protein may include one or more, such as two or three of such conservative substitutions. In some embodiments a polypeptide according to this disclosure includes a sequence that has four or more conservative substitutions in comparison to a naturally occurring EYA1 protein. In some embodiments a variant includes a sequence that has five or more, such as six or more conservative substitutions.

Non-conservative substitutions may lead to more substantial changes, e.g., with respect to the charge, dipole moment, size, hydrophilicity, hydrophobicity or conformation of the polypeptide. In some embodiments the polypeptide includes one or more, such as two non-conservative substitutions. In some embodiments a variant of a naturally occurring EYA1 protein includes three or four non-conservative substitutions. The variant may also include five or more, e.g. six, or seven or more of such non-conservative substitutions.

The invention relates to a compound for use in preventing, treating and/or delaying progression of aberrant fibroblast proliferation by administering to a subject an antisense oligonucleotide against a sequence included in the splice junction of intron 9-10/exon 10, and/or an antisense oligonucleotide against a sequence included in the splice junction of intron 9-10/exon 10 exon 10/intron 10-11 of the sequence encoding the protein EYA1. The sequence included in the splice junction of intron 9-10/exon 10 may include the sequence of SEQ ID NO: 5 or of SEQ ID NO: 27 or consist of the sequence of SEQ ID NO: 5 or of SEQ ID NO: 27. The sequence included in the splice junction of intron 9-10/exon 10 may also include a portion of the sequence of SEQ ID NO: 5or of SEQ ID NO: 27 or be a portion of the sequence of SEQ ID NO: 5 or of SEQ ID NO: 27. The sequence included in the splice junction of exon 10/intron 10-11 of the sequence encoding the protein EYA1 may include the sequence of SEQ ID NO: 4 or of SEQ ID NO: 14 or consist of the sequence of SEQ ID NO: 4 or of SEQ ID NO: 14. The sequence included in the splice junction of intron 9-10/exon 10 may also include a portion of the sequence of SEQ ID NO: 4 or of SEQ ID NO: 14 or be a portion of the sequence of SEQ ID NO: 4 or of SEQ ID NO: 14.

This method can thus be understood as antisense therapy. Without being bound by theory, the antisense oligonucleotide is thought to bind to the pre-mRNA transcript produced by the EYA1 gene at the respective splice junction. Thereby the particular splice junction is blocked and in effect hidden. As a result, the inclusion of exon 10 into the mature mRNA is prevented. Exon skipping is being effected or promoted. Thereby the formation of isoform EYA1C can be thought to in effect be enhanced, and the amount of isoform EYA1A can be thought to be reduced. Put differently, the ratio between isoform EYA1C and EYA1A can be thought to be shifted toward isoform EYA1C.

### Therapeutic applications

A compound for preventing, treating or delaying progression of a disease that involves aberrant fibroblast proliferation, e.g. fibrosis or cancer, in a subject as disclosed herein may be provided in a composition which further includes a suitable carrier, excipient or diluent. In typical embodiments a respective composition includes a compound described herein. Such a composition may, e.g., be a diagnostic, a cosmetic or a pharmaceutical composition. For therapeutic or cosmetic purposes, the composition is a pharmaceutical composition including a pharmaceutical carrier, excipient or diluent, i.e. not being toxic at the dosages and a concentration employed.

A compound for preventing, treating or delaying progression of a disease that involves aberrant fibroblast proliferation as disclosed herein is useful as a medicament. Typically, such a medicament includes a therapeutically effective amount of a molecule as disclosed above. Accordingly, a respective molecule can be used for the production of a medicament useful in the treatment of fibrosis.

In one aspect, a use in treating fibrosis is provided. The use includes the step of administration of a pharmaceutically effective amount of a molecule as described herein, such as a low molecular weight compound. Administration is generally to a subject in need thereof. In one embodiment, the pharmaceutical composition described above, which includes such pharmaceutically effective amount of the compound is administered to the subject. The medicament referred to above may be administered to a subject.

The subject in need of a treatment can be a human or a non-human animal. Typically, the subject is a mammal, e.g., a mouse, a rat, rabbit, a hamster, a dog, a cat, a monkey, an ape, a goat, a sheep, a horse, a chicken, a guinea pig or a pig. In typical embodiments, the subject is diagnosed with a fibrosis or may acquire such a disorder.

The compound for treating fibrosis as disclosed herein may be included in a pharmaceutical composition as indicated above. The pharmaceutical composition may be applied by one or more of various suitable routes of administration. Administration can for instance be conducted parenterally. In some embodiments administration is carried out intramuscularly. In some embodiments administration is carried out intravenously as a bolus or by continuous infusion. Administration is in some embodiments conducted intraarticularly. In some embodiments administration is done intrasynovially. Administration may in some embodiments be subcutaneously. In some embodiments administration is carried out topically, e.g., to the skin or the eye. Administration is in some embodiments carried out rectally. In some embodiments administration is done dermally such as intradermally, subcutaneously or transdermally. Administration can in some embodiments be performed locally. Further suitable modes of administration include, but are not limited to intracerebrally, intracerebrospinally, intrathecally, epidurally, or intraperitoneally; orally; urogenitally; intravitreally; systemically; intravenously; intraocularly; oticly; intranasally; by inhalation; sublingually; buccally, for example. Typical routes of administration are the topical, rectal, local, intranasal, intravenous and/or intradermal routes of administration.

The listing or discussion of a previously published document in this specification should not necessarily be taken as an acknowledgement that the document is part of the state of the art or is common general knowledge.

The following are examples, illustrating the methods, uses and agents disclosed herein. It is understood that various other embodiments may be practiced, given the general description provided above.

### EXAMPLES

The examples illustrate techniques that can be used in a method and a use disclosed herein. The examples are presented in the form of an overview, with details of the methods and reagents following thereafter.

### Example 1 - Fibrotic fibroblasts resist genotoxic stress by accelerated recruitment of high-fidelity NHEJ DDR

The extent of DNA damage is commonly assessed by immunolabeling with antibodies directed at histone γH2A.X. Histone H2A.X contains a conserved S¹³⁹QEY¹⁴²-COOH motif which is subject to post-translational modifications upon DNA damage involving serine 139 (S139) and tyrosine 142 (Y142) residues: While H2A.X S139 is phosphorylated upon double strand breaks (γH2A.X^{pS139}, then commonly referred to as yH2A.X), the phorphorylation status of the γH2A.X Y142 residue (γH2A.X^{pY142}) is considered to serve as a modifier for ensuing DDR. γH2A.X immunolabeling and G2/M DNA damage checkpoint activation are highly abundant in fibrotic fibroblasts of chronically injured parenchymal organs including kidney, heart, lung and liver (Fig. 2A-K, Fig. 3A-E). Based on the observation of H2A.X enrichment within renal fibroblasts (Fig. 4A-G, 5A-H) and a previous established hereditary link of impaired DDR and fibrosis in nephropathies (Zhou, W., et al., Nat Genet (2012) 44, 910-915), the inventors decided to focus on the kidney. Human fibroblasts isolated from fibrotic kidney biopsies (TK567, TK568, TK573) accumulated more rapidly than primary fibroblasts from non-fibrotic kidneys in culture (TK544, TK555, TK560, Fig. 2L) as had been reported in numerous previous studies proliferation (Tampe & Zeisberg, 2014, supra; Bechtel et al., 2010, supra; LeBleu et al., 2013, supra; Rodemann & Muller, 1990, supra), seemingly contradicting observed DNA damage checkpoint activation.

To gain insights into distinct DDR pathways, double strand brake repair and γH2A.X dephosphorylation dynamics were compared in early passage fibroblasts under neocarcinostatin (NCS)-induced genotoxic stress (Krishnan, N, et al., J Biol Chem (2009) 284, 16066-16070). In fibroblasts isolated from fibrotic kidneys, accelerated repair of double strand brakes and γH2A.X clearance correlated with premature γH2A.X^{p142} dephosphorylation (Fig. 2M-R), indicating utilization of modified DDR mechanisms. The two major repair mechanisms of double strand brakes in actively dividing cells are non-homologous end joining (NHEJ) and homologous recombination (HR). NHEJ repairs DSBs by direct ligation of broken ends without requiring prolonged cell cycle arrest, and NHEJ occurs during all phases of actively cycling cells. In contrast, checkpoint activation and G2/M cell cycle arrest are prequisites for HR to occur, enabling alignment of sister chromatids and strand invasion. Hence, high-fidelity NHEJ is prolific by enabling rapid repair of double strand brakes and continued cell cycle progression (Mao, Z, et al., DNA Repair (Amst) (2008) 7, 1765-1771). Fibrotic fibroblasts displayed accelerated formation of 53Bp1/γH2A.X foci (NHEJ) instead of Rpa1/γH2A.X foci (HR) (Fig. 2O, P, S, T), confirmed by substantially increased NHEJ pEGFP-Pem1-Ad2 and reduced HR pDR-GFP reporter activities (Fig. 2 U, V, and **Fig. 6A****)** (Seluanov, A, et al., Proc Natl Acad Sci U.S.A. (2004) 101, 7624-7629; Pierce, AJ, et al., Genes Dev (1999) 13, 2633-2638). Based on these observations, the inventors hypothesized that accelerated recruitment of NHEJ core proteins is a feature of rapidly dividing fibroblasts as a prerequisite of fibrotic fibroblast accumulation to occur.

### Example 2 - EYA1 isoforms determine DDR cell fate decisions and CKD progression

Previous studies identified γH2A.X^{pY142} as substrate of members of the evolutionary conserved family of eyes absent proteins. Because EYA1 was linked to renal disease progression and fibrosis in patients in two independent genome-wide screening studies (Bechtel, W. et al., Nat Med (2010) 16, 544-550; Kottgen, A. et al., BMC Med Genet (2008) 9, 49, doi:10.1186/1471-2350-9-49), the inventors explored a possible role of EYA1 in observed modified DDR mechanisms. While total EYA1 mRNA expression levels did not differ significantly between fibrotic and non-fibrotic kidneys (Fig. 7A), it was observed that fibrosis was associated with significantly increased expression of transcript variant EYA1A, whereas EYA1C levels were substantially decreased within fibrotic fibroblasts (Figs. 7B to D, Figs. 6B to Fl). Ratio of EYA1A over EYA1C abundance within renal biopsies was associated with accelerated decline of renal function towards dialysis-dependent end-stage renal disease (ESRD, Fig. 7E), implicating that EYA1 impacts fibroblast accumulation and renal disease progression.

The inventors next aimed to elucidate whether observed EYA1 isoform switching was causally relevant for fibroblast accumulation and progression of kidney fibrosis. Because distinct EYA1 transcript variants are not present in mice (Abdelhak, S. et al., Nat Genet (1997) 15, 157-164), doxycycline (DOX)-inducible transgenic mice were generated, overexpressing human splice variants EYA1A (*rtTACMV*;*hEYA1A-pTreTight,* referred to as *EYA1A^{tg}*) or EYA1C (*rtTACMV*;*hEYA1C-pTireTight,* referred to as *EYA1C^{tg}*) allowing assimilable overexpression of distinct EYA1 isoforms in experimental models of kidney fibrosis not affected by spontaneous phenotypes upon transgene induction (Fig. 7F).

Transcriptional profiling and gene set enrichment revealed that enhanced fibrosis in EYA1A overexpressing mice was mostly related to pathways involved in DNA damage response and cell cycle checkpoint regulation (GSE92934), correlated with enhanced γH2A.X^{pY142} clearance and accumulation of NHEJ constituent 53Bp1 as compared to either uninduced transgenic mice or DOX-treated wildtype littermates (data not shown). Accelerated γH2A.X^{pY142} clearance was associated with increased fibroblast proliferation and fibrosis in kidneys of EYA1A overexpressing mice upon challenge with unilateral ureteral obstruction (UUO, Figs. 7F to H) or 5/6 nephrectomy (5/6Nx). Increased EYA1A levels and enhanced γH2A.X^{pY142} clearance as a prerequisite of fibrotic fibroblast accumulation that we had observed within fibroblasts of human renal biopsies was associated with accelerated decline of renal excretory function (Fig. 8). Dephosphorylation assays using phosphorylated peptides for the EYA1 phosphotyrosine (ENDpYINASL) and phosphoserine phosphatase activity (RRApSVA) confirmed increased tyrosine phosphatase activity of EYA1A, correlating with enhanced interaction with γH2A.X^{pY142} (data not shown).

In control studies, administration of *in vivo* morpholinos to specifically inhibit transgenic EYA1A translation (EYA1A-VMO) reversed pro-fibrotic effects specifically in EYA1A overexpressing mice (Figs. 9A to D). Transcriptional profiling and gene set enrichment revealed that enhanced fibrosis in EYA1A overexpressing mice was mostly related to pathways involved in DNA damage response and cell cycle checkpoint regulation (GSE92934, data not shown), correlated with enhanced γH2A.X^{pY142} clearance and attenuation of DNA damage checkpoint activation within fibrotic fibroblasts of chronically injured kidneys (data not shown).

Reduced checkpoint activation within renal fibroblasts correlated with accumulation of 53Bp1 and Xrcc4 (NHEJ constituents) instead of Rpal and Rad51 recombinase (HR constituents), phenocopying the association of increased EYA1A levels, enhanced γH2A.X^{pY142} clearance, attenuated DNA damage checkpoint activation and predominant recruitment of NHEJ core proteins that the inventors had observed within fibroblasts of human renal biopsies. In synchronized renal transgenic fibroblasts, EYA1A overexpression induced premature γH2A.X^{pY142} dephosphorylation and clearance of DSBs (Figs. 10A and B) and increased re-entry into cell cycle (as indicated by attenuated γH3^{pS10}, Fig. 10C). In line with the inventors' previous findings, premature γH2A.X^{pY142} dephosphorylation was found to be associated with accelerated recruitment of high-fidelity NHEJ core proteins and NHEJ *pEGFP-Pem1-Ad2* reporter activity (Figs. 10D and E). These observations were confirmed in kidneys of EYA1A overexpressing mice harbouring NHEJ reporter transgenes (*EYA1A^{tg}*;*R26NHEJ,* Figs. 10 Gto I) (Vaidya A., et al., PLoS Genet (2014) 10, e1004511), providing further evidence that high-fidelity NHEJ becomes the predominant repair pathway when EYA1A was increased.

To elucidate causality between the observed EYA1A-induced shift towards NHEJ, enhanced fibroblast accumulation and fibrosis, the inventors next administered the small molecule NHEJ inhibitor SCR7 to EYA1A^{tg} mice challenged with UUO (Srivastava M., et al., Cell (2012) 151, 1474-1487). SCR7 (20 mg/kg i.p. every alternate day) significantly reduced fibrosis and fibroblast accumulation in EYA1A overexpressing mice (Figs. 10 J to O, T, V and W), further supporting that high-fidelity NHEJ becomes the predominant repair pathway specifically in EYA1A overexpressing mice.

Because the inventors had observed robust 53Bp1/γH2A.X foci formation and 53Bp1 accumulation upon EYA1A overexpression in fibroblast cultures and chronically injured kidneys, we next administered *in vivo* morpholinos blocking 53Bp1 translation (53Bp1-VMO) to EYA1Atg mice challenged with UUO. Rationale for this approach was that previous studies had established 53Bp1 as direct suppressor of HR, that depletion of 53Bp1 is capable to shift the cell fate decision from NHEJ to HR (Zimmermann M., et al., Science (2013) 339, 700-704; Bunting S.F., et al., Cell (2010) 141, 243-254). Depletion of 53Bp1 within chronically injured kidneys upon 53Bp1-VMO administration correlated with increased presence of Rpal in EYA1A-overexpressing mice (indicative of increased HR, Figs. 10 R, S, X and Y). Reversal of EYA1A-induced NHEJ cell fate decision attenuated fibroblast accumulation and renal fibrosis (Figs. 10 P to S, Z, and Z1), reversing the effect of EYA1A overexpression and suggesting that NHEJ causally contributes to fibrosis and fibroblast accumulation.

In contrast to established mechanistic link between EYA1A and high-fidelity NHEJ, overexpression of EYA1C in EYA1C^{tg} mice attenuated fibrosis in both models and reduced fibroblast accumulation independent of DNA damage checkpoint activation or DDR cell fate decisions (Figs. 7F and G, 8A and B, 10A and B), suggesting that EYA1A and EYA1C possess distinct biological activities and prompting us to further explore underlying mechanisms.

### Example 3 - Inclusion of critical exon 10 determines EYA1 tyrosine phosphatase activity

Based on their findings, the inventors hypothesized that EYA1 splicing in humans could serve as means to realize distinct phosphatase substrate specificities. Because the two major differences between EYA1 isoforms EYA1A and EYA1C are the use of alternate *exons 1*/*1'* and distinct inclusion/skipping of *exon 10* (Fig. 11A) (Abdelhak et al. 1997, supra), they asked if alternative splicing of either of the two regions could alter EYA1 substrate specificities. To explore the causal contribution of alternate *exon 1*/*1'* and *exon 10* for the observed distinct serine and tyrosine phosphatase activities, they overexpressed mutant EYA1A in which *exon 10* had been removed (*EYA1AΔe10*)*,* mutant EYA1C where *exon 1'* was replaced with *exon 1* (*EYA1CΔ1'::e1*) or EYA1C to which *exon 10* had been added (*EYA1C::e10*) (Figs. 11A).

When *exon 10* was absent (*EYA1C* and *EYA1AΔe10*)*,* activity to dephosphorylate peptides encoding for the EYA1 phosphatase histone substrate motif ENDpYINASL were reduced (Fig. 11B), correlating with failure to accelerate fibroblast proliferation (Fig. 11C). Instead, dephosphorylation assays using the synthetic serine phosphatase substrate RRApSVA demonstrated superior serine phosphatase activity (Fig. 11D). In contrast, overexpression of *EYA1C::e10* phenocopied dephosphorylating efficacy and fibroblast accumulation of EYA1A (Fig. 11B,C), suggesting that EYA1 tyrosine phosphatase activity depends on the absence/presence of alternative spliced *exon 10.* EYA1C containing *exon 10* (*EYA1C::e10*) phenocopied EYA1A with regard to premature γH2A.X^{pY142} dephosphorylation, γH2A.X clearance and accelerated recruitment of NHEJ (Figs. 11F to 11I). In contrast, deletion of EYA1A critical *exon 10* (*EYA1AΔe10*) normalized premature γH2A.X^{pY142} dephosphorylation and γH2A.X clearance, associated with accelerated HR (Figs. 11F to 11I). Inclusion of *exon 10* to EYA1C enhanced NHEJ, whereas deletion of *exon 10* from EYA1A increased HR responses in previous established reporters (Figs. 11J and K) (Seluanov A., et al., Proc Natl Acad Sci U S A (2004) 101, 7624-7629; Pierce A.J., Genes Dev (1999) 13, 2633-2638).

To elucidate if pro-fibrotic activity of EYA1A (*exon 10* included) was specifically due to its increased tyrosine phosphatase activity, we next utilized established EYA1 γH2A.X^{pY142} tyrosine phosphatase inhibitors Na₃VO₄ (blocking tyrosine phosphatase activity) and caffeine (blocking ATM-dependent recruitment of EYA1 towards yH2A.X) (Cook P.J., et al., Nature (2009) 458, 591-596; Rayapureddi, J.P., et al., Nature (2003) 426, 295-298). Either Na₃VO₄ or caffeine reversed EYA1A-induced premature γH2A.X^{pY142} dephosphorylation and fibroblast accumulation, whereas neither Na₃VO₄ nor caffeine affected γH2A.X^{pY142} in EYA1C overexpressing cells (Fig. 12A and B).

To gain insights into a possible mechanistic connection between previously observed γH2A.X clearance and γH2A.X^{pY142} dephosphorylation in EYA1A overexpressing mice, we next administered pre-determined doses of Na₃VO₄ (8 mg/kg i.p. once per day) or caffeine (12.5 mg/kg i.p. once per day) to *EYA1Atg* mice challenged with UUO (Rayapureddi et al., 2003, supra; Ostrowski J., et al., J Hepatol (2000) 32, 965-974; Barone L.M., et al., J Cell Biochem (1993) 52, 171-182). Na₃VO₄ and caffeine effectively blocked γH2A.X^{pY142} dephosphorylation and reversed the enhancing impact of EYA1A overexpression on renal fibrogenesis (Figs. 13 A to D), further supporting that the pro-fibrotic effect of EYA1A is dependent on its tyrosine phosphatase activity, and demonstrating that this effect can be pharmacologically targeted by inhibition of its tyrosine phosphatase activity.

Because inhibition of tyrosine phosphatase activity by Na₃VO₄ or caffeine is not limited to EYA1, the inventors next screened for additional compounds for selective modulation of EYA1A phosphatase activity. Among 736 compounds contained in the NCI mechanistic set, they identified 228 inhibitors of EYA1A phosphatase activity (Fig. 14).

### Example 4 - Intronic SNP rs13259388 affects EYA1 exon 10 splicing and disease progression in humans

The inventors next aimed to gain insights into the mechanisms underlying unfavourable EYA1 isoform by generating minigenes harbouring *exons 1-10* and intronic fragments flanking *exon 10* containing cis regulatory elements (-1165 relative to *intron 9-10*/*exon 10 junction,* Fig. 15C). Using *intron 9-10* deletion mutants, we identified a critical role of intronic region -920 bp to -634 bp (Figs. 15D to H).

Because the intronic region upstream EYA1 *exon 10* contains a predicted splicing cis regulatory motif (Fig. 15C) (Akerman M., et al., Genome Biol (2009) 10, R30; Paz I., et al., Nucleic Acids Res (2010) 38, W281-285), we administered morpholinos to block aforementioned splicing *cis* regulatory motif (ASmotif-893WT) and observed enhanced EYA1 *exon 10* inclusion (Fig. 16A), indicating that identified motif plays an important role in EYA1 *exon 10* splicing. Because this motif is disrupted by a common single nucleotide polymorphism at -893 bp (SNP rs13259388 C>T: ccggtgtgC/Tattttatg, Figs. 15A and B) with minor allelic frequency for "T" of 0.2242 in the 1000Genome phase 1 population with possibly broad clinical implication (C. Genomes Project et al., Nature (2012) 491, 56-65), we hypothesized that this polymorphism could affect EYA1 exon 10 splicing. To test this hypothesis, we next analyzed impact of a C>T point mutation at -893 bp (minigene 4 SNP, mirroring SNP rs13259388, Fig. 16B) on EYA1 exon 10 skipping and inclusion in renal fibroblasts. While renal fibroblasts transfected with minigene 4 harboring the wildtype "C" allele displayed exon 10 skipping, exon 10 was retained when the mutated minigene 4 SNP containing the "T" allele was used (Figs. 16C and D).

The inventors next screened and compared renal fibroblasts that had been established from patients homozygous for the wildtype "C" allele (TK173) or homozygous for the SNP rs13259388 "T" allele (TZ1, Fig. 16E). Homozygous SNP rs13259388 TZ1 cultures mirrored enhanced exon 10 inclusion (Fig. 16F), resulting in accelerated NHEJ DDR (Figs. 20A to 20F). Morpholinos to block SNP rs13259388 (ASmotif-893SNP) motif in TZ1 did not further enhance EYA1 *exon 10* inclusion (Fig. 16G), indicating that identified splicing cis regulatory motif plays an important role in EYA1 *exon 10* splicing and SNP *rs13259388* is associated with disruption of such motif. Increased EYA1 *exon 10* inclusion in TZ1 fibroblasts and TK173 transfected with ASmotif-893WT correlated with accelerated accumulation as compared to control TK173 fibroblast cultures (Fig. 16H).

The inventors hypothesized that SNP *rs13259388* was a risk factor for accelerated renal fibrosis. Within samples matched for fibrosis, fibroblast accumulation and renal excretory function, they observed increased EYA1 isoform switching towards EYA1A, lower abundance of γH2A.X^{pS139}, γH2A.X^{pY142} and enhanced NHEJ DDR among biallelic SNP *rs13259388* carriers (*SNP+*/*+,* Figs. 17A to I). Because corresponding fibroblast cultures isolated from kidneys of biallelic SNP *rs13259388* carriers accumulated more rapidly (Fig. 17J), ratio of EYA1A over EYA1C abundance within renal biopsies was previously associated with accelerated decline of renal function, and EYA1A overexpression had causally accelerated fibroblast accumulation and renal fibrogenesis in rodent models, we next analyzed disease progression towards ESRD in biallelic SNP *rs13259388* carriers. Independent of kidney fibrosis extent or renal excretory function (Figs. 19A to D), an overall 5.8-fold increase in relative risk to progress towards ESRD within 5 years among homozygous SNP *rs13259388* carriers resulted in ESRD within a mean of 14.3 months as compared to 30.5 months in patients negative for SNP *rs13259388* (Fig. 17K).

Because isoform EYA1A is not limited to SNP *rs13259388* carriers but is the predominant isoform in any fibrotic human kidney, the inventors also aimed to gain insights into additional mechanisms underlying EYA1 *exon 10* splicing. Transforming Growth Factor-beta1 (TGF-β1) is a principal mediator of fibrosis and fibroblast proliferation (Tampe & Zeisberg, 2014, supra), and identified splicing cis regulatory motif is a known binding site of TGF-β1-responsive splicing factors SRp40, SRp55 and SF2/ASF (Li X., & Manley J.L., Cell (2005) 122, 365-378). Accelerated fibroblast accumulation upon TGF-β1 exposure corresponded with increased mRNA expression of isoform *EYA1A* and decreased levels of isoform *EYA1C* and accelerated observed EYA1 isoform switch in TZ1 fibroblasts cultures (Figs. 18A to E), further corroborating relevance of TGF-β1 in alternative splicing regulation and suggesting that SNP *rs13259388* in humans accelerates EYA1 *exon 10* inclusion.

After it had been observed that forced EYA1 *exon 10* inclusion through oligonucleotides blocking identified splicing enhancer motif could induce the pro-fibrotic proliferative fibroblast phenotype, the inventors next asked if modulation of EYA1A *exon 10* alternative splicing (disrupting EYA1 tyrosine phosphatase activity) could restore physiological HR/NHEJ balance and susceptibility to checkpoint activation. By generating oligonucleotides targeting *intron 9-10*/*exon 10* and *exon 10*/*intron 10-11* splice junctions (EYA1Δe10-ASO), *exon 10* inclusion was effectively blocked (Fig. 21), correlating with normalization of premature γH2A.X^{pY142} dephosphorylation, increased HR DDR, attenuated proliferative activity and normalized DNA damage checkpoint activation **(****Figs. 20A to H** **),** confirming the causal link between SNP *rs13259388,* enhanced EYA1A generation and accelerated fibroblast proliferation and suggesting that SNP rs13259388-associated fibrosis risk can be therapeutically targeted in principle.

### Discussion

Sustained fibroblast proliferation is a hallmark of tissue fibrosis. In light of abundant DNA damage within fibrotic tissues that prompts epithelial cells to enter cell cycle arrest and senescence, fibroblast require distinct DDR pathways to enable sustenance of proliferation. Here, we demonstrate that accelerated recruitment of NHEJ core proteins enables proliferating fibroblasts to escape DNA damage checkpoint activation, and that this switch in humans is caused by premature dephosphorylation of the Y142 residue of γH2A.X through eyes absent homolog EYA1A as a critical *exon 10* is retained. The present data also show that the common intronic SNP *rs13259388* causally worsens clinical outcome of chronic kidney disease patients through accelerated EYA1 *exon 10* retention. Of note, this mechanism not only depends on presence of EYA1 tyrosine phosphatase activity, but also on recruitment of its substrate γH2A.X.

In this regard, relative abundance of H2A.X within nucleosomes differs substantially among cell types (Bonner W.M., et al., Nat Rev Cancer (2008) 8, 957-967, doi:10.1038/nrc2523; Rogakou, E. P., et al., J Biol Chem (1998) 139 273, 5858-5868). Within the kidney, H2A.X is far more abundant in fibroblasts than in tubular epithelial cells, and we did not observe an impact of EYA1A overexpression on tubular epithelial cell proliferation. The inventors' findings that EYA1A-induced premature γH2A.X^{pY142} dephosphorylation is associated with γH2A.X clearence is in line with existing literature demonstrating that phosphorylation of the S139 residue of H2A.X is critical for checkpoint activation upon DNA damage and that γH2A.X clearance terminates checkpoint activation (Stewart G.S., et al., Nature (2003) 421, 961-966). The present data unifies studies from different fields which demonstrated that γH2A.X is abundantly present within fibrotic human kidneys (Zhou et al., 2012, supra), that γH2A.X^{pY142} is a substrate of EYA1 tyrosine phosphatase activity (Cook et al., 2009, supra; Rayapureddi et al., 2003, supra), that γH2A.X is involved in DDR cell fate decisions (*13*), and that HR is a prerequisite for checkpoint activation (Stewart et al., 2003, supra). The present findings are also supported by publicly available CKD expression profiling datasets (GSE69438) that demonstrate that accelerated fibrosis correlated with increased expression of NHEJ core proteins (and decreased expression of HR) (Ju W., et al., Sci Transl Med (2015) 7, 316ra193).

It is furthermore shown that aberrant EYA1 *exon 10* inclusion is enhanced by intronic, noncoding SNP *rs13259388,* a common variant, in a disease relevant manner (Genomes Project et al., 2012, supra). Generally, EYA1 splicing does not occur in any other species but humans, not even in other primates (Aken, B.L., et al. The Ensembl gene annotation system. Database (Oxford) 2016 (2016), doi:10.1093/database/baw093). The relevance of SNP *rs13259388* for EYA1 *exon 10* retention is supported by phylogenetic analysis which revealed presence of a conserved genomic region within *EYA1* corresponding to identified intronic splicing *cis* regulatory motif among primate species in which EYA1 is not alternatively spliced and EYA1 *exon 10* is retained (Aken et al., 2016, supra).

Plausibility of identified impact of SNP *rs13259388* as critical splicing cis regulatory motif of EYA1 *exon 10* splicing is supported by Mfold pre-mRNA secondary structure analysis, predicting loop formation through RNA bridges connecting identified distal splicing cis regulatory motif to proximity of EYA1 *exon 10* (Fig. 29B), in effect recapitulating the classical example of alternative splicing regulation proximal to alternative exons (Lovci, M.T., et al. Nat Struct Mol Biol (2013) 20, 1434-1442).

It can furthermore be taken from Fig. 29 that EYA1 *exon 10* spicing is not limited to the kidney, but also present in chronically injured heart, lung and liver. This is in line with mining of publicly available human exon array datasets (GSE41409): compared to normal kidney, EYA1 *exon 10* splicing is equally detectable in brain, colon, lung, and heart (Figs. 29C to G; Ling, M.H., et al., BMC Genomics (2013) 14, 243). In light of the absence of clinically approved drugs to target progressive kidney fibrosis, the present findings point to several new targets for possible therapeutic intervention such as modulation of EYA1 splicing, inhibition of EYA1 tyrosine phosphatase activity and/or specific targeting of the NHEJ pathway or DNA damage checkpoint activation. Hence, the present inventors have identified a unique mechanism of alternative splicing, allowing dynamic realization of serine phosphatase/transcription factor and tyrosine/histone γH2A.X^{pY142} phosphatase activity of EYA1, and providing several novel therapeutic targets to inhibit fibrogenesis of the kidney.

### Materials and Methods

### Human kidney specimens

The use of parts of human kidney biopsies for research purposes was approved by the Ethics Committee of the University Medicine Goettingen, and written consent was obtained from all subjects before kidney biopsy.

### Animals

All studies and inclusion/exclusion of animals were performed according to the German animal care and ethics legislation and had been carried out with the approval of the local government authorities (LAVES) and the University Medicine Goettingen. Experimental protocols are detailed below.

### Generation of doxycycline (DOX)-inducible transgenic mice

German All transgenic mouse strains were obtained by the same founders and did not obtain spontaneous phenotypes. From 3 days before surgery, *C57BL*/*6* (referred as *wildtype*) or transgenic mice overexpressing human splice variants EYA1A (*rtTACMV;hEYA1A-pTreTight,* referred to as *EYA1A^{tg}*) and EYA1C (*rtTACMYV;hEYA1C-pTreTight,* referred to as *EYA1C^{tg}*) were treated with either vehicle buffer (glucose 5%) or DOX (2 mg/ml, Sigma, St. Louis, USA) within drinking water *ad libidum* and renewal every 2 days.

### Unilateral ureteral obstruction (UUO)

Mice were anesthetized with isoflurane inhalation (2-3%), analgesia was performed by subcutaneous injection of 0.1 mg/kg body weight per day Buprenorphine. The ureter was separated from the surrounding tissues and two ligatures were placed about 5 mm apart in upper two-thirds of the left ureter to obtain reliable obstruction. Mice were sacrificed 3, 7 or 10 days after ureteral obstruction for further analyses.

### 5/6 nephrectomy (5/6Nx)

The left kidney was exposed via a mid-line incision and the renal capsule was removed. The renal pedicle was temporarily clamped and approximately 7/8 of cortex was resected. The remnant kidney was wrapped with part of the mesentery and the clamp on the renal pedicle was removed, bleeding from the cut surfaces being prevented by 5 minutes of digital pressure. The abdominal incision was then closed and mice were allowed to recover.

### Na₃VO₄ and caffeine treatment

Mice were injected intraperitoneally with Na₃VO₄ (8 mg/kg i.p. once per day, Sigma, St. Louis, USA) or caffeine (12.5 mg/kg i.p. once per day, Sigma, St. Louis, USA) starting one day prior of surgery, control mice received equivalent volume of PBS or DMSO.

### Lentivirus packaging

Lentiviral *pLenti-SceI* was produced in 293T virus packaging cells after transfection using a Packaging System Mix (Abmgood, Richmond, Canada) according to the manufacture's protocol. Lentiviral supernatant was collected 3 days after transfection, filtered through a 0.45 µm filter and concentrated with ultracentrifugation at 28,000 g at 4°C using a 4% sucrose/PBS cushion for 3 hours. After supernatant removal viral particles were resuspended in PBS and stored at -80°C. Lentivirus titration was determined by Lenti-X qRTPCR Titration Kit (Clonetech, Heidelberg, Germany) and 8 µg/mL Polybrene (Sigma, St. Louis, USA) was used for *in vivo* application.

### EYA1A^{tg};R26NHEJ transgenic reporter mice

*R26NHEJ* mice were crossed with *EYA1A^{tg}* to generate *EYA1Atg;R26NHEJ* reporter mice. Mice were challenged with UUO and locally injected with intracortical *pLenti-SceI* (5x108 TU/100 µL) at 5 injection sites each.

### SCR7 treatment

Mice were injected intraperitoneally with SCR7 (20 mg/kg i.p. every alternate day, Sigma, St. Louis, USA) starting one day prior of surgery, control mice received equivalent volume of DMSO.

### In vivo morpholino (VMO) treatment

The inventors obtained 25 nucleotide antisense morpholino sequences (Gene Tools, Philomath, USA). The *in vivo* morpholinos contain a covalently linked octaguanidine dendrimer as a delivery moiety to facilitate entry into cells *in vivo.* Mice were injected intraperitoneally with 12.5 mg/kg body weight *in vivo* morpholinos in saline at a final volume of 100 µL every other day starting two days prior of surgery. A control *in vivo*

### Lentivirus packaging

Lentiviral *pLenti-SceI* was produced in 293T virus packaging cells after transfection using morpholino that targets a human β-globin intron mutation was used as standard control. *In vivo* morpholino sequences are those of SEQ ID NOs: 9 (EYA1A-VMO), 10 (53Bp1-VMO) and 16 (control).

### Histology

Formalin-fixed, paraffin-embedded kidneys and hearts from mice, and HOPE- fixed, paraffin-embedded human specimens were sectioned at 3 µm, Masson's Trichrome Stain (MTS) was performed at the University Medicine Göttingen. For morphometric analysis of interstitial fibrosis in mice and human biopsies, we assessed the fibrotic area using cellSens (Olympus, Tokyo, Japan) software. Ten visual fields were selected randomly for each MTS stained section at 200x magnification and the relative interstitial fibrotic area was evaluated by using a 10 mm² graticule.

### Immunohistochemistry

Formalin-fixed, paraffin-embedded kidneys and hearts from mice, and HOPE-fixed, Formalin-fixed, paraffin-embedded kidney sections from mice as well as from HOPE-fixed, paraffin-embedded human specimens were deparaffinized in xylene and rehydrated in ethanol containing distilled water. Tissue sections were stained using antibodies against p-Chk1^{pS317} (12302P, Cell Signaling, Danvers, USA), Rad51 (sc-8349, Santa Cruz Biotechnology, Dallas, USA), Xrcc4 (sc-8285, Santa Cruz Biotechnology, Dallas, USA), H2A (ab13923, Abcam Biochemicals, Cambridge, UK), H2A.X (ab11175, Abcam Biochemicals, Cambridge, UK), γH2A.X^{pS139} (ab2893, Abcam Biochemicals, Cambridge, UK) and γH2A.X^{pY142} (ab94602, Abcam Biochemicals, Cambridge, UK), peroxidase labeling was performed using Vectastain Universal Elite ABC Kit (Vector Laboratories, Burlingame, USA) according to the manufacturer's protocol.

AEC Substrate-Chromogen (Dako, Glostrup, Denmark) was applied for peroxidase visualization according to the manufacturer's protocol. Nuclear counterstain was performed by using Mayer's Hematoxylin Solution (Sigma, St. Louis, USA).

### Immunofluorescence staining of tissue sections

Formalin-fixed, paraffin-embedded kidney sections from mice as well as from HOPE-fixed, paraffin-embedded human specimens were deparaffinized in xylene and rehydrated in ethanol containing distilled water. For immunofluorescent staining, EYA1 isoform antisera or primary antibodies against αSMA (A5228, Sigma, St. Louis, USA), Collagen-1 (ab34710, Abcam Biochemicals, Cambridge, UK), γH2A.X^{pS139} (ab2893, Abcam Biochemicals, Cambridge, UK), γH2A.X^{pS139} (05-636, Merck Millipore, Billerica, UK), γH2A.X^{pY142} (ab94602, Abcam Biochemicals, Cambridge, UK), p-Chk1^{pS317} (12302P, Cell Signaling, Danvers, USA), p-Chk2^{pT68} (2197S, Cell Signaling, Danvers, USA), Rpal (2267S, Cell Signaling, Danvers, USA), Rad51 (sc-8349, Santa Cruz Biotechnology, Dallas, USA), 53Bp1 (sc-22760, Santa Cruz Biotechnology, Dallas, USA), Xrcc4 (sc-8285, Santa Cruz Biotechnology, Dallas, USA) and GFP (MA5-15256, Thermo Fisher Scientific, Waltham, USA) were used, secondary antibodies were labeled with Alexa Fluor 488, 594 or 647 (Life Technologies, Carlsbad, USA). Renal basement membranes were stained with antibodies against Collagen-4 (1340-30, SouthernBiotech, Birmingham, USA). Nuclear staining was performed using 4',6-diamidino-2-phenylindole (DAPI, Vector Laboratories, Burlingame, USA). Quantifications were performed per high power field at 400x magnification, representative confocal pictures using an LSM 780 confocal laser scanning microscope (Zeiss, Oberkochen, Germany) are shown.

### Primary mouse fibroblasts

For isolation of primary mouse fibroblasts, freshly isolated kidneys were cut into small pieces (1-2 mm³) and transferred into sterile filtered Collagenase Type II (100 U/mL) dissolved in serum free Dulbecco's modified Eagle medium (Gibco, Carlsbad, USA). After 45 minutes of digestion at 37°C with mild agitation, cells were spun down and transferred into Dulbecco's modified Eagle medium (Gibco, Carlsbad, USA) supplemented with 2 mmol/L L-glutamine, 100 g/ml penicillin, 100 g/ml streptomycin, and 20% heat-inactivated fetal bovine serum (Cellgro, Manassas, USA) at 37 °C in 5% CO₂. Primary kidney fibroblasts emerged within a few days as adherent cells on the monolayer and were maintained in Dulbecco's modified Eagle medium (Gibco, Carlsbad, USA) supplemented with 2 mmol/L L-glutamine, 100 g/mL penicillin, 100 g/mL streptomycin, and 10% heat-inactivated fetal bovine serum (Cellgro, Manassas, VA) at 37 °C in 5% CO₂. For all experiments, fibroblasts from passages 1 to 3 were used.

### Primary human fibroblasts

The use of parts of nephrectomy specimens or kidney biopsies for research purposes was approved by the ethics committee of the Medical University of Goettingen, and written consent was obtained from all patients prior to kidney biopsy. For isolation of primary human kidney fibroblasts, renal biopsy cylinders were cut and immersed in Dulbecco's modified Eagle medium (Gibco, Carlsbad, USA) supplemented with 2 mmol/L L-glutamine, 100 g/ml penicillin, 100 g/ml streptomycin, and 20% heat-inactivated fetal bovine serum (Cellgro, Manassas, USA) at 37 °C in 5% CO₂ until fibroblasts had grown out to confluency. Primary human fibroblasts were used at passages 2 and 3. The use of parts of nephrectomy specimens or kidney biopsies for research purposes was approved by the ethics committee of the Medical University of Goettingen, and written consent was obtained from all patients prior to kidney biopsy. For isolation of primary human kidney fibroblasts, renal biopsy cylinders were cut and immersed in Dulbecco's modified Eagle medium (Gibco, Carlsbad, USA) supplemented with 2 mmol/L L-glutamine, 100 g/ml penicillin, 100 g/ml streptomycin, and 20% heat-inactivated fetal bovine serum (Cellgro, Manassas, USA) at 37 °C in 5% CO₂ until fibroblasts had grown out to confluency. Primary human fibroblasts were used at passages 2 and 3.

### Cell culture

Human SV40/large T antigen-immortalized monoclonal kidney fibroblasts (TK173 and TZ1) were cultured in Dulbecco's modified Eagle medium (Gibco, Carlsbad, USA) supplemented with 2 mmol/L L-glutamine, 100 g/mL penicillin, 100 g/mL streptomycin, and 10% heat-inactivated fetal bovine serum (Cellgro, Manassas, VA) at 37°C in 5% CO₂. HK-2 (ATTC, Manassas, USA) are immortalized proximal tubule epithelial cells derived from normal adult human kidney. All cells were routinely tested negative for the presence of mycoplasma contamination. None of the cell lines used in this manuscript is listed in the ICLAC and NCBI Biosample database of misidentified cell lines. Cells were cultured in Dulbecco's modified Eagle's (DMEM, Gibco, Carlsbad, USA) medium supplemented with 100 g/mL penicillin, 100 g/mL streptomycin and 10% heat-inactivated fetal bovine serum (FBS, Sigma, St. Louis, USA) at 37°C in 5% CO₂.

### G2 cell synchronization and neocarzinostatin (NCS) treatment

Transgenic *EYA1A^{tg}* and *EYA1C^{tg}* primary fibroblast cultures were seeded in 8 well chamber slides (Thermo Fisher Scientific, Waltham, USA) at a concentration of 2 x 10³ cells per well in antibiotic-free DMEM (Gibco, Carlsbad, USA) supplemented with 10% heat-inactivated fetal bovine serum (FBS, Sigma, St. Louis, USA). After 12 hours, thymidine (2 mM, Sigma, St. Louis, USA) and DOX (1 µg/mL, Sigma, St. Louis, USA) were added to culture media for 20 hours and replaced by antibiotic-free DMEM (Gibco, Carlsbad, USA) supplemented with 10% heat-inactivated FBS (Sigma, St. Louis, USA) and DOX (1 µg/mL, Sigma, St. Louis, USA). After 4 hours, nocodazole (25 ng/mL, Sigma, St. Louis, USA) and DOX (1 µg/mL, Sigma, St. Louis, USA) were added to culture media for additional 12 hours. Culture media was replaced by antibiotic-free DMEM (Gibco, Carlsbad, USA) supplemented with 10% heat-inactivated FBS (Sigma, St. Louis, USA) and NCS (10 ng/mL, Sigma, St. Louis, USA), cells were collected at indicated time points. For overexpression experiments, murine and human fibroblast cultures were seeded in 8 well chamber slides (Thermo Fisher Scientific, Waltham, USA) at a concentration of 2 x 10³ cells per well in antibiotic-free DMEM (Gibco, Carlsbad, USA) supplemented with 10% heat-inactivated fetal bovine serum (FBS, Sigma, St. Louis, USA).

After 24 hours, 2 µg plasmid DNA was transfected using Lipofectamine 2000 reagent (Invitrogen, Carlsbad, USA) for 4 hours and culture media was replaced by antibiotic-free DMEM (Gibco, Carlsbad, USA) supplemented with 10% heat-inactivated FBS (Sigma, St. Louis, USA). After additional 20 hours, thymidine (2 mM, Sigma, St. Louis, USA) was added to culture media for 20 hours and replaced by antibiotic-free DMEM (Gibco, Carlsbad, USA) supplemented with 10% heat-inactivated FBS (Sigma, St. Louis, USA). After 4 hours, nocodazole (25 ng/mL, Sigma, St. Louis, USA) was added to culture media for additional 12 hours. Culture media was replaced by antibiotic-free DMEM (Gibco, Carlsbad, USA) supplemented with 10% heat-inactivated FBS (Sigma, St. Louis, USA) and NCS (10 ng/mL, Sigma, St. Louis, USA), cells were collected at indicated time points.

### Immunofluorescence staining of fixed cells

Cells were fixed in 4% paraformaldehyde (PFA) for 10 minutes at room temperature, permeabilized with 0.5% Triton-X 100 for 10 minutes and blocked with 3% bovine serum albumin (BSA) for 1 hour at 4°C. For immunofluorescent staining, primary antibodies against γH2A.X^{pS139} (05-636, Merck Millipore, Billerica, UK), γH2A.X^{pY142} (ab94602, Abcam Biochemicals, Cambridge, UK), Rpa1 (2267S, Cell Signaling, Danvers, USA), Rad51 (sc-8349, Santa Cruz Biotechnology, Dallas, USA), 53Bp1 (sc-22760, Santa Cruz Biotechnology, Dallas, USA), Xrcc4 (sc-8285, Santa Cruz Biotechnology, Dallas, USA), DDK (TA50011-100, OriGene, Rockville, USA) were used, secondary antibodies were labeled with Alexa Fluor 488, 594 or 647 (Life Technologies, Carlsbad, USA). Nuclear staining was performed using DAPI (Vector Laboratories, Burlingame, USA). An LSM 780 confocal laser scanning microscope (Zeiss, Oberkochen, Germany) was used, representative confocal pictures are shown.

### COMET assay

Neutral COMET assay was performed as recommended by the manufacturer (Trevigen, Gaithersburg, USA). Briefly, a suspension of 500 cells were included in low-melting agarose and run on a pre-coated agarose-covered slide. After the agarose gelled, slides were run at 21 V for 30 minutes at 4°C and subsequently stained with SYBR Green diluted 1:10,000 in TE buffer (pH 7.5).

### NHEJ/HR reporter plasmids

Primary murine fibroblast cultures were stably transfected using Lipofectamine 2000 reagent as recommended by the manufacturer (Invitrogen, Carlsbad, USA). Briefly, 2 µg of circular *pEGFP-Pem1-Ad2* or *pDR-GFP* (AddGene, Cambridge, USA), resistant colonies were selected with 6 µg/mL puromycin (Thermo Fisher Scientific, Waltham, USA). After transfection of an I-SceI rare-cutting endonuclease from Saccharomyces cerevisiae expression vector (*pCBASceI*, AddGene, Cambridge, USA) to introduce a DSB at genomic I-SceI sites, NHEJ/HR repair events were visualized by presence of cells positive for EGFP/GFP.

### Morpholino treatment in vitro

For *in vitro* experiments, a final concentration of 10 µM morpholinos (without a covalently linked octaguanidine dendrimer) blocking the intronic splicing *cis* regulatory *wildtype* (ASmotif-893WT) or SNP *rs13259388* motif (ASmotif-893SNP) were scrape delivered to TK173 or TZ1, respectively. A control morpholino that targets a human β-globin *intron* mutation was used as standard control (control-VMO: SEQ ID NO: 16; EYA1A-VMO: SEQ ID NO: 9; 53BP1-VMO: SEQ ID NO: 10).

### Antisense oligonucleotides

Oligonucleotides targeting *intron 9-10*/*exon 10* (EYA1Δe10-5'ASO, SEQ ID NO: 5) and *exon 10*/*intron 10-11* (EYA1Δe10-3'ASO, SEQ ID NO: 4) splice junctions (referred to as EYA1Δe10-ASO) were transfected using Lipofectamine 2000 reagent (Invitrogen, Carlsbad, USA) for 4 hours and culture media was replaced by antibiotic-free DMEM (Gibco, Carlsbad, USA) supplemented with 10% heat-inactivated FBS (Sigma, St. Louis, USA).

### Peptide synthesis and antibody generation

The antigenic peptides were selected from the protein sequence using the Lasergene software (DNASTAR, Madison, USA) and additionally for non-homologeous sequence pattern: The peptides were synthesized with an additional N-terminal cysteine using standard Fmoc/tBu chemistry on a multiple peptide synthesizer, Syro II (MultiSynTech, Witten, Germany) as recently described. The single peptides were purified using reversed phase-HPLC and their identity was confirmed using ESI-MS and MALDI-TOF-MS. Peptide purities were >95% as determined by analytical reversed phase-HPLC. Four peptides as antigens were used: CEEMIFNLADTHLF FNDLE [330-347, SEQ ID NO: 38] (EYA1 isoform A), CGSSESPSGPKLGNSHINS [15-32, SEQ ID NO: 17] (EYA1 isoform C), CMEMQDLTSPHSRLSGSSES [1-19, SEQ ID NO: 18] (EYA1 isoform C), GISSYGIKTEGGLS [135-148, SEQ ID NO: 19] (EYA1 isoform C). The peptides were specifically coupled via their N-terminal cysteine to maleimidobenzoyl-functionalized-keyhole hole hemocyanin (MBS-KLH) and the antisera were obtained after repeated immunization of rabbits with a 1: 1-mixture of the peptide-KLH conjugate.

### Mutant constructs

Mutant EYA1A in which *exon 10* had been removed (*EYA1AΔe10*) was generated by amplification from EYA1A overexpression vector (OriGene, Rockville, USA) with specific restriction enzyme sites for *exons 1-9* (EcoRI and KpnI) and *exons 11-16* (KpnI and Xho). Mutant EYA1C where *exon 1'* was replaced with *exon 1* (*EYA1CΔ1'::e1*) was generated by amplification from EYA1A and EYA1C overexpression vectors (OriGene, Rockville, USA) with specific restriction enzyme sites for *exons 1-4* (EcoRI and NheI) and *exons 5-16* (NheI and XhoI), respectively. Mutant EYA1C to which *exon 10* had been added (*EYA1C::e10*) was generated by amplification from EYA1C and EYA1A overexpression vectors (OriGene, Rockville, USA) with specific restriction enzyme sites for *exons 1'-9*/*11-16* (BglII and HindIII) and *exon 10* (HindIII and BglII), respectively. Standard PCR reactions with high-fidelity taq-polymerase under condition of denaturing at 98°C for 30 seconds, annealing at 72°C for 30 seconds and elongation at 72°C for 30 seconds to 45 seconds depending on the length of the fragment for 45 cycles were used. Generated fragments were cloned, constructs were validated by sequencing of the connected regions.

### Minigene constructs

Minigens harboring *exons 1-10* and intronic fragments flanking *exon 10* containing cis regulatory elements -1165 relative to *intron 9-10*/*exon 10* junction (*minigene 3*), lacking -1165 bp to - 920 bp relative to *intron 9-10*/*exon 10* junction (*minigene 4*) or lacking -1164 bp to -634 bp relative to *intron 9-10*/*exon 10* junction (*minigene 5*) were generated. EYA1 *intron 9-10, exon 10 and intron 10-11* fragments were amplified from bacclone *RP11-11K9* (minigenes 3, 4 and 5) or genomice DNA extracted from human renal fibroblasts which had been established from patients homozygous for the SNP *rs13259388* "T" allele (TZ1^{*SNP*+/+}) at -893 bp relative to *intron 9-10*/*exon 10* junction *(minigene 4 SNP)* with specific restriction enzyme sites (KpnI and NotI) by standard PCR reactions with high-fidelity taq-polymerase under condition of denaturing at 98°C for 30 seconds, annealing at 72°C for 30 seconds and elongation at 72°C for 30 seconds to 45 seconds depending on the length of the fragment for 45 cycles.

These fragments were cloned into *pCMV6-Entry* mammalian vector (OriGene, Rockville, USA), constructs were validated by sequencing of the connected regions. After detecting the right construct, DNA was isolated with MINI Prep Kit (Qiagen, Hilden, Germany). EYA1 *exon 1-9* fragments were amplified from EYA1 Isoform A overexpression plasmid with specific restriction enzyme sites (NheI and KpnI). This fragment was cloned into the constructs containing one of the fragments with *exon 10.* Again, constructs were validated by sequencing of the connected regions.

### RNA isolation

Total RNA was extracted from cells using TRIzol Reagent (Life technologies, Carlsbad, USA), tissue was shredded using TissueLyser LT (Qiagen, Hilden, Germany). Subsequent RNA purification procedure was performed by PureLink RNA Mini Kit (Ambion, Carlsbad, USA) according to the manufacturer's protocol.

### RNA sequencing and gene set enrichment

Total RNA extracted from UUO kidneys were submitted to Transcriptome Sequencing Service of Eurofins Genomics and provided in GEO (accession number GSE92934, reviewer link: https://www.ncbi.nlm.nih.gov/geo/query/acc.cgi?token=wnatgiqsrpytxcv&acc=GSE92934). Raw read counts were created using HTSeq. Only reads with unique mapping positions were considered for read counting. Only reads overlapping exon-features were counted. All reads mapping to features with the same identifer were summed, the "gene"-attribute was used as feature identifer. Reads mapping to multiple features with different identifer were ignored for read counting. Gene set enrichment and process analysis (values of *p<0.001*) were performed using Gene Ontology enRIchment analysis and visuaLizAtion tool (GORILLA).

### Quantitative real-time PCR quantification (qRT-PCR)

qRT-PCR analyses were performed using the ABI One Step Plus instrument and software (PE Applied Biosystems, Inc., Foster City, CA). Using predesigned primers for total *EYA1* (Hs00166804_m1, Applied Biosystems, Carlsbad, USA), *EYA1A* (Hs01011616_m1, Applied Biosystems, Carlsbad, USA) and *EYA1C* (Hs01004900_g1, Applied Biosystems, Carlsbad, USA), qRT-PCR was performed with the TagMan^{®} Universal PCR Master Mix (Applied Biosystems, Carlsbad, USA) using 10 µL of diluted total RNA, 10 µmol/L of the probe, and 10 µmol/L primers in a 50 µl final reaction mixture. Reverse transcription of input RNA to cDNA was performed at 48°C for 30 minutes. After 10 minutes incubation at 95°C, 40 PCR cycles were performed consisting of 15 seconds of denaturation at 95°C and hybridization of probe and primers for 1 minute at 60°C each. Reactions were done in triplicates.

Relative expression levels were determined using standard methods. The gene expression value for the gene of interest was normalized to the value determined from rRNA *18S* to adjust variances in the quality of RNA and the amount of input RNA. For SYBR-based real-time PCR, cDNA synthesis was performed by using DNase I digestion (Invitrogen, Carlsbad, USA) and SuperScript II Reverse Transcriptase (Invitrogen, Carlsbad, USA) according to the manufacturer's protocol. Briefly, 1 µL of reverse-transcribed cDNA was added to the reaction mixture containing the primer pair (200 nmol/L each) and diluted 2x Fast SYBR Green Master Mix (Applied Biosystems, Carlsbad, USA) in a final volume of 20 µL for each PCR reaction. The real-time PCR reactions were performed in a 96-well reaction plate using the StepOne Plus Real-Time System (Applied Biosystems, Carlsbad, USA) and were done in triplicates. An initiation step at 95°C for 20 seconds was followed by 40 cycles at 95°C for 3 seconds and 60°C for 30 seconds, with one cycle of dissociation at 95°C for 15 seconds, 60°C for 60 seconds, and 95°C for 15 seconds.

The intercalation of SYBR Green dye and its fluorescent signal is directly proportional to the amount of amplified DNA and was transformed into the cycle threshold (Ct). For normalization, the Ct values of the housekeeping genes *Gapdh*/*GAPDH* were subtracted from the Ct values of the gene of interest to generate the dCt values. The relative expression levels were analyzed in technical triplicates and calculated using the equation 2^{-ddCt}. Sources and sequences of the oligonucleotides are listed in Fig. 35.

Electrophoresis of PCR products was done on a Bioanalyzer 2100 (Agilent Technologies, Santa Clara, USA) according to the manufacturer's protocol. Electrophoresis results are shown as virtual gel images and DNA concentrations relative to corresponding input DNA as described in our previous publications.

### Western blot analyses

Tissue and cells were homogenized in NP40 lysis buffer (Life technologies, Carlsbad, USA) supplemented with protease inhibitor cocktail (Roche, Basel, Switzerland). After sonication, protein samples were resolved by a 4-12% Bis-Tris polyacrylamide gel electrophoresis system (Novex, Carlsbad, USA) and transferred onto a nitrocellulose membrane (GE Healthcare, Freiburg, Germany), followed by a blocking step with 5% dry milk or 5% BSA in TBST (TBS pH 7.2, 0.1% Tween-20) to prevent unspecific bindings.

After incubation with EYA1 isoform antisera or respective primary antibodies against H2A.X (ab11175, Abcam Biochemicals, Cambridge, UK), γH3^{pS10} (ab47297, Abcam Biochemicals, Cambridge, UK), p-Chk1^{pS317} (12302P, Cell Signaling, Danvers, USA), p- Chk2^{pT68} (2197S, Cell Signaling, Danvers, USA), β-actin (A5316, Sigma, St. Louis, USA) and Gapdh (5G4, HyTest, Turku, Finland), secondary HRP-conjugated antibodies were used (Dako, Glostrup, Denmark). Luminescence was detected by using chemiluminescent substrate (Cell Signaling, Danvers, USA) on a ChemiDoc XRS system (Bio-Rad, Hercules, USA).

### pNPP assay

p-Nitrophenyl Phosphate (PNPP) is a non-proteinaceous, non-specific substrate used in the art to assay protein, alkaline and acid phosphatases. The PNPP phosphatase activity is measured using a continuous or single-point spectrophotometric assay based on the ability of phosphatases to catalyze the hydrolysis of PNPP to p-nitrophenol, a chromogenic product with absorbance at 405 nm. Any available protocol can be followed to determine phosphatase activity using pNPP.

### Phosphatase assay

Tyrosine and serine phosphatase activities in total cell lysates were determined using phosphotyrosine (V2471, Promega, Fitchburg, USA) and phosphoserine (17-128, Merck Millipore, Darmstadt, Germany) phosphatase assay systems according to the manufacturer's protocol.

### Analyses of publicly available array datasets

Human transcriptome array data are shown as log2 median centered intensities extracted from Nephroseq database (accession number GSE69438). To predict differential splicing events for *EYA1,* exon array CEL files (GEO accession number GSE41409) were taken into account and analyzed using AltAnalyze software and default parameters referenced to normal kidney exon array datasets. Splicing index algorithm were taken into account to assess splicing events, splicing index scores were obtained by subtracting normalized intensities of reference from experimental groups. Splicing index of 0 indicates equal inclusion rates between the two groups, positive values indicate enrichment of exons in experimental groups and negative values indicate enrichment of exons in the control groups as compared to experimental groups.

### Identification of candidate cis regulatory elements

Candidate cis regulatory elements in alternative and constitutive splicing of mammalian exons was performed using SFmap with the SNP *rs13259388* flanking region.

### RNA secondary structure prediction

Prediction of RNA bridge formation upstream EYA1 *exon 10* was performed using Mfold.

### Statistical analysis

The numbers of biological replicates are indicated in the corresponding figure legends. For single group comparison, Student's t test was used to determine statistical significance. One-way ANOVA with Bonferroni post-hoc analysis was used for multiple comparisons of samples to determine statistical significance. Linear regression was performed comparing indicated pairs of parameters, *r²* and values of *p* are indicated in the corresponding graphs. Statistical significance was defined as values of *p*<*0.05,* indicated as * *p*<*0.05*, ** *p<0.01*, *** *p<0.001* or **** *p<0.0001.* Prism 5 software (GraphPad, La Jolla, USA) was used for statistical analysis.

## Claims

1. A compound that reduces the level of protein variant EYA1A in a parenchymal organ for use in a method of preventing, treating or delaying progression of fibrosis and/or desmoplasia in said parenchymal organ, wherein the compound is an antisense oligonucleotide against at least one of a portion of at least 11 consecutive bases within the sequence of SEQ ID NO: 34 and/or is a reverse complement of the portion within the sequence of SEQ ID NO: 34, an antisense oligonucleotide against at least one of a portion of at least 11 consecutive bases within the sequence of SEQ ID NO: 35 and/or is a reverse complement of the portion within the sequence of SEQ ID NO: 35, an antisense oligonucleotide against at least one of a portion of at least 11 consecutive bases within the sequence of SEQ ID NO: 36 and/or is a reverse complement of the portion of the sequence of SEQ ID NO: 36.

2. The compound for use of claim 1, wherein the aberrant fibroblast proliferation is at least one of desmoplasia and fibrosis.

3. The compound for use of claim 2, wherein the antisense oligonucleotide against a portion of at least 11 consecutive bases within the sequence of SEQ ID NO: 34 is
(a) an antisense oligonucleotide against the sequence of SEQ ID NO: 39; a sequence that is reverse complementary to the sequence of SEQ ID NO: 39; a sequence that is reverse complementary to a portion of the sequence of SEQ ID NO: 39; or a reverse complement of a portion of SEQ ID NO: 39; or
(b) an antisense oligonucleotide against the sequence of SEQ ID NO: 5 or a sequence that is reverse complementary to the sequence of SEQ ID NO: 5.

4. The compound for the use of claim 2, wherein the antisense oligonucleotide against a portion of at least 11 consecutive bases of the sequence of SEQ ID NO: 35 is
(a) an antisense oligonucleotide against the sequence of SEQ ID NO: 40; a sequence that is reverse complementary to the sequence of SEQ ID NO: 40; a sequence that is reverse complementary to a portion of the sequence of SEQ ID NO: 40; or a reverse complement of a portion of SEQ ID NO:_40; or
(b) an antisense oligonucleotide against the sequence of SEQ ID NO: 4 or a sequence that is reverse complementary to the sequence of SEQ ID NO: 4.

5. The compound for use of claim 1 or 2, wherein the antisense oligonucleotide against a portion of at least 11 consecutive bases of the sequence of SEQ ID NO: 36 is an antisense oligonucleotide of SEQ ID NO: 9.

6. The compound for use of any of the preceding claims, wherein the antisense oligonucleotide is a phosphorodiamidate morpholino oligonucleotide.

7. The compound for use of any of the preceding claims, wherein the subject expresses a naturally occurring EYA1 protein comprising the sequence of SEQ ID NO: 2.

8. The compound for use of claim 7, wherein the nucleic acid sequence encoding the protein EYA1 comprises the nucleic acid sequence of SEQ ID NO: 7 or the nucleic acid sequence of SEQ ID NO: 8.

## Patentansprüche

1. Verbindung, die den Gehalt der Proteinvariante EYA1A in einem Parenchymorgan senkt, zur Verwendung in einem Verfahren zur Vorbeugung, Behandlung oder Verzögerung des Fortschreitens von Fibrose und/oder Desmoplasie in dem Parenchymorgan, wobei die Verbindung ein Antisense-Oligonukleotid gegen mindestens einen Abschnitt von mindestens 11 aufeinanderfolgenden Basen innerhalb der Sequenz von SEQ ID NO: 34 und/oder ein Reverse-Komplement des Abschnitts innerhalb der Sequenz von SEQ ID NO: 34 ist, ein Antisense-Oligonukleotid gegen mindestens einen Abschnitt von mindestens 11 aufeinanderfolgenden Basen innerhalb der Sequenz von SEQ ID NO: 35 und/oder ein Reverse-Komplement des Abschnitts innerhalb der Sequenz von SEQ ID NO: 35 ist, ein Antisense-Oligonukleotid gegen mindestens einen Abschnitt von mindestens 11 aufeinanderfolgenden Basen innerhalb der Sequenz von SEQ ID NO: 36 und/oder ein Reverse-Komplement des Abschnitts der Sequenz von SEQ ID NO: 36 ist.

2. Verbindung zur Verwendung nach Anspruch 1, wobei die abnorme Fibroblastenproliferation mindestens eines von Desmoplasie und Fibrose ist.

3. Verbindung zur Verwendung nach Anspruch 2, wobei das Antisense-Oligonukleotid gegen einen Abschnitt von mindestens 11 aufeinanderfolgenden Basen innerhalb der Sequenz von SEQ ID NO: 34
(a) ein Antisense-Oligonukleotid gegen die Sequenz von SEQ ID NO: 39 ist; eine Sequenz, die reverskomplementär zur Sequenz von SEQ ID NO: 39 ist; eine Sequenz, die reverskomplementär zu einem Abschnitt der Sequenz von SEQ ID NO: 39 ist; oder ein Reverse-Komplement eines Abschnitts von SEQ ID NO: 39 ist; oder
(b) ein Antisense-Oligonukleotid gegen die Sequenz von SEQ ID NO: 5 oder eine Sequenz, die zur Sequenz von SEQ ID NO: 5 reverskomplementär ist.

4. Verbindung zur Verwendung nach Anspruch 2, wobei das Antisense-Oligonukleotid gegen einen Abschnitt von mindestens 11 aufeinanderfolgenden Basen der Sequenz von SEQ ID NO: 35
(a) ein Antisense-Oligonukleotid gegen die Sequenz von SEQ ID NO: 40 ist; eine Sequenz, die reverskomplementär zur Sequenz von SEQ ID NO: 40 ist; eine Sequenz, die reverskomplementär zu einem Abschnitt der Sequenz von SEQ ID NO: 40 ist; oder ein Reverse-Komplement eines Abschnitts von SEQ ID NO: 40 ist; oder
(b) ein Antisense-Oligonukleotid gegen die Sequenz von SEQ ID NO: 4 oder eine Sequenz, die zur Sequenz von SEQ ID NO: 4 reverskomplementär ist.

5. Verbindung zur Verwendung nach Anspruch 1 oder 2, wobei das Antisense-Oligonukleotid gegen einen Abschnitt von mindestens 11 aufeinanderfolgenden Basen der Sequenz von SEQ ID NO: 36 ein Antisense-Oligonukleotid der SEQ ID NO: 9 ist.

6. Verbindung zur Verwendung nach einem der vorstehenden Ansprüche, wobei das Antisense-Oligonukleotid ein Phosphorodiamidat-Morpholino-Oligonukleotid ist.

7. Verbindung zur Verwendung nach einem der vorstehenden Ansprüche, wobei das Subjekt ein natürlich vorkommendes EYA1-Protein exprimiert, umfassend die Sequenz von SEQ ID NO: 2.

8. Verbindung zur Verwendung nach Anspruch 7, wobei die für das EYA1-Protein kodierende Nukleinsäuresequenz die Nukleinsäuresequenz von SEQ ID NO: 7 oder die Nukleinsäuresequenz von SEQ ID NO: 8 umfasst.

## Revendications

1. Composé qui réduit le niveau du variant protéique EYA1A dans un organe parenchymateux pour une utilisation dans un procédé de prévention, de traitement ou de retardement de la progression de la fibrose et/ou de la desmoplasie dans ledit organe parenchymateux, dans lequel le composé est un oligonucléotide antisens vis-à-vis d'au moins l'une d'une partie d'au moins 11 bases consécutives dans la séquence de SEQ ID NO : 34 et/ou est un complément inverse de la partie au sein de la séquence de SEQ ID NO : 34, un oligonucléotide antisens vis-à-vis d'au moins l'une d'une partie d'au moins 11 bases consécutives dans la séquence de SEQ ID NO : 35 et/ou est un complément inverse de la partie au sein de la séquence de SEQ ID NO : 35, un oligonucléotide antisens vis-à-vis d'au moins l'une d'une partie d'au moins 11 bases consécutives dans la séquence de SEQ ID NO : 36 et/ou est un complément inverse de la partie de la séquence de SEQ ID NO : 36.

2. Composé pour une utilisation selon la revendication 1, dans lequel la prolifération aberrante de fibroblastes est au moins l'une parmi la desmoplasie et la fibrose.

3. Composé pour une utilisation selon la revendication 2, dans lequel l'oligonucléotide antisens vis-à-vis d'une partie d'au moins 11 bases consécutives dans la séquence de SEQ ID NO : 34 est
(a) un oligonucléotide antisens vis-à-vis de la séquence de SEQ ID NO : 39 ; une séquence qui est inversement complémentaire à la séquence de SEQ ID NO : 39 ; une séquence qui est inversement complémentaire d'une partie de la séquence de SEQ ID NO : 39 ; ou un complément inverse d'une partie de SEQ ID NO : 39 ; ou
(b) un oligonucléotide antisens vis-à-vis de la séquence de SEQ ID NO : 5 ou une séquence qui est inversement complémentaire à la séquence de SEQ ID NO : 5.

4. Composé pour l'utilisation selon la revendication 2, dans lequel l'oligonucléotide antisens vis-à-vis d'une partie d'au moins 11 bases consécutives de la séquence de SEQ ID NO : 35 est
(a) un oligonucléotide antisens vis-à-vis la séquence de SEQ ID NO : 40 ; une séquence qui est inversement complémentaire à la séquence de SEQ ID NO : 40 ; une séquence qui est inversement complémentaire d'une partie de la séquence de SEQ ID NO : 40; ou un complément inverse d'une partie de SEQ ID NO : 40 ; ou
(b) un oligonucléotide antisens vis-à-vis la séquence de SEQ ID NO : 4 ou une séquence qui est inversement complémentaire à la séquence de SEQ ID NO : 4.

5. Composé pour une utilisation selon la revendication 1 ou 2, dans lequel l'oligonucléotide antisens vis-à-vis une partie d'au moins 11 bases consécutives de la séquence de SEQ ID NO : 36 est un oligonucléotide antisens de SEQ ID NO : 9.

6. Composé pour une utilisation selon l'une quelconque des revendications précédentes, dans lequel l'oligonucléotide antisens est un oligonucléotide morpholino phosphorodiamidate.

7. Composé pour une utilisation selon l'une quelconque des revendications précédentes, dans lequel le sujet exprime une protéine EYA1 naturelle comprenant la séquence de SEQ ID NO : 2.

8. Composé pour une utilisation selon la revendication 7, dans lequel la séquence d'acides nucléiques codant la protéine EYA1 comprend la séquence d'acides nucléiques de SEQ ID NO : 7 ou la séquence d'acides nucléiques de SEQ ID NO : 8.
